# EUROPEAN PATENT APPLICATION

(11) **EP 1 892 528 A1**
(43) Date of publication of application: **27.02.2008**
(21) Application number: 06291358.7
(22) Date of filing: 25.08.2006
(51) Int. Cl.: G01N 33/50

(54) **Use of a modulating agent that interacts with the pbd of plk proteins for modulating ifn induction**

(71) Applicant: INSTITUT PASTEUR, 75724 Paris Cédex 15 (FR)
(72) Inventor: Meurs, Eliane, 75015 Paris (FR); Vitour, Damien, 91570 Bièvres (FR); Dabo, Stéphanie, 75018 Paris (FR); Vidalain, Pierre-Olivier, 92800 Puteaux (FR); Tangy, Frédéric, 93260 Les Lilas (FR); Jacob, Yves, 28130 Maintenon (FR)
(74) Representative: Desaix, Anne

(57) **Abstract**

The invention relates to use of a modulating agent for the preparation of a medicinal composition for modulating interferon (IFN) induction in a cell, and in particular for increasing IFN interferon in a cell, wherein said modulating agent interacts with the polo-box domain (PBD) of one or several polo-like kinase (PLK) protein(s). Such modulating agents can be used for increasing IFN production in a patient in need thereof, especially for the prophylaxis or the treatment of a patient infected with a virus, and in particular, for restoring or improving the innate immune response in a HCV-chronically infected patient. The present invention also relates to methods of screening for a modulating agent having the ability to interfere with interaction of PLK proteins with the Cardif protein and the ability to modulate IFN induction. The invention also relates to particular polypeptides and peptides, especially polypeptides and peptides derived from portions of the Cardif protein, which polypeptides or peptides, upon binding to the PBD domain of one or several PLK protein(s) prevent the PLK-dependant inhibition of the IFN induction pathway and thus increase or restore IFN induction in a cell.

## Description

The invention relates to use of a modulating agent that interacts with the polo-box domain (PBD) of one or several polo-like kinase(s) (PLK) proteins, such as PLK1 and PLK2, for the preparation of a medicinal composition for modulating interferon (IFN) induction in a cell, and in particular for increasing IFN production in a cell. Such modulating agents can be used for increasing IFN production in a patient in need thereof, especially for the prophylaxis or the treatment of a patient infected with a virus, and in particular, for restoring or improving the innate immune response in a HCV-chronically infected patient.

The present invention also relates to methods of screening for a modulating agent having the ability to interfere with binding of PLK proteins to the Cardif protein and the ability to modulate IFN induction.

The invention also relates to particular polypeptides and peptides, especially polypeptides and peptides derived from portions of the Cardif protein, which polypeptides or peptides, upon binding to the PBD domain of a or several PLK proteins, prevent the PLK-dependant inhibition of the IFN induction pathway and thus increase or restore IFN induction in a cell.

The cellular innate immune response is triggered by a variety of pathogens, such as bacteria or viruses and is essential to limit the initial spread of these pathogens. Pathogen agents are recognized through specific motifs or PAMPS (Pathogen-Associated Molecular Patterns) by different members of the Toll-like receptors (TLRs) family. After PAMPs recognition, the cytosolic Toll/interleukin (IL)-1 receptor domain (TIR) of the TLRs interacts with adaptor proteins, including Myd88, TIRAP/ Mal and TRIF, and leads to activation of MAPKs, NF-κB and IRF3 transcription factors by several distinct or overlapping signaling pathways (4).

Type-I interferons (IFNs), which include IFN-α and IFN-β (IFN-α/β), are important elements of the innate immune response. IFN is first elicited as IFNα and then also as IFNβ, and later as any type of existing IFN (in particular type-II IFN, e.g. IFNγ). The three major classes of IFN (α, β and γ) have been defined based on differences in amino acid structure.

IFN-α/β induction takes place after interaction of extracellular nucleic acids with members of the Toll like receptor family (TLRs) (1, 11, 18, 19) and after viral infection and intrusion into the cytoplasm through the interaction of viral double-stranded RNAs (dsRNAs) with specific cellular RNA helicases, such as RIG-I (retinoic-acid-inducible gene I, also called DDX58; 45) or MDA-5 (melanoma-differentiation-associated gene 5, also called Helicard; 2). RIG-I or MDA-5 consist in two N-terminal CARD (caspase recruitment domain), followed by an RNA helicase domain. Interaction of viral dsRNA with RIG-I or MDA-5 in the cytosol induces a change in the conformation of RIG-I or MDA-5. The N-terminal CARD of activated RIG-I or MDA5 then triggers IFN induction (21, 44, 45) through association with the recently identified IPS-1/MAVS/VISA/Cardif protein (22, 29, 35, 43). The particularity of this latter adapter protein, referred to here as Cardif (CARD adaptator inducing IFN-β), is to localize to the mitochondrial membrane (35). Cardif, in turn, recruits three kind of kinases, the MAPKs, the IKKαβγ complex and the TBK1/IKKε kinases, which results in activation of three essential transcription factors, AP-1 (Activator protein 1), NF-κB (nuclear factor κB) and IRF3 (IFN regulatory factor) respectively, which cooperate in induction of antiviral type I IFN. The IFN produced by an infected cell is secreted to surrounding cells.

Cardif contains a N-terminal CARD that interacts with both RIG-I and MDA-5 RNA helicase. The C-terminal part of Cardif comprises a transmembrane region that targets Cardif to the outer mitochondrial membrane. Knockout mice for cardif show impaired antiviral responses against different RNA viruses (23).

The genome of the Hepatitis C virus (HVC) presents structured dsRNA regions, such as its 5'NTR and 3'NTR which can trigger IFNβ induction soon after viral intrusion into the cellular host (39). However, Hepatitis C virus (HCV) interferes with IFN induction through the action of its NS3/4A (hepatitis C virus non-structural proteins 3 and 4A) protease, which is now known to cleave the mitochondria-bound protein Cardif that recruits TBK1 and IKKε (25, 27, 29). In addition, the NS3/4A protease can cleave the TRIF adapter that links the IFN-inducing kinases to the dsRNA-activated TLR3 (24), thus emphasizing the importance of these two IFN signaling pathways and the necessity for the virus to inhibit them in order to favor its propagation.

Whereas the interaction of the adapters TRIF or Cardif with their respective upstream partners TLR3 or RIG-I appears to be direct, their association with the downstream signaling pathways is complex. For instance, Cardif was shown to interact with IKKε (27, 29, 43) and TBK1 (43), not to interact with TBK1 (29) or interact with neither kinase (22). It was shown to interact with TRAF2 (43), TRAF6 (35, 43) and with FADD and RIP-1(22), however these two latter interactions were not confirmed (35). Similarly, Cardif could interact with IKKα and IKKβ (29) or not (35). In each of these cases, also, it is not known whether Cardif interacts directly with its downstream partners or if other partners are involved.

Recently, the inventors of the present invention have showed that IKKε, but not TBK1, colocalized strongly with Cardif at the mitochondrial membrane. HCV infection results in disruption of the Cardif/IKKε association, through the action of the HCV NS3/4A protease, which cleaves Cardif at residue 508 and causes dissociation of Cardif and IKKε (27).

To characterize further the association between Cardif and IKKε, the inventors of the present invention used a yeast two-hybrid screen according to the ORFeome procedure (32) and performed a search for Cardif and IKKε-interacting proteins. This procedure revealed that the polo-like kinase PLK1 is a common partner to Cardif and IKKε and that PLK2, another member of the polo-like kinase (PLK) family, is also a partner for Cardif (see example C-1). Sequencing of the PLK1 and PLK2 cDNA fragments recovered from the yeast positive clones showed that interaction of PLK1 with Cardif and IKKε and interaction of PLK2 with Cardif occur via the PBD of PLK1 and PLK2. These interactions were confirmed by transient transfection and coprecipitation techniques, which also revealed interaction between IKKε and PLK2 (see example C-2).

PLK1 and PLK2 belong to the family of the polo-like kinases (PLKs), a family of conserved serine/threonine (Thr/Ser) kinases found in organisms ranging from yeast to humans. The PLK family also includes PLK3 and PLK4.

PLK proteins, and in particular human PLK proteins, contain a N-terminal Serine/threonine kinase catalytic domain that is closely related to several members of the superfamily of protein kinases, and a unique C-terminal polo-box domain (PBD). The PBD of PLKs contains one or several polo-boxes (PB; 1 PB in human PLK4 and 2 PBs in human PLK1, 2 and 3). The crystal structures of the PLK1 C-terminus showed that the PBD comprises two β₆α motifs, which correspond to the two polo-box (PB). The three dimensional structures of the two polo-boxes within any one PLK are similar. The sequence of the PBD is highly conserved among all the PLK family members.

The PDB of PLKs functions as a single phosphopeptide-binding module and crystallisation of the human PLK1 PBD showed that the phosphopeptide binds to an interface formed between the two polo-boxes (reviewed in (3)). The PDB of PLKs is crucial for substrate interaction, for targeting PLK1 to specific subcellular multiprotein complexes and for regulating the kinase activity of the PLK1 (40). The PBD of all human PLKs binds to peptides that comprise phosphoserine (pSer) and/or phosphothreonine (pThr) and have a strong substrate affinity for Ser in the pThr/pSer-1 position and show a modest preference for proline in the pThr/pSer+1 position. In the absence of a bound substrate, the PBD inhibits the basal activity of the kinase domain. Phosphorylation-dependent binding of the PBD to its ligands releases the kinase domain, while simultaneously localizing polo-like kinases to specific subcellular structures.

The PLKs play an essential role during normal cell mitosis, during cytokinesis and in the maintenance of the fidelity of the checkpoint controls. In addition, PLKs have been implicated in tumor development. In particular, deregulation of PLK1 could contribute to tumor development; PLK1 is overexpressed in human tumors and different chemical inhibitors targeting PLK1 present a high therapeutic interest for cancer therapy (40).

In addition, and in contrast to a previous study that suggests that PLKs are not involved in cardif-mediated IFN induction (44), the inventors of the present invention have now provided evidences that PLK1 and PLK2 play a regulatory role to control IFN induction through Cardif upon viral infection:
- IKKε and the PBD of PLK1 interact with the same domain of Cardif, i.e., the domain from amino acid residues 364 and 540, and more probably the domain within amino acid residues 364 and 470 (see example C-3);
- overexpression of the PBD of PLK-1 interferes with Cardif/IKKε association (see example C-4);
- overexpression of the PBD of PLK1 or of the full length PLK1 or of the full length PLK2 strongly inhibits the ability of Cardif to induce IFN synthesis; in contrast, the intrinsic ability of the downstream IKKε to induce IFN is unaffected by overexpression of PLK1 or PLK2; this suggests that PLK1 and PLK2 specifically interfere with the Cardif/IKKε association in the IFN signaling pathway (see example C-5);
- PLKs can interfere with the ability of the cells to cause an antiviral response; PLK1 and PLK2 are able to strongly inhibit the increase in IFN induction observed in response to an external stimulus such as an infection by the Sendai virus (see example C-5).

The ability of PLK to control IFN induction might be of importance in the cell cycle in order to restrict the IFN-mediated apoptosis-inducing program and favor cell proliferation.

As indicated above, HCV infection also provokes abrogation of IFN induction by cleaving Cardif through its NS3/4A protease. Interestingly, HCV replication is tightly coupled to host cell proliferation and viral RNA synthesis was found enhanced during the S phase of the cell cycle. HCV chronic infection leads to development of hepatocarcinoma, and PLK1 is known to be overexpressed in human tumours. Several chemical inhibitors can now target the phosphopeptide docking site of PLK1 and represent a high therapeutic interest for cancer therapy.

Standard HCV treatment includes IFN (IFN-α), which is often combined with an antiviral drug called ribavirin, such a combination therapy being usually taken for 6 months to 1 year. However, this treatment, which is expensive, is associated with significant adverse effects, in particular flu-like symptoms (including a low-grade fever, chills, headache, muscle and joint aches, fatigue, and weakness), nausea and other gastrointestinal problems, loss of appetite, psychiatric symptoms (irritability, confusion, emotional instability, insomnia and sleep disturbances and a lack or concentration), thyroid abnormalities, hair loss, skin reactions and, in more severe cases, depression, organ damage, and blood conditions. Moreover, less than half of the patients who are treated, have a sustained virologic response (defined as lack of detectable HCV RNA in the serum six months after stopping therapy). Thus, although addition of ribavirin to interferon represents a significant advance in treatment for HCV infection, there remains a need for alternative treatment regimens (in particular for less expensive and non-toxic treatments) against chronic hepatitis C.

The present invention is in particular based on the assumption that deregulation of PLK, and in particular of PLK1, for instance an excessive expression of one or several PLK(s), can lead to disruption of the Cardif/IKKε interaction and, hence, concour with the ability of NS3/4A to cleave Cardif for the abrogation of the IFN inducing pathway and the innate immune response. In particular, in long-term HCV-infected patients, the innate immune response may be weakened through the combined action of :
- the HCV NS3/4A protease that cleaves Cardif and
- the activity of PLK proteins, in particular PLK-1, that disrupts Cardif/IKKε interaction. Therefore, the generation of inhibitors specifically abrogating the ability of PLKs to target Cardif, possibly in addition to inhibitors targeting NS3/4A, could be beneficial for the restoration of the innate immune response in HCV-chronically infected patients.

Targeting polo-like kinase 1 for cancer therapy represents a current goal and different inhibitors have been generated and are under clinical trials. In particular, the ON01910 compound targets PLK1 at or near the peptide-binding site, and inhibits this kinase with an IC50 of 9-10 nM. This drug is currently under clinical trial in cancer patients (38).

In view of the ability of PLK1 to target Cardif and interfere with IFN induction and, hence, the development of the innate immune response, the synthesis of peptides derived from the Cardif sequence and corresponding to phosphopeptide binding to PLK1 may lead to the generation of specific inhibitors that prevent the ability of PLK to abrogate IFN induction, and hence help to protect the innate immune response, while not affecting the normal cell proliferation.

Moreover, previous studies suggest that other viruses have developed mechanisms for counteracting IFN induction pathway to evade from the host's immune response. Indeed, non-structural proteins NS1 and NS2 of human respiratory syncytial virus were also shown to suppress the activation and nuclear translocation of IRF-3, and to inhibit the induction of IFN-α/β (37). In addition, Cardif has recently been shown to be indispensable for response against various single-stranded RNA (ssRNA) viruses, including Newcastle disease virus (NDV; a paramyxovirus), vesicular stomatitis virus (VSV, a rhabdovirus), Sendai virus (SeV; a paramyxovirus) and encephalomyocarditis virus (EMCV, a cardiovirus) (23).

Therefore, the use of modulating agents according to the present invention could be useful for the prevention and/or the treatment of many double-stranded or single-stranded RNA virus.

**BRIEF DESCRIPTION OF THE FIGURES**

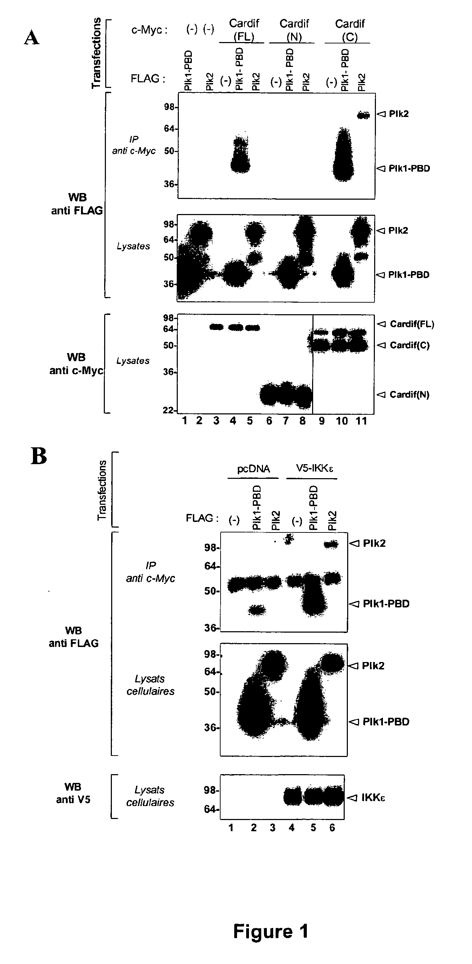

**Figure 1:** coimmunoprecipitation data showing interaction of Cardif and IKKε with PLK1-PBD and PLK2. 293T cells (7x 10⁵ cells) were transfected with: **A.** 2 µg of pcDNA3.1(+) empty (1-2) or expressing the different c-Myc-Cardif constructs, full length (FL :3-5; SEQ ID NO: 2), 1-153 N-terminus (6-8; SEQ ID NO: 18) or 154-540 C-terminus (9-11; SEQ ID NO: 20) in the presence of pcDNA-Hygro-FLAG (3,6,9), pflag-PLK1-PBD (1,4,7,10) or pflag-PLK2 (2,5,8,11); **B.** 2 µg of pcDNA3.1(+) empty (1-2) or expressing V5-IKKε (4-6) in the presence of pcDNA-Hygro-FLAG (1,4), pflag-PLK1-PBD (2 and 5) or pflag-PLK2 (3 and 6). Two days after transfection, the cell extracts were subjected to immunoprecipitation with 2 µl of a monoclonal antibody directed against the c-Myc epitope or 3µl of a monoclonal antibody directed against the V5 epitope. The presence of PLK1-PBD and PLK2 was revealed by immunoblot using a monoclonal antibody M2 anti-FLAG. Expression of the PLK, Cardif and IKKε constructs was controlled in the total cell extracts using anti-FLAG, anti-cMyc and anti-V5 monoclonal antibodies, respectively.

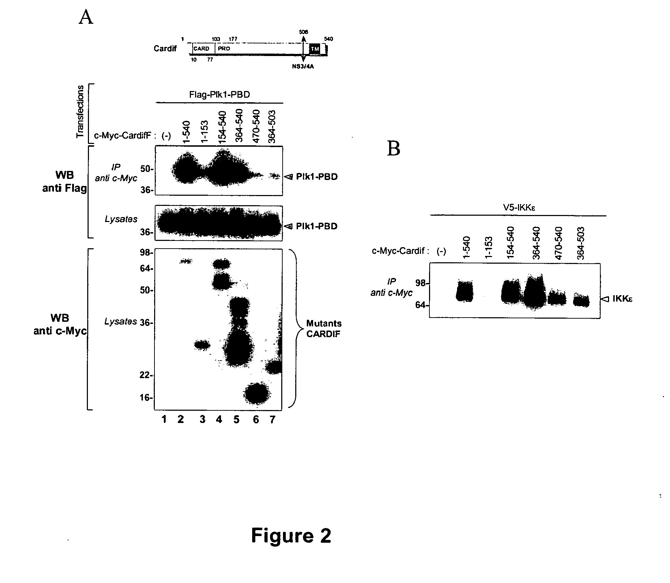

**Figure 2:** identification of the domains of Cardif that interacts with PLK1-PBD and with IKKε. Detection of PLK1-PBD (with an anti-FLAG monoclonal antibody), or IKKε (with an anti-V5 monoclonal antibody) and of Cardif (with an anti-cMyc monoclonal antibody) by immunoblot after cotransfection of 293T cells (7x 105 cells) either with PLK1-PBD (**A;** 0.5 µg of pflag-PLK1-PBD) or with IKKε (**B;** 5µg of pcDNA3.1(+)V5-IKKε), and both cases, with cardif (c-Myc-Cardif) either as full length (1-540; SEQ ID NO: 1), as the N-terminal CARD-like domain (1-153; SEQ ID NO: 17) or as the C-terminal domain of Cardif, complete (154-540; SEQ ID NO:19) or fragmented (364-540 (SEQ ID NO: 3), 470-540 (SEQ ID NO: 21) and 364-503 (SEQ ID NO: 23)). Two days after transfection, the cell extracts were subjected to immunoprecipitation with 2 µl of a monoclonal antibody directed against the c-Myc epitope. The presence of PLK1-PBD or of IKKε in the complexes was revealed by immunoblot using anti-FLAG or anti-V5 monoclonal antibodies. Expression of PLK1-PBD, IKKε and Cardif were controlled in the total cell extracts using an anti-FLAG, an anti-V5 and an anti-cMyc monoclonal antibody respectively.

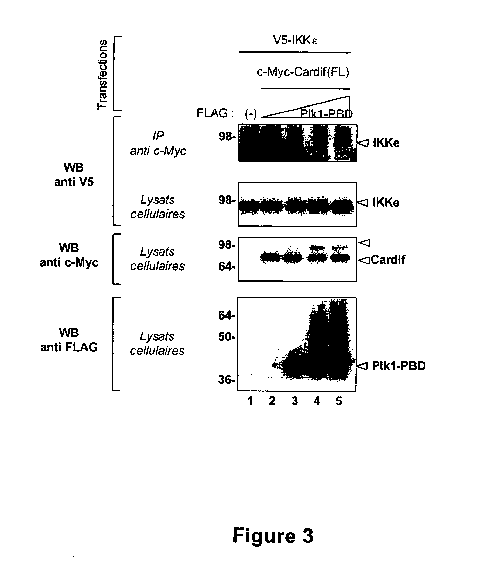

**Figure 3:** effect of PLK1-PBD overexpression on Cardif/IKKε association. 293T cells (7x 105 cells) were transfected with IKKε (lines 1-5 ;2µg of pcDNA3.1(+)V5-IKKε) and Cardif (lines 2-5; 2µg of pcDNA3.1(+)c-Myc-Cardif FL) and with different concentrations of PLK1-PBD (lines 3-5; 0.1, 0.4 or 1.6µg pflag-PLK1-PBD). Two days after transfection, the cell extracts were subjected to immunoprecipitation with 2 µl of a monoclonal antibody directed against the c-Myc epitope. The presence of IKKε in the complexes was revealed by immunoblot using anti-V5 monoclonal antibodies. Expression of IKKε, Cardif and PLK1-PBD was controlled in the total cell extracts using an anti-V5, anti-cMyc and anti-FLAG monoclonal antibody respectively.

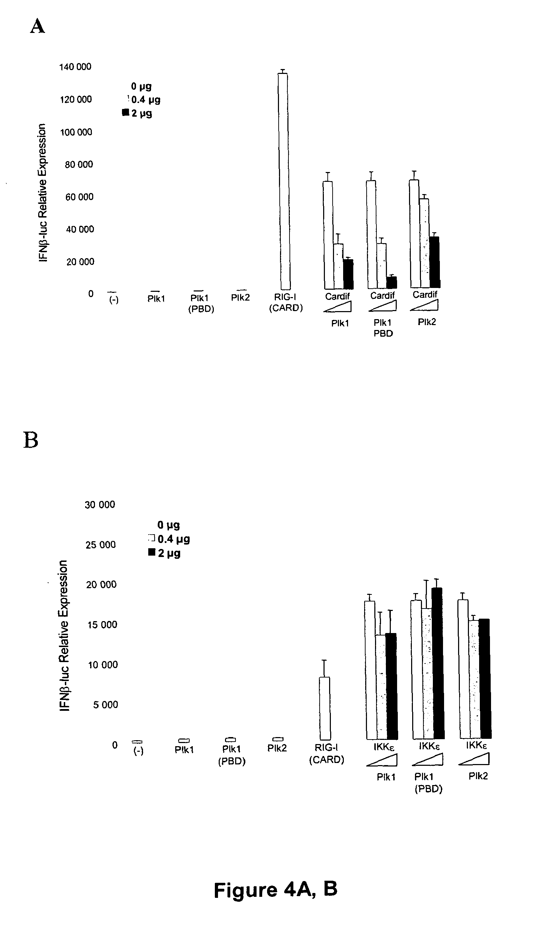

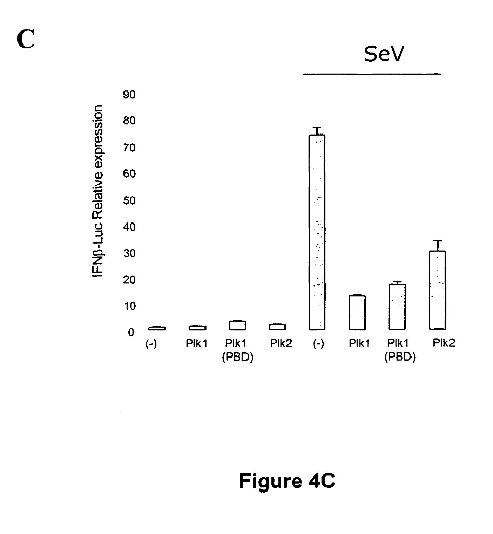

**Figure 4:** Reporter assay showing the effect of PLK1 or PLK2 overexpression on IFN induction.
In panel **A**, 293T cells (1.4 x 105 cells) were cotransfected with IFNβ-PGL3 (80 ng) and pRSV-βgal PGL3 (150 ng) in the presence of cardif full length (400 ng of the plasmid expressing pcDNA3.1 (+)c-Myc-Cardif FL) alone or in presence of two different concentrations (400ng and 2µg) of pcDNA3.1(+)c-Myc-PLK1, pflag-PLK1-PBD or pflag-PLK2. Each of the PLK expressing plasmid was also expressed individually in the cells and pEF-BOS-FLAG-RIG-I (ΔC) was transfected as positive control for luciferase induction. Luciferase activity was measured two days after transfection. The results are the mean of two experiments and are expressed as relative activity corresponding to the ratio of luciferase activity to the βgal expression in each sample. Expression of the different constructs was controlled by immunoblotting.
In panel **B**, the experiment was conducted as in A, except that the Cardif plasmid was replaced by the IKKε plasmid (pcDNA3.1 (+)V5-IKKε).
In panel **C**, the 293T cells were cotransfected with 80 ng of IFNβ-PGL3, 150 ng of pRSV-βgal and 400 ng of the PLK plasmid. One series of the transfected cells was infected 24h after the onset of transfection with 40 HAU/ml of Sendaï virus.

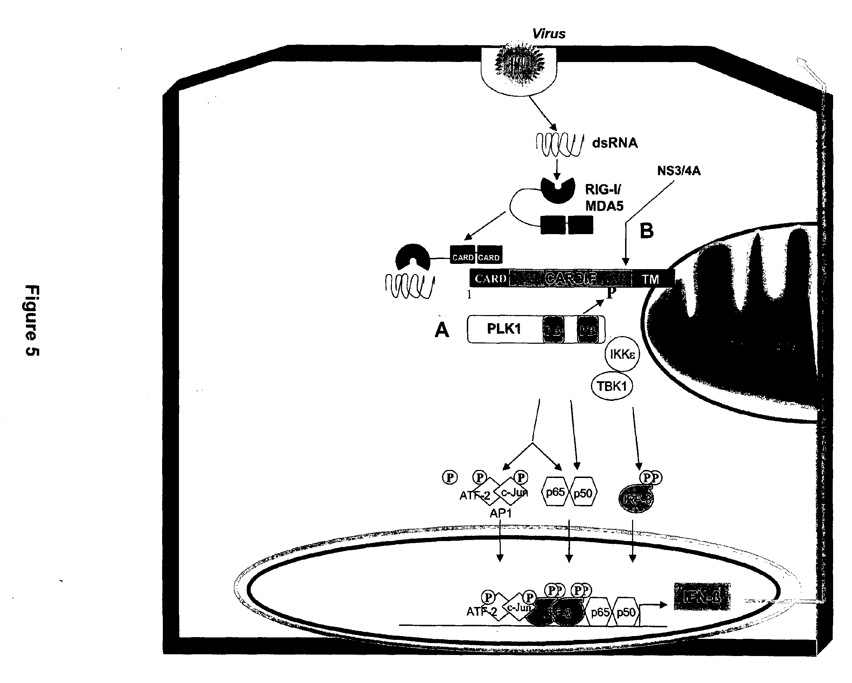

**Figure 5:** Scheme illustrating the modulation of the IFN induction pathway by PLK proteins and by the HCV NS3/4A protease. In the cytoplasm of a cell infected by a virus, the viral RNA (double stranded RNA; dsRNA) interacts with cellular RNA helicases such as RIG-I or MDA-5 RNA. Thus activated helicases associate with the Cardif protein, said Cardif protein being localized to the mitochondrial membrane. Activation of Cardif, results in activation of transcriptional factors, in particular in the phosphorylation of IRF-3, which bind to the IFN-β promoter in the nucleus and activate the IFN-β gene. The IFN produced by the infected cell is then secreted to surrounding cells. **A.** PLK proteins, and in particular PLK1, regulate the IFN induction pathway. The polo-box binding domain of PLK(s), which comprises one or several polo-box (PB; 2 PB in PLK1), is a phosphopeptide binding module that interacts with the Cardif protein. Interaction of PLKs with Cardif provokes inhibition of IFN induction by competition with IKKε (possibly, also with TBK1), IKKε and TBK1 being downstream partners of Cardif. **B.** Cardif is also a target for the HCV NS3/4A protease, which cleaves Cardif at amino acid residue 508 and causes dissociation of Cardif and IKKε. Therefore, the use of modulating agents that inhibit the Cardif/PLK association, possibly in addition to inhibitors targeting NS3/4A, could enable increasing or restoring IFN induction in a cell infected with a virus.

**DETAILED DESCRIPTION OF THE INVENTION**

The present invention relates to the use of a modulating agent for the preparation of a medicinal composition for modulating IFN induction in a cell, wherein said modulating agent interacts with the PBD of one or several PLK protein(s).

By "PLK protein", it is meant any poly-like kinase as defined above. This term includes naturally occurring allelic variants of the protein; and includes shortened proteins wherein one or more amino acid is removed from either or both ends of the full-length protein, or from an internal region of the protein, providing that the resulting molecule retains the ability of the full-length protein to interact with the Cardif protein. This term also includes lengthened proteins wherein one or more amino acid is added to either or both ends of the protein molecule, or to an internal location in the protein, providing that the resulting molecule retains the ability of the full-length protein to interact with the Cardif protein. The definition also includes proteins having substituted amino acid residues with respect to the naturally occurring allelic variants thereof, to the extent that the substituted protein does retain the ability to interact with the Cardif native protein.

The PLK protein is preferably a vertebrate PLK, preferably of mammalian origin, including human or non-human PLKs. In addition, the PLK protein is preferably chosen in the group consisting of PLK-1, PLK-2, PLK-3 and PLK-4, and is more preferably PLK-1 or PLK-2. In a particular embodiment, the PLK is the human PLK-1 (SEQ ID NO: 32) or the human PLK2 (SEQ ID NO: 42).

The "PBD" means the polo-box domain of the PLK that comprises a phosphopeptide-binding motif, as defined herein.

By "IFN induction", is meant induction of IFN transcription from the genome of the cell; it encompasses IFN-α/β, which is indeed regulated primarily at the transcriptional level in cells *in vivo.*

When the modulating agent "interacts with the PBD", it means that said modulating agent interferes either directly or indirectly on the PBD of one or several PLK protein(s). In a particular embodiment, said modulating agent acts directly on the PBD of one or several PLK proteins, i.e. said modulating agent binds to the PBD of one or several PLK protein(s).

As used herein, a "modulating agent" is any agent that modulates, i.e., modifies IFN induction, either in a normal or in a pathologic condition.

By "modulates" or "modulating" is meant changing, by either an increase or a decrease. The increase or decrease may be a change of any value between 10% and 90%, or of any value between 30% and 60%, or may be over 100% when compared with the level observed in a control cell (for example a wild type cell, which is normally proliferating and which is not infected by a virus), or the level observed in the case of reference agent. Thus, said "modulating agent" enables either increasing of decreasing IFN induction; through its interaction with the PBD domain of one or several PLK, said "modulating agent" influences cardif/PLK interaction, which the inventor have shown to be involved in the inhibition of IFN induction (see above). If the modulating agent favours cardif/PLK interaction, IFN induction may decrease, whereas if the modulating agent interferes with cardif/PLK interaction, it is likely increase IFN induction.

In a preferred embodiment, a modulating agent as described above is used for restoring and/or increasing IFN induction in a cell, i.e., the modulating agent is used to repress the PLK-induced inhibition of IFN induction in a cell; interaction of the modulating agent with the PBD domain and in particular, binding of the modulating agent to the PBD domain of one or several PLK protein(s) interferes with the ability of PLK to interfere with cardif/IKKε interaction, i.e., alters the PLK-Cardif association and/or prevents said PLK proteins to associate with the Cardif protein, thus inhibiting and/or preventing the PLK-induced inhibition of IFN induction. As a consequence, IFN induction is restored and/or increased. Thus, the use of such a modulating agent that prevent PLK proteins to associate with the Cardif protein and thus prevent PLK proteins to inhibit IFN induction could enable restoring a therapeutical amount of IFN in a cell without having to administrate any IFN.

The "modulating agent" can be any chemical compound, PBD-specific antibody, polypeptide, peptide, or other biotherapeutics that specifically binds to or interacts with the PBD domain of PLK proteins. Chemical agents, referred to in the art as "small molecule" compounds are typically organic, non-peptide molecules, having a molecular weight up to 10,000, preferably up to 5,000, more preferably up to 1,000, and most preferably up to 500 daltons. Antibodies that specifically bind to the PBD of PLK proteins can be generated using known methods. Preferably the antibody is specific to a mammalian ortholog of PLK polypeptide, and more preferably, to human PLK. Antibodies may be for example polyclonal, monoclonal, humanized or chimeric antibodies, single chain antibodies or Fab fragments. Polypeptides and peptides of the invention, suitable for their ability to act as modulating agent according to the invention are especially described hereafter.

The term "agent" includes, but is not limited to, compounds which may be obtainable from or produced by any suitable source, whether natural or not. Where appropriate, said modulating agent may be synthesized by any chemical or biological synthesis techniques

The modulating agent can be labelled by joining, either covalently or non-covalently, a substance that provides for a detectable signal. A wide variety of labels and conjugation techniques are known and are reported extensively in both the scientific and patent literature. Suitable labels include enzymes, substrates, cofactors, inhibitors, fluorescent moieties, chemiluminescent moieties, bioluminescent moieties, magnetic particles, and the like.

Said modulating agent can be prepared in a "medicinal composition" containing one or more suitable diluents and/or carriers or excipients, and which may comprise other active ingredients, as in combination therapy.

Modulating agents that are able to interact with PLK proteins, and in particular with the PBD of PLK proteins are known in the art. For example, several inhibitors that have been generated for targeting PLK1 for cancer therapy. In particular, the ONO1910 compound targets PLK1 at or near the peptide-binding site, and inhibits the kinase activity with an IC50 of 9-10 nM. However, this pharmacophore inhibits also a range of different kinases which lack PBD, indicating that this domain is not a specific target for ONO1910. This drug is however powerful and under current clinical trial in cancer patients (38).

The term "cell" encompasses prokaryotic and eukaryotic cells, in particular animal, and more particularly mammalian cells such as human cells as well as non-human cells. In addition, this term refers to dividing or non-dividing cells as well as to cells that are infected by a virus, or to non-infected cells. Furthermore the term cell is used to designate either cells in a culture or calls in the environment of an organism, especially an animal or human body. Accordingly when an effect is to be achieved in a cell, it is either to be achieved in a cell *in vitro* or to be achieved in a cell *in vivo,* i.e., in an organism, especially in a human patient.

In a particular embodiment, the cell, preferably a human cell, is infected by a virus, in particular by an RNA virus.

In a more particular embodiment, the cell is infected by an RNA virus that targets the IFN induction pathway, i.e., that targets the Cardif protein.

In a further particular embodiment, said virus is a single-stranded or a double-stranded RNA virus, chosen among the families consisting of:
- the flaviviridae, especially the genus hepacivirus, represented by the Hepatitis C virus (HCV),
- the paramyxoviridae, especially the genus paramyxovirus, comprising the Newcastle disease virus (NDV), the human respiratory syncytial virus, and the Sendai virus (SeV),
- the myxoviridae, especially the genus myxovirus, comprising the influenza virus,
- the rhabdoviridae, especially of the genus rhabdoviruses, comprising the vesicular stomatitis virus (VSV) and
- the picornaviridae, especially of the genus cardioviruses, comprising the encephalomyocarditis virus (EMCV).

In a more particular embodiment, said virus is the influenza virus or the HCV.

In another embodiment, a modulating agent as defined above is used for the preparation of a medicinal composition for increasing IFN induction (by repressing the PLK-induced inhibition of IFN induction) in a patient in need thereof.

In a particular embodiment, a modulating agent as defined above is used for the preparation of a medicinal composition for the prophylaxis or the treatment of a patient infected with a virus, in particular for the prophylaxis or the treatment of a patient infected with a virus that targets the IFN induction pathway.

In a particular embodiment, a modulating agent as defined above is used for restoring or increasing the innate immune response in a patient infected with a virus, especially in a patient infected with the HCV, and more especially, in a HCV-chronically infected patient.

As used herein, the patient that would benefit from the administration of the medicinal composition described herein includes any animal which can benefit from these methods. In a preferred embodiment, the patient is a human patient.

"Prophylaxis" is understood to be any degree of inhibition of the time of onset or severity of signs or symptoms of the virus infection, including, but not limited to, the complete prevention of the virus infection. This requires that the medicinal composition is provided to the patient in advance of any symptoms. Such a prophylactic administration serves to prevent, ameliorate, and/or reduce the severity of any subsequent infection.

"Virus infection" as used herein means that an RNA virus has attached to one or several cell(s) and that the viral RNA has penetrated into said cell(s).

When provided as a "treatment", the modulating agent is provided at (or shortly after) the onset of a contamination by the virus or on the occurrence of a symptom of infection or disease. The term "treatment" encompasses the curative effect achieved with the modulating agents of the invention and also the alleviation of the symptoms observed in a patient or the improvement of the patient's condition. It encompasses also the effects of detrimental consequences of virus infection, especially malignant states, including tumors resulting from virus infection when said effect improves the condition of the patient. The term "treatment" thus encompasses the slowing, interrupting, arresting or stopping of the progression of virus infection and/or detrimental consequences of virus infection; a treatment does not necessarily require the complete elimination of all disease symptoms and signs of virus infection.

Thus, the present modulating agent may be administered to a mammalian animal, or more preferably to a human patient, who is anticipated to develop a viral infection (prophylaxis) or after the occurrence of a viral infection, especially after the symptoms of viral infection have arised (treatment).

The medicinal composition may be administered in the form of a liquid solution or suspension, tablet, pill, powder, suppository, polymeric microcapsules or microvesicles, liposomes, and injectable or infusible solutions. The route of administration can include the typical routes including, for example, orally, subcutaneously, transdermally, intradermally, rectally, intramuscularly, intravenously, intra-arterially and parenterally.

In a particular embodiment, the medicinal composition further comprises a therapeutically effective amount of an inhibitor of a viral protease, especially of an inhibitor of a viral NS2/3 protease or a viral NS3/4A protease and more especially of an inhibitor of the HCV NS3/4A protease.

As used herein, "a therapeutically effective amount" refers to an amount effective, at dosages and for periods of time necessary, to achieve the desired therapeutic result, such as inhibition of the activity of the NS3/4A protease. The therapeutically effective amount may vary according to factors such as the infection state, age, sex, and weight of the individual being treated, and the ability of the compound to elicit a desired response in the individual. Dosage regimens may be adjusted to provide the optimum therapeutic response. A therapeutically effective amount is also one in which any toxic or detrimental effects of the compound are outweighed by the therapeutically beneficial effects.

As used herein, an "inhibitor" refers to any compound that is able to inhibit the action of the protein of interest. Any HCV NS3-4A protease inhibitor, such as VX-950 (developed by Vertex Pharmaceuticals), ITMN 191 (developed by InterMune), BILN-2061 (developed by Boehringer Ingelheim) or SCH 503034 (developed by Schering-Plough) can be used. Such an inhibitor can be used either alone or in combination with other protease inhibitor(s) in the medicinal composition of the invention.

The present invention also relates to a method of screening a test compound for its ability to interfere with the binding of the PBD domain of one or several PLK protein(s) to a Cardif protein, the method comprising the steps of:
(a) providing an assay system comprising:
   - a PLK protein or a fragment thereof containing the PBD or a PBD fragment that retains the ability of said PLK protein to bind to a Cardif protein, and
   - a Cardif protein;
(b) contacting said assay system with said test compound in conditions enabling the interaction between said test compound and said PLK protein or fragment thereof;
(c) determining whether said test compound interferes with the binding of the PBD of said PLK protein(s) or fragment thereof to said Cardif Protein.

Optionally, this method further comprises a step of:
(d) detecting a change in IFN induction in said assay system, relative to controls.

Furthermore, the present invention also relates to a method of screening a test compound for its ability to modulate (i.e. either increase or decrease) IFN induction, the method comprising the steps of:
(a) providing an assay system comprising:
   - a PLK protein or a fragment thereof containing the PBD or a PBD fragment that retains the ability of said PLK protein to bind to a Cardif protein, and
   - a Cardif protein;
(b) contacting the system with said test compound in conditions enabling the interaction between said test compound and said PLK protein or fragment thereof;
(c) detecting a change in IFN induction in said assay system, relative to controls.

Said PLK proteins and PBD used in the above-described methods of screening are as described herein. In particular embodiments, said PLK1 and PLK2 proteins are the human PLK1 protein (SEQ ID NO: 32; GenBank accession number NM_005030) and the human PLK2 protein (SEQ ID NO: 42; GenBank accession number NM_006622) respectively.

As used herein, a "fragment" of a PLK protein or of a PBD of a PLK protein is defined as a part of a full length wild type PLK protein or a part of a wild type PDB respectively, provided that said fragment retains the ability of said full-length PLK protein or said wild type PDB to interact with, and especially binds to a Cardif protein.

Said "Cardif protein" used in the methods of screening described herein encompasses a portion, an analogue or a variant thereof, provided that said portion, analogue or variant retains the ability of the Cardif protein to interact with the PBD of said PLK. In a particular embodiment, said Cardif protein is the human Cardif protein (SEQ ID NO: 2; GenBank accession number DQ181928).

As used herein, a "portion" of a Cardif protein is defined as a part of a full length wild type Cardif protein, provided that said portion retains the ability of the full-length Cardif protein to interact with, and especially binds the PBD domain of PLK proteins.

As used herein, a "variant" of a Cardif protein refers to any polypeptide or peptide modified with respect to the original polypeptide or peptide, provided that said variant retains the ability of the original polypeptide or peptide to interact with the PBD domain of PLK proteins. The variants differ from the original polypeptide or peptide by at least one amino acid substitution, preferably 1, 2, 3, 4 or 5 amino acid substitutions, either conservative, semi-conservative or non-conservative, or by at least one amino acid deletion or insertion, preferably 1, 2, 3, 4 or 5 deletion or insertion in the original amino acid sequence.

As used herein, the "assay system" may be cell-based (e.g. an animal, a cell, a cell lysate or a cell extract) or cell-free. In a particular embodiment, the assay system is a cell, either a prokaryotic cell, in particular, an *Escherichia coli* cell, or an eukaryotic cell, in particular, a yeast cell or a human cell. In addition, said cell can be either infected or non-infected with a virus. In a more particular embodiment, said cell is infected by a virus, especially by the HCV.

The "test compound" can be any chemical compound, antibody, polypeptide, peptide, or other biotherapeutics. It is either a purified natural compound or a compound synthesized by any chemical or biological synthesis technique. The test compound may be fused to a peptide tag for detection, or to another tag.

By "determining" is meant analysing the effect of a test compound on said assay system. This analysis can be performed by antibody labelling, immunoprecipitation, immunofluorescence assays, ELISA, structural analysis, or any other method known to those skilled in the art. Such an analysis may consist, for example, in determining the quantity of PLK protein(s) which is bound to the Cardif protein before and after step (b), wherein a decrease in the quantity of the PLK protein(s) which is bound to the Cardif protein after step (b) compared to the level of quantity of the PLK protein(s) bound to the Cardif in the absence of said test compound, indicates that the test compound indeed interferes with the binding of the PBD to Cardif.

By "detecting" is meant any convenient *in vitro* or *in vivo* test enabling to assess an increase or a decrease in IFN induction in said assay system relative to controls.

In a preferred embodiment, the above-described screening methods of the present invention are carried out *in vitro.*

The present invention also relates to a modulating agent identified by the screening methods described herein.

The present invention also relates to a modulating agent as described herein that binds to the PBD of one or several PLK protein(s), characterized in that it is a polypeptide or a peptide.

A "peptide" or "polypeptide" is any chain of three or more amino acids, including naturally occurring or non-naturally occurring amino acid residues or amino acid residue analogues, regardless of post-translational modification (e.g., glycosylation or phosphorylation).

Said peptide or polypeptide may be obtainable from or produced by any suitable source, whether natural or not. Where appropriate, said peptide or polypeptide may be synthesized by any chemical or biological synthesis techniques.

In a particular embodiment, said modulating agent (as described herein), and especially said peptide or polypeptide, is able to prevent or interfere with interaction of one or several PLKs with Cardif, in particular in an *in vitro* test.

As used herein, the *"in vitro* test" may consist in a method of screening as described herein.

In a particular embodiment, the amino acid sequence of such a polypeptide or peptide comprises or consists in a sequence derived from a portion of a Cardif protein, provided that said portion of Cardif, upon binding to the PBD of one or several PLK(s), prevents or interferes with binding of said PLK(s) to Cardif in a *in vitro* test as described herein. Accordingly, the polypeptide or peptide of the invention is shorter than the native Cardif protein, and especially is a derived from a portion of the Cardif protein. The polypeptide or peptide constituting the modulating agent is such that it is capable of binding to the PBD of one or several PLKs, especially to the PBD of human PLKs such as the human PLK1. When binding to the PBD of PLK, it prevents the interaction of said PLK with Cardif, especially prevents binding or interferes with binding of PLK with Cardif, thereby abolishing the inhibition of IFN induction through PLK in a cellular environment, especially *in vivo.*

Said "portion of a Cardif protein" is described herein.

"Derived from" as used herein means that the polypeptide has the sequence of a portion of the Cardif protein or alternatively is modified as compared to the sequence of the wild type Cardif protein, especially the wild type human Cardif protein (SEQ ID NO: 2; GenBank accession number DQ181928). This modification may be at least one substitution, especially a conservative substitution (substitution of an amino acid with another amino acid having similar properties such that the folding or activity of the protein is not significantly affected), deletion or insertion in the amino acid sequence. In a particular embodiment, the polypeptide or peptide is 70%, especially 80%, or in particular 90% identical to the region of Cardif from which it is derived.

In a particular embodiment, the amino acid sequence of the modulating agent comprises or consists in a sequence derived from the portion of the human Cardif protein ranging from amino acid residue 364 to amino acid residue 540 (SEQ ID NO: 4) or from the portion of the human Cardif protein ranging from amino acid residue 364 to amino acid residue 470 (SEQ ID NO: 6), provided that upon binding to the PBD domain of PLKs, said portion prevents binding of PLKs to the Cardif protein in an *in vitro* test, as defined above.

In particular embodiments, the amino acid sequence of the modulating agent comprises less than 30 contiguous amino acids, preferably less than 20, more preferably less than 15, and most preferably less than 10 contiguous amino acids of the region of cardif that interacts with the PBD of PLK proteins, especially the region ranging from amino acid residue 364 to amino acid residue 540 of the human Cardif protein (SEQ ID NO: 4) or the region ranging from amino acid residue 364 to amino acid residue 470 of the human Cardif protein (SEQ ID NO: 6).

In a particular embodiment, the peptide or polypeptide of the modulating agent contains 3 to 20, especially 3 to 15, or 3 to 10, especially 3 to 8 amino acid residues.

In a particular embodiment, the amino acid sequence of the modulating agent comprises amino acid residues S (for Serine) T (for threonine) and P (for Proline), either contiguous or not, especially forming an STP or a PST motif in the sequence of the peptide or polypeptide, possibly with some repetition of all or part of these residues in the peptide or polypeptide.

In a particular embodiment, the amino acid sequence of the modulating agent comprises a sequence chosen in the group consisting of STP (SEQ ID NO: 7), STVPSKLPTSS (SEQ ID NO: 9), PINSTRA (SEQ ID NO: 11), STVPTD (SEQ ID NO: 13) and STSLGMGP (SEQ ID NO: 15) or a variant thereof, provided the S, T, and P amino acid residues are present in the variant which retains the ability to bind to the PBD of one or several PLKs.

Said "variant thereof' refers to any peptide modified with respect to the original peptide, provided that said variant retains the ability of the original peptide to interact with the PBD domain of PLK proteins. The variants differ from the original peptide by at least one amino acid substitution, preferably 1, 2, 3, 4 or 5 amino acid substitutions, either conservative, semi-conservative or non-conservative, or by at least one amino acid deletion or insertion, preferably 1, 2, 3, 4 or 5 deletion or insertion in the original amino acid sequence.

In another embodiment, the modulating agent is a peptide chosen in the group consisting of STP (SEQ ID NO: 7), STVPSKLPTSS (SEQ ID NO: 9), PINSTRA (SEQ ID NO: 11), STVPTD (SEQ ID NO: 13) and STSLGMGP (SEQ ID NO: 15) or a variant thereof, provided the S, T, and P amino acid residues are present in the variant which retains the ability to bind to the PBD of one or several PLKs. A "variant thereof" is as described herein.

In a particular embodiment, the peptide chosen in the group consisting of STP (SEQ ID NO: 7), STVPSKLPTSS (SEQ ID NO: 9), PINSTRA (SEQ ID NO: 11), STVPTD (SEQ ID NO: 13) and STSLGMGP (SEQ ID NO: 15) or a variant thereof, is derived from a portion of a Cardif protein, especially from a human Cardif protein, as described herein.

In another particular embodiment, the modulating agent is phosphorylated. In a further particular embodiment, the modulating agent is a phosphorylated peptide (i.e. a phosphopeptide) or a phosphorylated polypeptide. In a further particular embodiment, the peptide or polypeptide is phosphorylated on one or several S and/or T amino acid residues.

Another aspect of the invention relates to the use of the above-described portion of the Cardif protein in the screening methods described herein, for identifying candidate modulating agent able to interfere with PLK/Cardif interaction and to modulate IFN induction.

The present invention also relates to a polynucleotide that codes for a modulating agent as described herein.

In a particular embodiment, the nucleotide sequence of the polynucleotide according to the present invention comprises or consists in a sequence chosen in the group consisting of SEQ ID NO: 8, SEQ ID NO: 10, SEQ ID NO: 12, SEQ ID NO: 14 and SEQ ID NO: 16, or a sequence modified by insertion, deletion or substitution of one or several nucleotides(s) in SEQ ID NO: 8, SEQ ID NO: 10, SEQ ID NO: 12, SEQ ID NO: 14 and SEQ ID NO: 16 respectively, provided that said modified sequence codes for the same polypeptide as that having respectively the sequence SEQ ID NO: 7, SEQ ID NO: 9, SEQ ID NO: 11, SEQ ID NO: 13, and SEQ ID NO: 15. It will be understood by a skilled person that numerous different nucleotide sequences can encode the same protein as a result of the degeneracy of the genetic code. In addition, it is to be understood that skilled persons may, using routine techniques, make nucleotide substitutions that do not substantially affect the activity encoded by the nucleotide sequence of the present invention to reflect the codon usage of any particular host organism in which the target is to be expressed.

The present invention also relates to a cloning or expression vector that comprises a polynucleotide insert under the control of its regulation, cloning or expression elements consisting of a polynucleotide as described above. Any appropriate cloning or expression vector can be used. The necessary transcriptional and translational signals, including promoter/enhancer element, can derive from the native cardif gene and/or its flanking regions or can be heterologous. A variety of host-vector expression systems may be utilized, such as mammalian cell systems infected with virus (e. g. vaccinia virus, adenovirus, etc.), insect cell systems infected with virus (e.g. baculovirus); microorganisms such as yeast containing yeast vectors, or bacteria transformed with bacteriophage, plasmid, or cosmid DNA.

The present invention also relates to a cell culture chosen among the group consisting of primary cell cultures (*i.e.,* a culture prepared from cells or tissues directly obtained from an animal) of bacteria or of eukaryotic cells (optionally non-human) and cell lines (i.e., populations of cells resulting from the first subculture of a primary culture or from subsequent serial passaging of the cells), characterized in that it comprises a polynucleotide or a vector as described above.

Another aspect of the invention relates to a medicinal composition comprising one or several modulating agent(s) or the vector as described herein, and one or several pharmaceutically acceptable carrier(s), vehicles(s), diluent(s), excipient(s) or adjuvant(s) or any combination thereof. The pharmaceutical compositions may be for human or animal usage in human and veterinary medicine. The choice of pharmaceutical carrier, excipient or diluent can be selected with regard to the intended route of administration and standard pharmaceutical practice. The pharmaceutical compositions may comprise as, or in addition to, the carrier, excipient or diluent, any suitable binder(s), lubricant(s), suspending agent(s), coating agent(s), solubilising agent(s). Preservatives, stabilizers, dyes and even flavouring agents may also be provided in the pharmaceutical composition.

In a particular embodiment, the medicinal composition according to the invention further comprises an inhibitor of a viral protease, especially an inhibitor of a viral NS2/3 protease or an inhibitor of a viral NS3/4A protease and more especially an inhibitor of the HCV NS3/4A protease. Said "inhibitor" is as described above.

All compositions quoted above can be formulated for enteral, parenteral, subcutaneous, intradermal, intramuscular or intravenous injection, oral administration and intranasal administration or inhalation.

The present invention also relates to the use of a modulating agent or a medicinal composition as described herein for interfering with PLK/Cardif interaction and thus modulating IFN induction in a cell as described herein. In particular, the present invention relates to the use of a modulating agent or a medicinal composition as described herein for increasing IFN production in a cell as described herein.

The present invention further relates to the use of a modulating agent or the medicinal composition as described above for the preparation of a medicinal composition for the prophylaxis or the treatment of a patient infected with a virus, especially an RNA virus, and in particular, for the treatment of a HCV infected patient.

The invention also provides kits for the treatment of a HCV infected patient, comprising:
- one or several modulating agent(s) or a medicinal composition as described above, and
- instructions including directions for administering at least one dose of the therapeutic substance to a patient in need thereof.

The invention also relates to a method for modulating IFN induction in a cell, comprising the step of contacting the cell with a modulating agent as defined above

The invention further relates to a method for treating a pathological condition involving IFN induction disorder, comprising the step of administering to a patient in need thereof a modulator agent or the pharmaceutical composition as defined in the application, in an amount sufficient to prevent PLK to interfere with INF induction pathway such that the innate immune response is restored in said patient.

The invention also relates to an antibody, either a polyclonal antibody or a monoclonal antibody, that specifically binds to a modulating agent as defined in this application.

In addition, the invention relates to a method of preparation of a polyclonal serum against the modulating agent as defined above, comprising the following steps:
- inoculating an animal with the modulating agent as defined above, either associated with an adjuvant or adjuvant-free, to induce an immune response, and
- collecting and purifying the produced antibodies directed against the polypeptide used for the inoculation.

Finally, the invention also relates to a method of preparation of monoclonal antibodies against the modulating agent as defined above, comprising the following steps:
- inoculating an animal, e.g. a Balb/c mouse, with the modulating agent as defined above, to induce an immune response;
- fusing spleen cells from the inoculated animal with myeloma cells to form a hybridoma;
- growing the hybridoma in conditions that allow antibody production;
- collecting and purifying the produced antibodies directed against the polypeptide used for the inoculation.

**EXAMPLES**

The invention will be further described in the following examples, which do not limit the scope of the invention described in the claims. The relevant disclosures of all references cited herein are specifically incorporated by reference.

**A. MATERIAL**

A-1. Yeast two-hybrid analysis

ORFs corresponding to IKKε (nucleotide 1 to 2148) and Cardif (nucleotide 1 to 1539) were fused in frame using the following primers:
Cardif-Forward: 
5'-GGGGACAACTTTGTACAAAAAAGTTGGCATGCCGTTTGCTGAAGACA
AGA;
Cardif-Reverse: 
5'-GGGGACAACTTTGTACAAGAAAGTTGGTTATGAGGGCCTGTGGCATG
G;
IKKe-Forward: 
5'-GGGGACAACTTTGTACAAAAAAGTTGGCATGCAGAGCACAGCCAATT
AC;
IKKe-Reverse: 
5'-GGGGACAACTTTGTACAAGAAAGTTGGTTAGACATCAGGAGGTGCTG
GGACTCTATT;
with the coding sequence of the DNA-binding domain of Gal4 using *in vitro* recombination (Gateway system, Invitrogen) as previously described (42). Then, Gal4-DB-IKKε and Gal4-DB-Cardif constructs were transfected in AH109 yeast cells (Clontech) according to the manufacturer's procedure. The two fusion proteins did not induce transactivation *per se*. In parallel, a commercial human spleen cDNA library cloned in the Gal4-AD pPC86 two-hybrid vector (Invitrogen) was transfected in the Y187 yeast cells (Clontech). A mating strategy was used for two-hybrid screening of the human spleen cDNA library (15). Positives colonies were selected from -50 million diploids on synthetic medium missing histidine and supplemented with 10 mM 3-amino-triazole (Sigma-Aldrich). The positive colonies were submitted to three rounds of screening to avoid contamination by irrelevant DNA (41). Positive clones were analyzed by direct sequencing of PCR products obtained from yeast colonies. The cDNA from these clones was then recovered and inserted into different acceptor Gateway vectors, in fusion with a triple Flag epitope.

**A-2. Cell cultures**

The Human Embryonic Kidney HEK 293T cells, the Huh-7 cell line, the Huh-7-5 cell line (5, 28) were cultured as in (8).

**A-3. Plasmids**

The pcDNA3.1-V5-IKKε was generated by PCR on an IKKε plasmid described previously (8, 36). The pcDNA3.0-c-Myc-Cardif full length (FL) was described in (26). The Cardif N terminus 1-153 (SEQ ID NO: 18) and the Cardif constructs 1-256 (SEQ ID NO: 26), 1-360 (SEQ ID NO: 28) and 1-466 (SEQ ID NO: 30) were generated by site-directed mutagenesis according to Stratagene. The Cardif C terminus (154-540; SEQ ID NO: 20) was generated by PCR. The sequence of the PLK1 and PLK2 cDNA fragments recovered from the yeast positive colonies corresponded to their polo-box domains (PLK1 323-603 (SEQ ID NO: 35) and PLK2 453-685 (SEQ ID NO: 43) for interaction with Cardif, and PLK1 275-603 (SEQ ID NO: 33) for interaction with IKKε). The full length PLK1 plasmid was obtained from Roy Goldsteyn (17). The PLK1 fragment was recovered from the yeast plasmid using the Gateway cloning procedure and inserted in frame 3' of a triple Flag epitope sequence into a translation optimized pCINeo vector (Promega) using recombinational cloning (Gateway, Invitrogen). It is referred to as pFlag-PLK1-PBD. The entire sequence for PLK2 (SEQ ID NO: 41) was copied by RT-PCR from Huh7 cells and subcloned in the triple Flag pCINeo vector (pFlag PLK2). The PLK1 and PLK1 PBD with the mutations His558A and Lys540M (SEQ ID NO: 39 and 40 respectively) were generated by site-directed mutagenesis on the pMyc-PLK1 and pFlag-PLK1-PBD plasmids.

The reporter plasmid ISG56-luc was provided by Cleveland Clinic Foundation.The reporter AP1-luc was from Promega.

**A-4. Antibodies**

The following antibodies were used:
- monoclonal antibody anti-V5 from Invitrogen ;
- monoclonal antibodies anti-c-Myc (A-14 and 9E10) and anti-PLK1 (F-8) from Santa Cruz;
- monoclonal antibody anti-FLAG (M2) from Sigma;
- polyclonal antibody against Cardif from Alexis Biochemicals;
- mouse monoclonal antibody against IKK (Mab against TBK1) from UBI
- Cy3-anti-mouse and anti-rabbit antibodies from Chemicon;
- Fluorescein- anti-Mouse and anti-Rabbit from Vector.

**B. METHODS**

**B-1. Transfection and reporter assay**

Cells were transfected using lipofectamine 2000 (InVitrogen) and the reporter assays were performed as described (8). 18 to 24 hours before transfection, the cells were seeded in 24-well plates (Falcon; Becton Dickinson) at different concentrations depending on the cell types (100 000/well for HEK293 cells, 80 000/well for Huh-7 cells). Three hours before transfection, the cells were washed and incubated in 300 µl of culture medium deprived of antibiotics and containing 10% serum. The required amount of lipofectamine 2000 (from a 1 mg/ml stock solution ; InVitrogen) was diluted with 75 µl of antibiotic-free and serum-free DMEM and mixed for 20 min at room temperature with 75 µl of the same medium containing DNA in the required combinations, to give a final ratio of 1.2 µl Lipofectamine/µg DNA. For each sample, the DNA mix was adjusted to 1/10th of their total content with a plasmid expressing RSV-β-galactosidase for normalization of the data. 150 µl of the medium covering the cells was removed and replaced with the 150 µl of the lipofectamine/DNA mix. After 3h incubation with the mix, the cells were washed twice with 10% serum-antibiotic free medium and further incubated in this medium in presence of antibiotics. At 24h after transfection, the culture medium was aspirated and the cells washed once in phosphate-buffer-saline (PBS). The cells in each well were then scraped and lysed in 200 µl of luciferase lysis buffer (25 mM Tris-phosphate [pH 7.6], 8 mM MgCl₂, 1 mM dithothreitol [DTT], 0.1% Triton X-100, 15% glycerol) per well. For each sample, 50 µl were mixed with 50 µl of Bright-Glo™ luciferase Assay Reagent (Promega) and analyzed for luciferase activity using the Reporter™ Microplate Luminometer (Turner designs). A different 50 µl aliquote was also taken from each sample and analysed for β-galactosidase activity using a β-galactosidase Enzyme assay system (Promega).

**B-2. Immunoblot analysis**

The different cells were transfected 24h after plating using lipofectamine 2000 (InVitrogen) at a concentration of 1.2 µl/µg of DNA. Two days after transfection, the cells were washed with PBS and scraped in 300 µl of lysis buffer Buffer 20 mM Tris pH 7.5, 150 mM NaCl, 10 % Glycerol, 1% Triton X-100, 1 mM DTT) containing 1 mM sodium orthovanadate (Na3VO4), 10 mM β-glycerophosphate and 50 mM sodium fluoride (NaF) as phosphatase inhibitors as described in (40). After 20 min of incubation on ice, the cell extracts were centrifuged at 12 000 g for 20 min at 4°C, transferred to other tubes and stored at -80°C. The immunoblot analysis was performed as described (8).

**B-3. Immunoprecipitation assay**

The cell extracts were incubated with 30 µl of A/G agarose beads (Santa Cruz Biotechnology) for 30 min and centrifuged for 5 min at 4°C at 3000g to clear them from non specific contaminants. The supernatants containing the cell extracts were then incubated with the requested antibodies overnight at 4°C, then 28 µl of A/G agarose beads was added to the mix and the incubation was continued at 4°C for 60 min. The mixtures were then centrifuged at 1500g for 5 min at 4°C to precipitate the complexes attached to the beads. These were subjected to three rounds of wash with 300 µl of lysis buffer and centrifugation at 1500 g. The beads were then resuspended in 70 µl of Laemmli lysis buffer, heated at 95°C and processed for SDS-PAGE.

**B-4. Immunofluorescence**

Huh7 cells were seeded at 150 000 cells/well in 24-well plates containing glass coverslips and grown overnight. Staining of mitochondria was performed with MitoTracker® Deep Red (Invitrogen/Molecular probes) and fixation and permeabilization of the cells were as described (27). Anti-Cardif antibodies were diluted 1:500, anti-PLK1 antibodies were diluted 1:200, Samples were analysis with wide-field inverted Axiovert 200M Zeiss microscope at magnification x63.

**C. RESULTS**

**C-1. Identification ofPLK1 as one common partner to Cardif and IKKε by yeast two-hybrid screen**

A yeast two-hybrid screen was conducted to perform a systematic search of partners linking two important components of the IFN signaling pathway: the mitochondria-bound cytosolic protein Cardif (also referred to as IPS-1/MAVS/VISA) and the IRF3-phosphorylating kinase IKKε. Out of a total of 50 x 10⁶ transformants obtained with a human spleen cDNA library, 27 and 80 positive colonies were obtained, respectively, when Cardif and IKKε were used as baits (Table 1).

Table 1: results of a systematic search by a yeast two-hybrid screen of putative partners for two important components of the IFN signaling pathway, Cardif and IKKε.

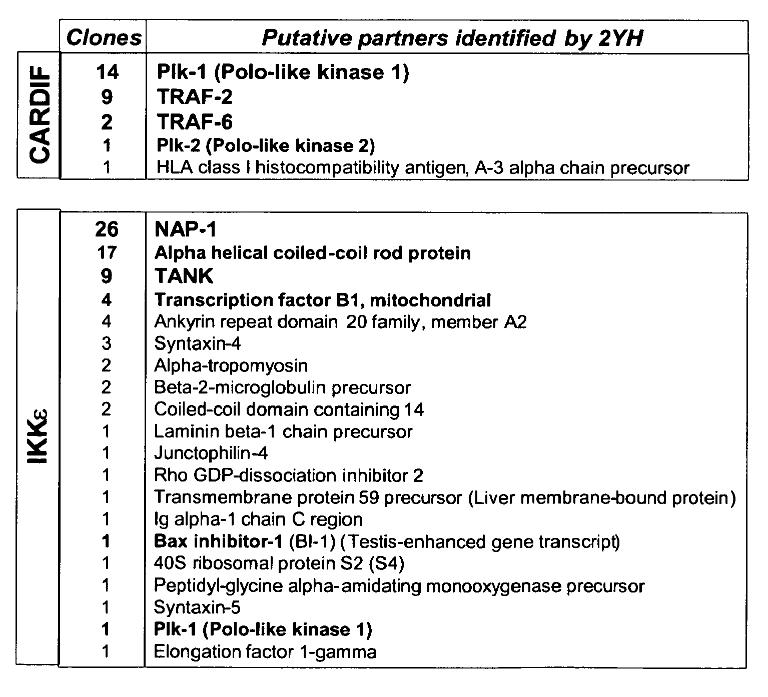

The majority of the partners for Cardif: PLK1 (14 positive clones), TRAF2 (9 clones) and TRAF6 (2 clones), corresponded well with those identified by a previous study, with the same frequency (43). In addition, two new partners, PLK2 and HLA class 1 were found. Among the partners for IKKε, the majority of the clones encode for NAP1 (26 positive clones), coiled coil domain (17 clones) and TANK (9 clones). NAP1 has been previously identified through a systematic search for TBK1 (also known as NAK) partners by yeast two hybrid (16) and was shown to interact with both TBK1 and IKKε, but not with the canonical kinases IKKα and IKKβ and the NEMO/IKKγ. Binding of IKKε to TANK has also been previously reported (31). Therefore, identification of these two partners for IKKε provides strong validation of the present yeast screen. Interestingly, TANK binds TRAF1, -2 and -3 (9), and TRAF3 has been recently described as a novel partner for Cardif, involved in the activation of the downstream IFN inducing pathway (33). It is therefore possible that Cardif associates with IKKε through a TRAF3/TANK interaction. TRAF3 was however not depicted in the present yeast search.

The present yeast hybrid search revealed PLK1 as the only partner common to Cardif and IKKε. The hit for IKKε was low (1 hit) as compared to that for Cardif (14 hits). However, another member of the PLK1 family, PLK2, was also identified as a partner for Cardif. The sequence of the PLK1 and PLK2 cDNA fragments recovered from the yeast positive clones corresponded to their polo-box domains (PLK1 323-603 (SEQ ID NO: 35) and PLK2 453-685 (SEQ ID NO: 43) for interaction with Cardif, and PLK1 275-603 (SEQ ID NO: 33) for interaction with IKKε). PLK1 was already found as partner for Cardif, by a previous yeast two-hybrid screen (43). The identification of PLK1 as partner for IKKε and PLK2 as partner for Cardif are novel findings. The relationships between Cardif, IKKε and the polo like kinases PLK1/2 were therefore investigate further.

**C-2. Interaction of Cardif and IKK**ε **with PLK1-PBD and PLK2**

To investigate the interaction of Cardif with the PLKs identified by the yeast two-hybrid, 293 T cells were transfected with vectors expressing Flag-tagged PLK1 and PLK2 constructs and c-myc-tagged Cardif. For the latter, we used constructs corresponding to its full length sequence (1-540; SEQ ID NO:2) as well as constructs encoding separately for its RIG-I binding, CARD-like N terminus domain (1-153; SEQ ID NO:18) and for its C terminus (154-540; SEQ ID NO:20). The results first confirmed the interaction of Cardif with the PLK1 fragment recovered from the yeast positive clones and showed that this interaction occurs specifically with the C terminus of Cardif. The PLK1 fragment (323-603; SEQ ID NO: 36) contains the two polo-box (PB) of PLK1 (region 407-603; SEQ ID NO: 38). PBDs are phosphopeptide-binding domains and crystallisation of the human PLK1 PBD showed that phosphopeptides bind to an interface formed between the two polo-boxes (reviewed in (3)). The coimmunoprecipitation data also allowed to confirm interaction between PLK2 in Cardif. Interestingly, the latter was detected only when PLK2 was expressed in presence of the Cardif C terminus but not in presence of full length Cardif (Figure 1 A). A similar experiment was performed in which 293 T cells were transfected with PLK1-PBD and IKKε. This allowed to confirm the interaction of PLK1-PBD with IKKε, depicted by the yeast screen. In addition, introduction of PLK2 in this assay, allowed to show that this kinase is also capable of interaction with IKKε (Figure 1 B). Therefore, these coprecipitation assays confirmed interaction of Cardif with both PLK1 and PLK2 as well as the interaction of IKKε with PLK1. In addition, this technique allowed to reveal an interaction between IKKε and PLK2, which was not detected by the yeast assay.

**C-3. PLK1-PBD and IKKε interact with the same domain of Cardif**

Different Cardif deletion mutants were generated to delineate the domains of Cardif which are involved in the recognition of PLK1 and to compare them with the Cardif domains necessary for the recruitment of IKKε. The results show that the PLK1-binding domains of Cardif are localized between the 364 and the 540 residue. It is possible that this region is located between 364 and 470, since the constructs 470-540 (SEQ ID NO: 22) could not retain PLK1. Another Cardif internal construct, comprised between 364 and 503 residue (22 Kda fragment; SEQ ID NO: 24) also did not bind PLK1, this may indicate that the transmembrane domain of Cardif and, possibly, is localization at the mitochondria outer membrane is necessary for the interaction with PLK1 (Figure 2A). In regards with binding of IKKε to Cardif, the domains 154-540 (SEQ ID NO: 20) and 364-540 (SEQ ID NO: 4) were also found to bind IKKε strongly. To the difference with the binding of Cardif to PLK1, the deletion 470-540 and the internal region 364-503 could retain significantly some IKKε. Altogether, these results show that the PLK1-binding motif of Cardif is contained within the 364 and 470 residues (SEQ ID NO: 6), within a region that binds strongly IKKε. The remaining C terminus of Cardif can still bind IKKε but with much less efficiency, indicating that IKKε can bind Cardif either directly or through other adapters but that its recruitment to Cardif is more efficient at the 364-470 region (Figure 2B). Therefore, association of PLK1 with both Cardif and IKKε revealed by the yeast two hybrid was confirmed by the coprecipitation assays which showed in addition that PLK1 can be pulled down by the the same region of Cardif that is involved in the Cardif/IKKε interaction.

**C-4. Overexpression of PLK1-PBD interferes with Cardif and IKKε association**

We then investigated whether the PLK1 PBDs could disrupt the interaction of IKKε with Cardif. 293 T cells were transfected with c-myc-Cardif full length and IKKε expressing vectors and the effect of increasing concentrations of PLK1-PBD on the Cardif/IKKε interaction was determined by coprecipitation assay. The results showed that overexpression of PLK1-PBD can profoundly affect the Cardif/IKKε association (Figure 3). The residual IKKε band that still coprecipitates with Cardif in presence of the highest dose of PLK1-PBD might represent some interaction of the kinase with Cardif which does not involve PLK1, as shown in figure 2 B.

**C-5. Overexpression of PLK1 and PLK2 inhibits IFN induction**

The above results indicates that either PLK1 is involved in the recruitment of IKKε by Cardif or that PLK1 targets the Cardif/IKKε association to dissociate it. A functional reporter assay was used to determine the effect of PLK on the IFN inducing pathway. We first found that neither PLK1, full length of its PBD domain, nor PLK2 has an intrinsic ability to induce IFN. In contrast, the ability of Cardif to induce the synthesis of IFN was strongly inhibited in presence of PLK1, either full length or its PBD domain. Induction of IFN by Cardif was also inhibited in presence of PLK2 (Figure 4A). The effect of the PLK on Cardif was specific since neither PLK1 nor PLK2 can inhibit the ability of IKKε to induce IFN (figure 4B). As PLK1 and PLK2 bind IKKε (figure 1 B) but do not interfere with its ability to induce IFN (figure 4B), we can hypothesize that these PLK interfere with the IFN inducing pathway by targeting a Cardif domain necessary for the recruitment of IKKε. Interestingly, the ability of PLK1 to inhibit the Cardif-mediated IFN induction is stronger when the PLK1-PBD domains are used in the functional assay. Inhibition by PLK2 was found to be less effective than that of PLK1 but the effect of the PLK2-PBD has not been examined.

In order to determine the effect of the PLKs in conditions where IFN induction is provoked by an external stimilus, the cells were submitted to Sendaï virus (SeV) infection. Infection by SeV resulted in a 75-fold stimulation of transcription from the IFNβ promoter. This induction was severely inhibited in presence of PLK1 and PLK1-PBD (about 80%) and still inhibited but to a lesser extend by PLK2 (57%) (Figure 4C). Therefore these results show that the polo-like kinases can interfere with the ability of the cells to mount an antiviral response, during a viral infection.

**C-6. Effect of silencing PLK1 on IFN induction**

Since overexpression of PLK can inhibit IFN induction, it is possible that depending on the state of cell proliferation and endogenous PLK expression, these polo-like kinases can regulate Cardif expression. We are investigating the IFN induction in response to a viral infection in conditions where PLK levels are down regulated by silencing.

**C-7. Is Cardif a docking protein for PLK?**

The polo-box domain of PLK1 is a phosphopeptide-binding motif, through which PLK can dock specific phosphorylation targets. If Cardif is a target for PLK1, this implies that Cardif has been previously marked through a priming phosphorylation. The pThr or pSer -containing phoshopeptide bind to one sequence contained between the two polo Box domains of PLK1. The preferred motifs targeted by PBD have been shown to contain S, T and binding is enhanced by the presence of a proline (3). The crystal structure of the PBD of PLK1 (10) with a bound phosphopeptide of sequence MQS(pT)PL (13) showed that the PLK1 Lys540 and His538 are the only residues that make direct contact with the phosphate group. The Cardif sequence does not contain an exact MQSTPL motif but presents several ST motifs with adjacent or close to proline residues (233 **STP** (SEQ ID NO: 7); 320 **ST**V**P**SKL**PTS**S (SEQ ID NO: 9); 349 **P**IN**ST**RA (SEQ ID NO: 11); 375 **ST**V**PT**D (SEQ ID NO: 13) and 444 **ST**SLGMG**P** (SEQ ID NO: 15)).

The motif STP at position 233 is the motif that is the closest from the consensus sequence described by Elia *et al.,* 2003 (13). However, the coprecipitation experiments indicated that PLK1 PBD could be recruited by Cardif between its residues 364 and 470, which gives the possibility for the sequences STVPTD, or STSLGMGP to serve as docking sites. PLK1 may also bind Cardif through other sites not related to phosphorylation. Therefore, in order to determine unambiguously whether PLK1 PBD recruits Cardif through its phosphopeptide recognition sites, we have generated PBD that lacks the ability to target proteins by mutating its His538 to Ala and Lys540 to Met (SEQ ID NO: 40). We are performing reporter and coprecipitation assays to determine whether the mutant PLK can still inhibit the ability of Cardif to induce IFN and whether the Cardif constructs can still bind phosphopeptide-docking PBD mutants.

**D. DISCUSSION**

The mitochondria-bound Cardif adapter plays an important role in the innate immune response as it activates in response to viral intrusion and provoke activation of three major signaling pathways, such as MAPK, NFκB and the IRF3-phosphorylating kinases TBK1/IKKε. These pathways lead to induction of IFN and other cytokines, involved in the host defence.

Here, we report the association of Cardif and IKKε with the endogenous proteins PLK1 and PLK2 and present evidence for a role of these proteins in controlling the IFN induction mediated by Cardif. Our results show that the PLKs can inhibit the Cardif/ IKKε association and interrupt the IFN signalling pathway. This leads to a novel function for PLK1 and PLK2, as regulators of IFN induction. The mechanism would involve recognition by these PLKs of specific Cardif motifs located at or near the motifs necessary to recruit the downstream kinases, such as IKKε.

HCV has been recently shown to also target Cardif, probably in order to favour its replication and propagation. Cardif was cleaved at residue 508 during HCV infection through the action of the viral NS3/4A protease. This provokes disruption of Cardif from the downstream signaling pathways kinases and, in particular, provokes dissociation of Cardif and IKKε (reviewed in (20)). PLKs would represent an endogenous cellular mechanism to control the function of Cardif, through a different mechanism. By down regulating IFN induction and the often associated apoptosis activation, PLKs, which are important for host cell proliferation, may thus tilt the balance in favour of cell growth.

Inhibition of IFN induction by the prolyl isomerase Pin1 has recently been reported (34). This work shows that pin1 binds to the IRF3 transcription factor and provokes its polyubiquitination and degradation. In a former report (12), it was shown that PLk1 can stabilize Pin1 by inhibiting its ubiquitination in human cells. Therefore, a link between these two findings lead to the hypothesis that the PLK1-mediated inhibition of IFN induction could be related to its effect on Pin1 and therefore locate downstream of Cardif. However, the work describes by Eckerdt et al shows that the kinase function of PLK1 is required to stabilize the function of Pin1. In our work, we show that the ability of PLK1 to inhibit the Cardif-mediated induction of IFN does not require its kinase function as it can be mediated only by its poloBox domain. Therefore, our data strongly suggest that PLK1 has the ability to inhibit IFN induction by Cardif through a mechanism different that the one involving Pin1.

The polo-like kinases play an essential role during mitosis, cytokinesis and in the maintenance of the fidelity of the checkpoint controls (3). In the light of the ability of both the PLKs and the HCV NS3/4A protease to target Cardif, it is interesting to recall that HCV replication was noticed to be tightly coupled to host cell proliferation and viral RNA synthesis was found enhanced during the S phase of the cell cycle. Highest levels were found in exponentially growing cells, followed by a sharp decline in resting cells, suggesting that cellular factors required for RNA replication and/or translation vary in abundance and become limiting in resting cells (30). Recently also, HCV replication was found to depend more on the integrity of a cytoskeleton than on mitosis. Indeed, cytoskeleton inhibitors such as vinblastine and nozocodazole blocked RNA synthesis in an HCV replicon cell culture system (7).

We postulate that deregulation of PLK1, for instance an excessive expression of PLK1, can lead to disruption of the Cardif/IKKε interaction and, hence, concour with the ability of NS3/4A to cleave Cardif for the abrogation of the IFN inducing pathway and the innate immune response (see figure 6). Therefore, inhibitors targeting PLK1, in addition to inhibitors targeting NS3/4A could be beneficial for the restoration of innate immune response in HCV-chronically infected patients. Targeting polo-like kinase 1 for cancer therapy represents a current goal and different inhibitors have been generated and are under clinical trials. In particular, the ONO1910 compound targets PLK1 at or near the peptide-binding site, and inhibits this kinase with an IC50 of 9-10 nM. However, this pharmacophore inhibits also a range of different kinases which lack PBD, indicating that this domain is not a specific target for ONO1910. This drug is however powerful and under current clinical trial in cancer patients (38).

In view of the ability of PLK1 to target Cardif and interferes with IFN induction and, hence, the development of the innate immune response, the synthesis of peptides derived from the Cardif sequence and corresponding to phosphopeptide binding to PLK1 may lead to the generation of specific inhibitors that prevent the ability of PLK to abrogate IFN induction, and hence help to protect the innate immune response, while not affecting the normal cell proliferation.

### REFERENCES

1. Alexopoulou, L., A. C. Holt, R. Medzhitov, and R. A. Flavell. 2001. Recognition of double-stranded RNA and activation of NF-kappaB by Toll-like receptor 3. Nature 413:732-8.
2. Andrejeva, J., K. S. Childs, D. F. Young, T. S. Carlos, N. Stock, S. Goodbourn, and R. E. Randall. 2004. The V proteins of paramyxoviruses bind the IFN-inducible RNA helicase, mda-5, and inhibit its activation of the IFN-beta promoter. Proc Natl Acad Sci U S A 101:17264-9.
3. Barr, F. A., H. H. Sillje, and E. A. Nigg. 2004. Polo-like kinases and the orchestration of cell division. Nat Rev Mol Cell Biol 5:429-40.
4. Barton, G. M., and R. Medzhitov. 2003. Toll-like receptors signaling pathways. Science 300:1524-1625.
5. Blight, K. J., J. A. McKeating, and C. M. Rice. 2002. Highly permissive cell lines for subgenomic and genomic hepatitis C virus RNA replication. J Virol 76:13001-14.
6. Bonnet, M. C., C. Daurat, C. Ottone, and E. F. Meurs. 2006. The N terminus of PKR is responsible for activation of the NF-kB signaling pathway by interacting with the IKK complex. Cell signalliing.
7. Bost, A. G., D. Venable, L. Liu, and B. A. Heinz. 2003. Cytoskeletal requirements for Hepatitis C Virus (HCV) RNA synthesis in the HCV replicon Cell culture System. J Virol 77:4401-4408.
8. Breiman, A., N. Grandvaux, R. Lin, C. Ottone, S. Akira, M. Yoneyama, T. Fujita, J. Hiscott, and E. F. Meurs. 2005. Inhibition of RIG-I-dependent signaling to the interferon pathway during hepatitis C virus expression and restoration of signaling by IKKepsilon. J Virol 79:3969-78.
9. Cheng, G., and D. Baltimore. 1996. TANK, a co-inducer with TRAF2 of TNF- and CD 40L-mediated NF-kappaB activation. Genes Dev 10:963-73.
10. Cheng, K. Y., E. D. Lowe, J. Sinclair, E. A. Nigg, and L. N. Johnson. 2003. The crystal structure of the human polo-like kinase-1 polo box domain and its phospho-peptide complex. Embo J 22:5757-68.
11. Diebold, S., T. Kaisho, H. Hemmi, S. Akira, and C. Reis e Sousa. 2004. Innate antiviral responses by means of TLR7-mediated recognition of single-stranded RNA. Science.
12. Eckerdt, F., Yuan, J., Saxena, K., Martin, B., Kappel, S., Lindenau, C., Kramer, A., Naumann, S., Daum, S., Fischer, G., Dikic, I., Kaufmann, M. & Strebhardt, K. 2005. Polo-like kinase 1-mediated phosphorylation stabilizes Pin1 by inhibiting its ubiquitination in human cells. J Biol Chem 280, 36575-83.
13. Elia, A. E., L. C. Cantley, and M. B. Yaffe. 2003. Proteomic screen finds pSer/pThr-binding domain localizing Plk1 to mitotic substrates. Science 299:1228-31.
14. Fitzgerald, K. A., M. S.M., K. L. Faia, D. C. Rowe, E. Latz, D. T. Golenbock, A. J. Coyle, S.-M. Liao, and T. Maniatis. 2003. IKKepsilon and TBK1 are essential components of the IRF3 signaling pathway. Nature Immunology 4:491-496.
15. Fromont-Racine, M., R. J.C, and P. Legrain. 1997. Toward a functional analysis of the yeast genome through exhaustive two-hybrid screens. Nature Genetics 16:277-282.
16. Fujita, F., Y. Taniguchi, T. Kato, Y. Narita, A. Furuya, T. Ogawa, H. Sakurai, T. Joh, M. Itoh, M. Delhase, M. Karin, and M. Nakanishi. 2003. Identification of NAP1, a regulatory subunit of IkappaB kinase-related kinases that potentiates NF-kappaB signaling. Mol Cell Biol 23:7780-93.
17. Golsteyn, R. M., S. J. Schultz, J. Bartek, A. Ziemiecki, T. Ried, and E. A. Nigg. 1994. Cell cycle analysis and chromosomal localization of human PIk1, a putative homologue of the mitotic kinases Drosophila polo and Saccharomyces cerevisiae Cdc5. J Cell Sci 107 (Pt 6):1509-17.
18. Heil, F., H. Hemmi, H. Hochrein, F. Ampenberger, C. Kirschning, S. Akira, G. Lipford, H. Wagner, and S. Bauer. 2004. Species-specific recognition of single-stranded RNA via toll-like receptor 7 and 8. Science 303:1526-9.
**19.** Hemmi, H., O. Takeuchi, T. Kawai, T. Kaisho, S. Sato, H. Sanjo, M. Matsumoto, K. Hoshino, H. Wagner, K. Takeda, and S. Akira. 2000. A Toll-like receptor recognizes bacterial DNA. 408:740-745.
20. Hiscott, J., R. Lin, P. Nakhaei, and S. Paz. 2006. MasterCARD: a priceless link to innate immunity. Trends Mol Med 12:53-6.
21. Kato, H., S. Sato, M. Yoneyama, M. Yamamoto, S. Uematsu, K. Matsui, T. Tsujimura, K. Takeda, T. Fujita, O. Takeuchi, and S. Akira. 2005. Cell type-specific involvement of RIG-I in antiviral response. Immunity 23:19-28.
22. Kawai, T., K. Takahashi, S. Sato, C. Coban, H. Kumar, H. Kato, K. J. Ishii, O. Takeuchi, and S. Akira. 2005. IPS-1, an adaptor triggering RIG-I- and Mda5-mediated type I interferon induction. Nat Immunol.
23. Kumar, H., Kawai, T., Kato, H., Sato, S., Takahashi, K., Coban, C., Yamamoto, M., Uematsu, S., Ishii, K. J., Takeuchi, O., Akira, S. 2006. Essential role of IPS-1 in innate immune responses against RNA viruses. J Exp Med. 203:1795-803.
24. Li, K., Z. Chen, N. Kato, M. Gale, Jr., and S. M. Lemon. 2005. Distinct poly(I-C) and virus-activated signaling pathways leading to interferon-beta production in hepatocytes. J Biol Chem 280:16739-47.
25. Li, X. D., L. Sun, R. B. Seth, G. Pineda, and Z. J. Chen. 2005. Hepatitis C virus protease NS3/4A cleaves mitochondrial antiviral signaling protein off the mitochondria to evade innate immunity. Proc Natl Acad Sci U S A 102:17717-22.
26. Lin, K., R. B. Perni, A. D. Kwong, and C. Lin. 2006. VX-950, a novel hepatitis C virus (HCV) NS3-4A protease inhibitor, exhibits potent antiviral activities in HCv replicon cells. Antimicrob Agents Chemother 50:1813-22.
27. Lin, R., J. Lacoste, P. Nakhaei, Q. Sun, L. Yang, S. Paz, P. Wilkinson, I. Julkunen, D. Vitour, E. F. M e u r s, and J. H i s c o t t. 2006. Dissociation of a MAVS/IPS/VISA/Cardif-IKke molecular complex from the mitochondrial outer membrane by hepatitis virus C NS3/4A proteolytic cleavage. J Virol.
28. Lindenbach, B. D., M. J. Evans, A. J. Syder, B. Wolk, T. L. Tellinghuisen, C. C. Liu, T. Maruyama, R. O. Hynes, D. R. Burton, J. A. McKeating, and C. M. Rice. 2005. Complete replication of hepatitis C virus in cell culture. Science 309:623-626.
29. Meylan, E., J. Curran, K. Hofmann, D. Moradpour, M. Binder, R. Bartenschlager, and J. Tschopp. 2005. Cardif is an adaptor protein in the RIG-I antiviral pathway and is targeted by hepatitis C virus. Nature.
30. Pietschmann, T., V. Lohmann, G. Rutter, K. Kurpanek, and R. Bartenschlager. 2001. Characterization of cell lines carrying self-replicating hepatitis C virus RNAs. J Virol 75:1252-64.
31. Pomerantz, J. L., and D. Baltimore. 1999. NF-kappaB activation by a signaling complex containing TRAF2, TANK and TBK1, a novel IKK-related kinase. EMBO J 18:6694-6704.
32. Rual, J. F., T. Hirozane-Kishikawa, T. Hao, N. Bertin, S. Li, A. Dricot, N. Li, J. Rosenberg, P. Lamesch, P. O. Vidalain, T. R. Clingingsmith, J. L. Hartley, D. Esposito, D. Cheo, T. Moore, B. Simmons, R. Sequerra, S. Bosak, L. Doucette-Stamm, C. Le Peuch, J. Vandenhaute, M. E. Cusick, J. S. Albala, D. E. Hill, and M. Vidal. 2004. Human ORFeome version 1.1: a platform for reverse proteomics. Genome Res 14:2128-35.
33. Saha, S. K., E. M. Pietras, J. Q. He, J. R. Kang, S. Y. Liu, G. Oganesyan, A. Shahangian, B. Zarnegar, T. L. Shiba, Y. Wang, and G. Cheng. 2006. Regulation of antiviral responses by a direct and specific interaction between TRAF3 and Cardif. Embo J 25:3257-63.
34. Saitoh, T., Tun-Kyi, A., Ryo, A., Yamamoto, M., Finn, G., Fujita, T., Akira, S., Yamamoto, N., Lu, K. P. & Yamaoka, S. 2006. Negative regulation of interferon-regulatory factor 3-dependent innate antiviral response by the prolyl isomerase Pin1. Nat Immunol 7, 598-605
35. Seth, R. B., L. Sun, C. K. Ea, and Z. J. Chen. 2005. Identification and Characterization of MAVS, a Mitochondrial Antiviral Signaling Protein that Activates NF-kappaB and IRF3. Cell 122:1-14.
36. Sharma, S., B. R. tenOever, N. Grandvaux, G. P. Zhou, R. Lin, and J. Hiscott. 2003. Triggering the interferon antiviral response through an IKK-related pathway. Science 300:1148-51.
37. Spann, K. M., Tran, K. C., Collins, P. L. 2005. Effects of nonstructural proteins NS1 and NS2 of human respiratory syncytial virus on interferon regulatory factor 3, NF-kappaB, and proinflammatory cytokines. J Virol. 79:5353-62.
38. Strebhardt, K., and A. Ullrich. 2006. Targeting polo-like kinase 1 for cancer therapy. Nat Rev Cancer 6:321-30.
39. Sumpter, R., Jr., Y. M. Loo, E. Foy, K. Li, M. Yoneyama, T. Fujita, S. M. Lemon, and M. Gale, Jr. 2005. Regulating Intracellular Antiviral Defense and Permissiveness to Hepatitis C Virus RNA Replication through a Cellular RNA Helicase, RIG-I. J Virol 79:2689-99.
40. Tanabe, Y., T. Nishibori, L. Su, R. M. Arduini, D. P. Baker, and M. David. 2005. Cutting edge: role of STAT1, STAT3, and STAT5 in IFN-alpha beta responses in T lymphocytes. J Immunol 174:609-13.
41. Vidalain, P. O., M. Boxem, H. Ge, S. Li, and M. Vidal. 2004. Increasing specificity in high-throughput yeast two-hybrid experiments. Methods 32:363-70.
42. Walhout, A. J., R. Sordella, X. Lu, J. L. Hartley, G. F. Temple, M. A. Brasch, N. Thierry-Mieg, and M. Vidal. 2000. Protein interaction mapping in C. elegans using proteins involved in vulval development. Science 287:116-22.
43. Xu, L. G., Y.-Y. Wang, K.-J. Han, L.-Y. Lii, Z. Zhai, and H.-B. Shu. 2005. VISA is an adapter protein required for Virus-Triggered IFN-b Signaling. Molecular Cell 19:1-14.
44. Yoneyama, M., M. Kikuchi, K. Matsumoto, T. Imaizumi, M. Miyagishi, K. Taira, E. Foy, Y. M. Loo, M. Gale, Jr., S. Akira, S. Yonehara, A. Kato, and T. Fujita. 2005. Shared and Unique Functions of the DExD/H-Box Helicases RIG-I, MDA5, and LGP2 in Antiviral Innate Immunity. J Immunol 175:2851-8.
45. Yoneyama, M., M. Kikuchi, T. Natsukawa, N. Shinobu, T. Imaizumi, M. Miyagishi, K. Taira, S. Akira, and T. Fujita. 2004. The RNA helicase RIG-I has an essential function in double-stranded RNA-induced innate antiviral responses. Nat Immunol 5:730-7.

## Claims

1. Use of a modulating agent for the preparation of a medicinal composition for modulating interferon (IFN) induction in a cell, wherein said modulating agent interacts with the polo-box domain (PBD) of one or several polo-like kinase (PLK) protein(s).

2. The use according to claim 1, for restoring and/or increasing IFN induction in a cell.

3. The use according to claim 1 or 2, wherein said PLK protein is a vertebrate PLK.

4. The use according to any of claims 1 to 3, wherein said PLK protein is a human PLK.

5. The use according to any of claims 1 to 4, wherein said PLK protein is chosen in the group consisting of PLK-1, PLK-2, PLK-3 and PLK-4.

6. The use according to claim 5, wherein said PLK protein is PLK-1.

7. The use according to any of claims 1 to 6, wherein said modulating agent binds to the PBD of one or several PLK protein(s).

8. The use according to any of claims 1 to 7, wherein said cell is a mammalian cell.

9. The use according to any of claims 1 to 8, wherein said cell is a human cell.

10. The use according to any of claims 1 to 9, wherein said cell is infected by a virus.

11. The use according to claim 10, wherein said virus is an RNA virus.

12. The use according to claim 11, wherein said virus targets the IFN induction pathway.

13. The use according to claim 12, wherein said virus is a virus chosen among the families consisting of:
- the flaviviridae, especially the genus hepacivirus represented by the Hepatitis C virus (HCV),
- the paramyxoviridae, especially the genus paramyxovirus, comprising the Newcastle disease virus (NDV), the human respiratory syncytial virus, and the Sendai virus (SeV),
- the myxoviridae, especially the genus myxovirus, comprising the influenza virus,
- the rhabdoviridae, especially of the genus rhabdoviruses, comprising the vesicular stomatitis virus (VSV) and
- the picornaviridae, especially of the genus cardioviruses, comprising the encephalomyocarditis virus (EMCV).

14. The use according to claim 13, wherein said virus is the HCV.

15. The use according to any of claims 1 to 14, for increasing IFN induction in a patient in need thereof.

16. The use according to any of claims 1 to 15, for the prophylaxis or the treatment of a patient infected with a virus that targets the IFN induction pathway.

17. The use according to any of claims 1 to 16, for restoring or increasing the innate immune response in a patient infected with a virus.

18. The use according to any of claims 1 to 17, for restoring or increasing the innate immune response in a patient infected with the HCV.

19. The use according to any of claims 10 to 18, wherein the medicinal composition further comprises a therapeutically effective amount of an inhibitor of a viral protease.

20. The use according to claim 19, wherein the medicinal composition further comprises a therapeutically effective amount of an inhibitor of a HCV protease, especially of the HCV NS3/4A protease.

21. A method of screening a test compound for its ability to interfere with the binding of the PBD domain of one or several PLK to a Cardif protein, the method comprising the steps of:
(a) providing an assay system comprising:
- a PLK protein or a fragment thereof containing the PBD or a PBD fragment that retains the ability of said PLK protein to bind to a Cardif protein, and
- a Cardif protein;
(b) contacting said assay system with said test compound in conditions enabling the interaction between said test compound and said PLK protein or fragment thereof;
(c) determining whether said test compound interferes with the binding of the PBD of said PLK proteins or fragment thereof to said Cardif Protein.

22. The method according to claim 21, wherein said method further comprises the step of
(d) detecting a change in IFN induction in said assay system, relative to controls.

23. A method of screening a test compound for its ability to modulate IFN induction, the method comprising the steps of:
(a) providing an assay system comprising:
- a PLK protein or a fragment thereof containing the PBD or a PBD fragment that retains the ability of said PLK protein to bind to a Cardif protein, and
- a Cardif protein;
(b) contacting the system with said test compound in conditions enabling the interaction between said test compound and said PLK protein or fragment thereof;
(c) detecting a change in IFN induction in said assay system, relative to controls.

24. The method according to any of claims 21 to 23, wherein said cell is a prokaryotic cell, in particular, an *Escherichia coli* cell.

25. The method according to any of claims 21 to 23, wherein said cell is a eukaryotic cell, in particular, a yeast cell or a human cell.

26. The method according to any of claims 21 to 25, wherein said cell is infected by a virus, especially by the HCV.

27. A modulating agent that binds to the PBD of one or several PLK protein(s), **characterized in that** it is a polypeptide or a peptide.

28. The modulating agent according to claim 27, **characterized in that** its amino acid sequence comprises or consists in a sequence derived from a portion of a Cardif protein, provided that said portion of Cardif, upon binding to the PBD domain of one or several PLK(s), prevents binding or interferes with binding of said PLK(s) to Cardif in a *in vitro* test.

29. The modulating agent according to claim 28 that is a peptide or a polypeptide comprising S, T and P amino acid residues, either contiguous or not in the sequence of the peptide or polypeptide.

30. The modulating agent according to any of claims 27 to 29, **characterized in that** its amino acid sequence comprises or consists in a sequence derived from the portion of the human Cardif protein ranging from amino acid residue 364 to amino acid residue 540 (SEQ ID NO: 4).

31. The modulating agent according to any of claims 27 to 30, **characterized in that** its amino acid sequence comprises or consists in a sequence derived from the portion of the human Cardif protein ranging from amino acid residue 364 to amino acid residue 470 (SEQ ID NO: 6).

32. The modulating agent according to any of claims 27 to 31, **characterized in that** its amino acid sequence comprises a sequence chosen in the group consisting of STP (SEQ ID NO: 7), STVPSKLPTSS (SEQ ID NO: 9), PINSTRA (SEQ ID NO: 11), STVPTD (SEQ ID NO: 13) and STSLGMGP (SEQ ID NO: 15) or a variant thereof.

33. The modulating agent according to any of claims 27 to 32, **characterized in that** it is a peptide chosen in the group consisting of STP (SEQ ID NO: 7), STVPSKLPTSS (SEQ ID NO: 9), PINSTRA (SEQ ID NO: 11), STVPTD (SEQ ID NO: 13) and STSLGMGP (SEQ ID NO: 15) or a variant thereof.

34. The modulating agent according to any of claims 27 to 33 that is phosphorylated.

35. A polynucleotide that codes for the modulating agent according to any of claims 27 to 34.

36. The polynucleotide according to claim 35, **characterized in that** its nucleotide sequence comprises or consists in a sequence chosen in the group consisting of SEQ ID NO: 8, SEQ ID NO: 10, SEQ ID NO: 12, SEQ ID NO: 14 and SEQ ID NO: 16 or a sequence modified by insertion, deletion or substitution of one or several nucleotides(s) in SEQ ID NO: 8, SEQ ID NO: 10, SEQ ID NO: 12, SEQ ID NO: 14 and SEQ ID NO: 16 respectively, provided that said modified sequence codes for the same polypeptide as that having respectively the sequence SEQ ID NO: 7, SEQ ID NO: 9, SEQ ID NO: 11, SEQ ID NO: 13, and SEQ ID NO: 15.

37. A cloning or expression vector that comprises a polynucleotide insert under the control of its regulation, cloning or expression elements consisting of a polynucleotide according to claim 35 or 36.

38. A medicinal composition comprising one or several modulating agent(s) according to any of claims 27 to 34 or the vector according to claim 35, and one or several pharmaceutically acceptable carrier(s), vehicle(s), diluent(s), excipient(s) or adjuvant(s) or any combination thereof.

39. The medicinal composition according to claim 38 that further comprises an inhibitor of the HCV NS3/4A protease.

40. The medicinal composition according to claim 38 or 39, which is formulated for enteral, parenteral, subcutaneous, intradermal, intramuscular or intravenous injection, oral administration and intranasal administration or inhalation.

41. The modulating agent according to any of claims 27 to 34 or the medicinal composition according to any of claims 36 to 38 for use for increasing IFN production in a cell.

42. The modulating agent according to any of claims 27 to 34 for the preparation of a medicinal composition for the prophylaxis or the treatment of a patient infected with a virus.

43. The modulating agent according to any of claims 27 to 34 for the preparation of a medicinal composition for the treatment of a HCV infected patient.

44. A kit for the treatment of a HCV infected patient, comprising:
- one or several modulating agent(s) according to any of claims 27 to 34 or the medicinal composition according to any of claims 38 to 40, and
- instructions including directions for administering at least one dose of the therapeutic substance to a patient in need thereof.

45. An antibody that specifically binds to the modulating agent according to any of claims 27 to 34.

46. The antibody according to claim 45 that is a polyclonal antibody or a monoclonal antibody.

47. A Method of preparation of a polyclonal serum against the modulating agent according to any of claims 27 to 34, comprising the following steps:
- inoculating an animal with the modulating agent according to any of claims 27 to 34, either associated with an adjuvant or adjuvant-free, to induce an immune response;
- collecting and purifying the produced antibodies directed against the polypeptide used for the inoculation.

48. A Method of preparation of monoclonal antibodies against the modulating agent according to any of claims 27 to 34, comprising the following steps:
- inoculating an animal, e.g. a Balb/c mouse, with the modulating agent according to any of claims 27 to 34, to induce an immune response;
- fusing spleen cells from the inoculated animal with myeloma cells to form a hybridoma;
- growing the hybridoma in conditions that allow antibody production;
- collecting and purifying the produced antibodies directed against the polypeptide used for the inoculation.


SEQUENCE LISTING

<110>  INSTITUT PASTEUR

<120>  USE OF A MODULATING AGENT THAT INTERACTS WITH THE PBD OF PLK
       PROTEINS FOR MODULATING IFN INDUCTION

<130>  B6884 _AD/SL/CAL

<160>  44

<170>  PatentIn version 3.3

<210>  1
<211>  1623
<212>  DNA
<213>  Homo sapiens

<220>
<221>  CDS
<222>  (1)..(1623)
<223>  CARDIF - full length sequence

<400>  1
atg ccg ttt gct gaa gac aag acc tat aag tat atc tgc cgc aat ttc      48
agc aat ttt tgc aat gtg gat gtt gta gag att ctg cct tac ctg ccc      96
tgc ctc aca gca aga gac cag gat cga ctg cgg gcc acc tgc aca ctc      144
tca ggg aac cgg gac acc ctc tgg cat ctc ttc aat acc ctt cag cgg      192
cgg ccc ggc tgg gtg gag tac ttc att gcg gca ctg agg ggc tgt gag      240
cta gtt gat ctc gcg gac gaa gtg gcc tct gtc tac gag agc tac cag      288
cct cgg acc tcg gac cgt ccc cca gac cca ctg gag cca ccg tca ctt      336
cct gct gag agg cca ggg ccc ccc aca cct gct gcg gcc cac agc atc      384
ccc tac aac agc tgc aga gag aag gag cca agt tac ccc atg cct gtc      432
cag gag acc cag gcg cca gag tcc cca gga gag aat tca gag caa gcc      480
ctg cag acg ctc agc ccc aga gcc atc cca agg aat cca gat ggt ggc      528
ccc ctg gag tcc tcc tct gac ctg gca gcc ctc agc cct ctg acc tcc 576
agc ggg cat cag gag aag gac aca gaa ctg ggc agt acc cac aca gca 624
ggt gcg acc tcc agc ctc aca cca tcc cgt ggg cct gtg tct cca tct 672
gtc tcc ttc cag ccc ctg gcc cgt tcc acc ccc agg gca agc cgc ttg 720
cct gga ccc aca ggg tca gtt gta tct act ggc acc tcc ttc tcc tcc 768
tca tcc cct ggc ttg gcc tct gca ggg gct gca gag ggt aaa cag ggt 816
gca gag agt gac cag gcc gag cct atc atc tgc tcc agt ggg gca gag 864
gca cct gcc aac tct ctg ccc tcc aaa gtg cct acc acc ttg atg cct 912
gtg aac aca gtg gcc ctg aaa gtg cct gcc aac cca gca tct gtc agc 960
aca gtg ccc tcc aag ttg cca act agc tca aag ccc cct ggt gca gtg 1008
cct tct aat gcg ctc acc aat cca gca cca tcc aaa ttg ccc atc aac 1056
tca acc cgt gct ggc atg gtg cca tcc aaa gtg cct act agc atg gtg 1104
ctc acc aag gtg tct gcc agc aca gtc ccc act gac ggg agc agc aga 1152
aat gag gag acc cca gca gct cca aca ccc gcc ggc gcc act gga ggc 1200
agc tca gcc tgg cta gac agc agc ttt gag aat agg ggc ctt ggg tcg 1248
gag ctg agt aag cct ggc gtg ctg gca tcc cag gta gac agc ccg ttc 1296
tcg ggc tgc ttc gag gat ctt gcc atc agt gcc agc acc tcc ttg ggc 1344
atg ggg ccc tgc cat ggc cca gag gag aat gag tat aag tcc gag ggc 1392
acc ttt ggg atc cac gtg gct gag aac ccc agc atc cag ctc ctg gag 1440
ggc aac cct ggg cca cct gcg gac ccg gat ggc ggc ccc agg cca caa 1488
gcc gac cgg aag ttc cag gag agg gag gtg cca tgc cac agg ccc tca 1536
cct ggg gct ctg tgg ctc cag gtg gct gtg aca ggg gtg ctg gta gtc 1584
aca ctc ctg gtg gtg ctg tac cgg cgg cgt ctg cac tag 1623

<210> 2
<211> 540
<212> PRT
<213> Homo sapiens

<400> 2

<210>  3
<211>  534
<212>  DNA
<213>  Homo sapiens

<220>
<221>  CDS
<222>  (1)..(534)
<223>  CARDIF - amino acid residues 364-540 of SEQ ID NO:01

<400>  3
cct act agc atg gtg ctc acc aag gtg tct gcc agc aca gtc ccc act      48
Pro Thr Ser Met Val Leu Thr Lys Val Ser Ala Ser Thr Val Pro Thr
1               5                   10                  15

gac ggg agc agc aga aat gag gag acc cca gca gct cca aca ccc gcc      96
Asp Gly Ser Ser Arg Asn Glu Glu Thr Pro Ala Ala Pro Thr Pro Ala
            20                  25                  30

ggc gcc act gga ggc agc tca gcc tgg cta gac agc agc ttt gag aat     144
Gly Ala Thr Gly Gly Ser Ser Ala Trp Leu Asp Ser Ser Phe Glu Asn
        35                  40                  45

agg ggc ctt ggg tcg gag ctg agt aag cct ggc gtg ctg gca tcc cag     192
Arg Gly Leu Gly Ser Glu Leu Ser Lys Pro Gly Val Leu Ala Ser Gln
    50                  55                  60

gta gac agc ccg ttc tcg ggc tgc ttc gag gat ctt gcc atc agt gcc     240
Val Asp Ser Pro Phe Ser Gly Cys Phe Glu Asp Leu Ala Ile Ser Ala
65                  70                  75                  80

agc acc tcc ttg ggc atg ggg ccc tgc cat ggc cca gag gag aat gag     288
Ser Thr Ser Leu Gly Met Gly Pro Cys His Gly Pro Glu Glu Asn Glu
                85                  90                  95

tat aag tcc gag ggc acc ttt ggg atc cac gtg gct gag aac ccc agc 336
Tyr Lys Ser Glu Gly Thr Phe Gly Ile His Val Ala Glu Asn Pro Ser
            100                 105                 110

atc cag ctc ctg gag ggc aac cct ggg cca cct gcg gac ccg gat ggc 384
Ile Gln Leu Leu Glu Gly Asn Pro Gly Pro Pro Ala Asp Pro Asp Gly
        115                 120                 125

ggc ccc agg cca caa gcc gac cgg aag ttc cag gag agg gag gtg cca 432
Gly Pro Arg Pro Gln Ala Asp Arg Lys Phe Gln Glu Arg Glu Val Pro
    130                 135                 140

tgc cac agg ccc tca cct ggg gct ctg tgg ctc cag gtg gct gtg aca 480
Cys His Arg Pro Ser Pro Gly Ala Leu Trp Leu Gln Val Ala Val Thr
145                 150                 155                 160

ggg gtg ctg gta gtc aca ctc ctg gtg gtg ctg tac cgg cgg cgt ctg 528
Gly Val Leu Val Val Thr Leu Leu Val Val Leu Tyr Arg Arg Arg Leu
                165                 170                 175

cac tag 534
His

<210> 4
<211> 177
<212> PRT
<213> Homo sapiens

<400> 4

Pro Thr Ser Met Val Leu Thr Lys Val Ser Ala Ser Thr Val Pro Thr
1               5                   10                  15

Asp Gly Ser Ser Arg Asn Glu Glu Thr Pro Ala Ala Pro Thr Pro Ala
            20                  25                  30

Gly Ala Thr Gly Gly Ser Ser Ala Trp Leu Asp Ser Ser Phe Glu Asn
        35                  40                  45

Arg Gly Leu Gly Ser Glu Leu Ser Lys Pro Gly Val Leu Ala Ser Gln
    50                  55                  60

Val Asp Ser Pro Phe Ser Gly Cys Phe Glu Asp Leu Ala Ile Ser Ala
65                  70                  75                  80

Ser Thr Ser Leu Gly Met Gly Pro Cys His Gly Pro Glu Glu Asn Glu
                85                  90                  95

Tyr Lys Ser Glu Gly Thr Phe Gly Ile His Val Ala Glu Asn Pro Ser
            100                 105                 110

Ile Gln Leu Leu Glu Gly Asn Pro Gly Pro Pro Ala Asp Pro Asp Gly
        115                 120                 125

Gly Pro Arg Pro Gln Ala Asp Arg Lys Phe Gln Glu Arg Glu Val Pro
    130                 135                 140

Cys His Arg Pro Ser Pro Gly Ala Leu Trp Leu Gln Val Ala Val Thr
145                 150                 155                 160

Gly Val Leu Val Val Thr Leu Leu Val Val Leu Tyr Arg Arg Arg Leu
                165                 170                 175

His

<210>  5
<211>  321
<212>  DNA
<213>  Homo sapiens

<220>
<221>  CDS
<222>  (1)..(321)
<223>  CARDIF - amino acid residues 364-470 of SEQ ID NO:01

<400>  5
cct act agc atg gtg ctc acc aag gtg tct gcc agc aca gtc ccc act      48
Pro Thr Ser Met Val Leu Thr Lys Val Ser Ala Ser Thr Val Pro Thr
1               5                   10                  15

gac ggg agc agc aga aat gag gag acc cca gca gct cca aca ccc gcc      96
Asp Gly Ser Ser Arg Asn Glu Glu Thr Pro Ala Ala Pro Thr Pro Ala
            20                  25                  30

ggc gcc act gga ggc agc tca gcc tgg cta gac agc agc ttt gag aat      144
Gly Ala Thr Gly Gly Ser Ser Ala Trp Leu Asp Ser Ser Phe Glu Asn
        35                  40                  45

agg ggc ctt ggg tcg gag ctg agt aag cct ggc gtg ctg gca tcc cag      192
Arg Gly Leu Gly Ser Glu Leu Ser Lys Pro Gly Val Leu Ala Ser Gln
    50                  55                  60

gta gac agc ccg ttc tcg ggc tgc ttc gag gat ctt gcc atc agt gcc      240
Val Asp Ser Pro Phe Ser Gly Cys Phe Glu Asp Leu Ala Ile Ser Ala
65                  70                  75                  80

agc acc tcc ttg ggc atg ggg ccc tgc cat ggc cca gag gag aat gag      288
Ser Thr Ser Leu Gly Met Gly Pro Cys His Gly Pro Glu Glu Asn Glu
                85                  90                  95

tat aag tcc gag ggc acc ttt ggg atc cac gtg                          321
Tyr Lys Ser Glu Gly Thr Phe Gly Ile His Val
            100                 105

<210>  6
<211>  107
<212>  PRT
<213>  Homo sapiens

<400>  6

Pro Thr Ser Met Val Leu Thr Lys Val Ser Ala Ser Thr Val Pro Thr
1               5                   10                  15

Asp Gly Ser Ser Arg Asn Glu Glu Thr Pro Ala Ala Pro Thr Pro Ala
            20                  25                  30

Gly Ala Thr Gly Gly Ser Ser Ala Trp Leu Asp Ser Ser Phe Glu Asn
        35                  40                  45

Arg Gly Leu Gly Ser Glu Leu Ser Lys Pro Gly Val Leu Ala Ser Gln
    50                  55                  60

Val Asp Ser Pro Phe Ser Gly Cys Phe Glu Asp Leu Ala Ile Ser Ala
65                  70                  75                  80

Ser Thr Ser Leu Gly Met Gly Pro Cys His Gly Pro Glu Glu Asn Glu
                85                  90                  95

Tyr Lys Ser Glu Gly Thr Phe Gly Ile His Val
            100                 105

<210>  7
<211>  3
<212>  PRT
<213>  Artificial

<220>
<223>  peptide

<400>  7

Ser Thr Pro
1

<210>  8
<211>  9
<212>  DNA
<213>  Homo sapiens

<400>  8
tccaccccc                                                                9

<210>  9
<211>  11
<212>  PRT
<213>  Artificial

<220>
<223>  peptide

<400>  9

Ser Thr Val Pro Ser Lys Leu Pro Thr Ser Ser
1               5                   10

<210>  10
<211>  33
<212>  DNA
<213>  Homo sapiens

<400>  10
agcacagtgc cctccaagtt gccaactagc tca                                    33

<210>  11

<211>  7
<212>  PRT
<213>  Artificial

<220>
<223>  peptide

<400>  11

Pro Ile Asn Ser Thr Arg Ala
1               5


<210>  12
<211>  21
<212>  DNA
<213>  Homo sapiens CARDIF

<400>  12
cccatcaact caacccgtgc t                                                21


<210>  13
<211>  6
<212>  PRT
<213>  Artificial

<220>
<223>  peptide

<400>  13

Ser Thr Val Pro Thr Asp
1               5


<210>  14
<211>  18
<212>  DNA
<213>  Homo sapiens

<400>  14
agcacagtcc ccactgac                                                    18


<210>  15
<211>  8
<212>  PRT
<213>  Artificial

<220>
<223>  peptide

<400>  15

Ser Thr Ser Leu Gly Met Gly Pro
1               5


<210>  16
<211>  24
<212>  DNA
<213>  Homo sapiens

<400>  16
agcacctcct tgggcatggg gccc                                             24

<210> 17
<211> 459
<212> DNA
<213> Homo sapiens

<220>
<221> CDS
<222> (1)..(459)
<223> CARDIF - amino acid residues 1-153 of SEQ ID NO:01

<400> 17
atg ccg ttt gct gaa gac aag acc tat aag tat atc tgc cgc aat ttc 48
Met Pro Phe Ala Glu Asp Lys Thr Tyr Lys Tyr Ile Cys Arg Asn Phe
1               5                   10                  15

agc aat ttt tgc aat gtg gat gtt gta gag att ctg cct tac ctg ccc 96
Ser Asn Phe Cys Asn Val Asp Val Val Glu Ile Leu Pro Tyr Leu Pro
            20                  25                  30

tgc ctc aca gca aga gac cag gat cga ctg cgg gcc acc tgc aca ctc 144
Cys Leu Thr Ala Arg Asp Gln Asp Arg Leu Arg Ala Thr Cys Thr Leu
        35                  40                  45

tca ggg aac cgg gac acc ctc tgg cat ctc ttc aat acc ctt cag cgg 192
Ser Gly Asn Arg Asp Thr Leu Trp His Leu Phe Asn Thr Leu Gln Arg
    50                  55                  60

cgg ccc ggc tgg gtg gag tac ttc att gcg gca ctg agg ggc tgt gag 240
Arg Pro Gly Trp Val Glu Tyr Phe Ile Ala Ala Leu Arg Gly Cys Glu
65                  70                  75                  80

cta gtt gat ctc gcg gac gaa gtg gcc tct gtc tac gag agc tac cag 288
Leu Val Asp Leu Ala Asp Glu Val Ala Ser Val Tyr Glu Ser Tyr Gln
                85                  90                  95

cct cgg acc tcg gac cgt ccc cca gac cca ctg gag cca ccg tca ctt 336
Pro Arg Thr Ser Asp Arg Pro Pro Asp Pro Leu Glu Pro Pro Ser Leu
            100                 105                 110

cct gct gag agg cca ggg ccc ccc aca cct gct gcg gcc cac agc atc 384
Pro Ala Glu Arg Pro Gly Pro Pro Thr Pro Ala Ala Ala His Ser Ile
        115                 120                 125

ccc tac aac agc tgc aga gag aag gag cca agt tac ccc atg cct gtc 432
Pro Tyr Asn Ser Cys Arg Glu Lys Glu Pro Ser Tyr Pro Met Pro Val
    130                 135                 140

cag gag acc cag gcg cca gag tcc cca 459
Gln Glu Thr Gln Ala Pro Glu Ser Pro
145                 150

<210> 18
<211> 153
<212> PRT
<213> Homo sapiens

<400> 18

Met Pro Phe Ala Glu Asp Lys Thr Tyr Lys Tyr Ile Cys Arg Asn Phe
1               5                   10                  15

Ser Asn Phe Cys Asn Val Asp Val Val Glu Ile Leu Pro Tyr Leu Pro
            20                  25                  30

Cys Leu Thr Ala Arg Asp Gln Asp Arg Leu Arg Ala Thr Cys Thr Leu
        35                  40                  45

Ser Gly Asn Arg Asp Thr Leu Trp His Leu Phe Asn Thr Leu Gln Arg
    50                  55                  60

Arg Pro Gly Trp Val Glu Tyr Phe Ile Ala Ala Leu Arg Gly Cys Glu
65                  70                  75                  80

Leu Val Asp Leu Ala Asp Glu Val Ala Ser Val Tyr Glu Ser Tyr Gln
                85                  90                  95

Pro Arg Thr Ser Asp Arg Pro Pro Asp Pro Leu Glu Pro Pro Ser Leu
            100                 105                 110

Pro Ala Glu Arg Pro Gly Pro Pro Thr Pro Ala Ala Ala His Ser Ile
        115                 120                 125

Pro Tyr Asn Ser Cys Arg Glu Lys Glu Pro Ser Tyr Pro Met Pro Val
    130                 135                 140

Gln Glu Thr Gln Ala Pro Glu Ser Pro
145                 150

<210>  19
<211>  1164
<212>  DNA
<213>  Homo sapiens

<220>
<221>  CDS
<222>  (1)..(1164)
<223>  CARDIF - amino acid residues 154-540 of SEQ ID NO:01

<400>  19
gga gag aat tca gag caa gcc ctg cag acg ctc agc ccc aga gcc atc      48
Gly Glu Asn Ser Glu Gln Ala Leu Gln Thr Leu Ser Pro Arg Ala Ile
1               5                   10                  15

cca agg aat cca gat ggt ggc ccc ctg gag tcc tcc tct gac ctg gca      96
Pro Arg Asn Pro Asp Gly Gly Pro Leu Glu Ser Ser Ser Asp Leu Ala
            20                  25                  30

gcc ctc agc cct ctg acc tcc agc ggg cat cag gag aag gac aca gaa      144
Ala Leu Ser Pro Leu Thr Ser Ser Gly His Gln Glu Lys Asp Thr Glu
        35                  40                  45

ctg ggc agt acc cac aca gca ggt gcg acc tcc agc ctc aca cca tcc      192
Leu Gly Ser Thr His Thr Ala Gly Ala Thr Ser Ser Leu Thr Pro Ser
    50                  55                  60

cgt ggg cct gtg tct cca tct gtc tcc ttc cag ccc ctg gcc cgt tcc      240
Arg Gly Pro Val Ser Pro Ser Val Ser Phe Gln Pro Leu Ala Arg Ser
65                  70                  75                  80

acc ccc agg gca agc cgc ttg cct gga ccc aca ggg tca gtt gta tct      288
Thr Pro Arg Ala Ser Arg Leu Pro Gly Pro Thr Gly Ser Val Val Ser
                85                  90                  95

act ggc acc tcc ttc tcc tcc tca tcc cct ggc ttg gcc tct gca ggg 336
Thr Gly Thr Ser Phe Ser Ser Ser Ser Pro Gly Leu Ala Ser Ala Gly
            100                 105                 110

gct gca gag ggt aaa cag ggt gca gag agt gac cag gcc gag cct atc 384
Ala Ala Glu Gly Lys Gln Gly Ala Glu Ser Asp Gln Ala Glu Pro Ile
        115                 120                 125

atc tgc tcc agt ggg gca gag gca cct gcc aac tct ctg ccc tcc aaa 432
Ile Cys Ser Ser Gly Ala Glu Ala Pro Ala Asn Ser Leu Pro Ser Lys
    130                 135                 140

gtg cct acc acc ttg atg cct gtg aac aca gtg gcc ctg aaa gtg cct 480
Val Pro Thr Thr Leu Met Pro Val Asn Thr Val Ala Leu Lys Val Pro
145                 150                 155                 160

gcc aac cca gca tct gtc agc aca gtg ccc tcc aag ttg cca act agc 528
Ala Asn Pro Ala Ser Val Ser Thr Val Pro Ser Lys Leu Pro Thr Ser
                165                 170                 175

tca aag ccc cct ggt gca gtg cct tct aat gcg ctc acc aat cca gca 576
Ser Lys Pro Pro Gly Ala Val Pro Ser Asn Ala Leu Thr Asn Pro Ala
            180                 185                 190

cca tcc aaa ttg ccc atc aac tca acc cgt gct ggc atg gtg cca tcc 624
Pro Ser Lys Leu Pro Ile Asn Ser Thr Arg Ala Gly Met Val Pro Ser
        195                 200                 205

aaa gtg cct act agc atg gtg ctc acc aag gtg tct gcc agc aca gtc 672
Lys Val Pro Thr Ser Met Val Leu Thr Lys Val Ser Ala Ser Thr Val
    210                 215                 220

ccc act gac ggg agc agc aga aat gag gag acc cca gca gct cca aca 720
Pro Thr Asp Gly Ser Ser Arg Asn Glu Glu Thr Pro Ala Ala Pro Thr
225                 230                 235                 240

ccc gcc ggc gcc act gga ggc agc tca gcc tgg cta gac agc agc ttt 768
Pro Ala Gly Ala Thr Gly Gly Ser Ser Ala Trp Leu Asp Ser Ser Phe
                245                 250                 255

gag aat agg ggc ctt ggg tcg gag ctg agt aag cct ggc gtg ctg gca 816
Glu Asn Arg Gly Leu Gly Ser Glu Leu Ser Lys Pro Gly Val Leu Ala
            260                 265                 270

tcc cag gta gac agc ccg ttc tcg ggc tgc ttc gag gat ctt gcc atc 864
Ser Gln Val Asp Ser Pro Phe Ser Gly Cys Phe Glu Asp Leu Ala Ile
        275                 280                 285

agt gcc agc acc tcc ttg ggc atg ggg ccc tgc cat ggc cca gag gag 912
Ser Ala Ser Thr Ser Leu Gly Met Gly Pro Cys His Gly Pro Glu Glu
    290                 295                 300

aat gag tat aag tcc gag ggc acc ttt ggg atc cac gtg gct gag aac 960
Asn Glu Tyr Lys Ser Glu Gly Thr Phe Gly Ile His Val Ala Glu Asn
305                 310                 315                 320

ccc agc atc cag ctc ctg gag ggc aac cct ggg cca cct gcg gac ccg 1008
Pro Ser Ile Gln Leu Leu Glu Gly Asn Pro Gly Pro Pro Ala Asp Pro
                325                 330                 335

gat ggc ggc ccc agg cca caa gcc gac cgg aag ttc cag gag agg gag 1056
Asp Gly Gly Pro Arg Pro Gln Ala Asp Arg Lys Phe Gln Glu Arg Glu
            340                 345                 350

gtg cca tgc cac agg ccc tca cct ggg gct ctg tgg ctc cag gtg gct 1104
Val Pro Cys His Arg Pro Ser Pro Gly Ala Leu Trp Leu Gln Val Ala
        355                 360                 365

gtg aca ggg gtg ctg gta gtc aca ctc ctg gtg gtg ctg tac cgg cgg     1152
Val Thr Gly Val Leu Val Val Thr Leu Leu Val Val Leu Tyr Arg Arg
    370                 375                 380

cgt ctg cac tag                                                     1164
Arg Leu His
385

<210>  20
<211>  387
<212>  PRT
<213>  Homo sapiens

<400>  20

Gly Glu Asn Ser Glu Gln Ala Leu Gln Thr Leu Ser Pro Arg Ala Ile
1               5                   10                  15

Pro Arg Asn Pro Asp Gly Gly Pro Leu Glu Ser Ser Ser Asp Leu Ala
            20                  25                  30

Ala Leu Ser Pro Leu Thr Ser Ser Gly His Gln Glu Lys Asp Thr Glu
        35                  40                  45

Leu Gly Ser Thr His Thr Ala Gly Ala Thr Ser Ser Leu Thr Pro Ser
    50                  55                  60

Arg Gly Pro Val Ser Pro Ser Val Ser Phe Gln Pro Leu Ala Arg Ser
65                  70                  75                  80

Thr Pro Arg Ala Ser Arg Leu Pro Gly Pro Thr Gly Ser Val Val Ser
                85                  90                  95

Thr Gly Thr Ser Phe Ser Ser Ser Ser Pro Gly Leu Ala Ser Ala Gly
            100                 105                 110

Ala Ala Glu Gly Lys Gln Gly Ala Glu Ser Asp Gln Ala Glu Pro Ile
        115                 120                 125

Ile Cys Ser Ser Gly Ala Glu Ala Pro Ala Asn Ser Leu Pro Ser Lys
    130                 135                 140

Val Pro Thr Thr Leu Met Pro Val Asn Thr Val Ala Leu Lys Val Pro
145                 150                 155                 160

Ala Asn Pro Ala Ser Val Ser Thr Val Pro Ser Lys Leu Pro Thr Ser
                165                 170                 175

Ser Lys Pro Pro Gly Ala Val Pro Ser Asn Ala Leu Thr Asn Pro Ala
            180                 185                 190

Pro Ser Lys Leu Pro Ile Asn Ser Thr Arg Ala Gly Met Val Pro Ser
        195                 200                 205

Lys Val Pro Thr Ser Met Val Leu Thr Lys Val Ser Ala Ser Thr Val
    210                 215                 220

Pro Thr Asp Gly Ser Ser Arg Asn Glu Glu Thr Pro Ala Ala Pro Thr
225                 230                 235                 240

Pro Ala Gly Ala Thr Gly Gly Ser Ser Ala Trp Leu Asp Ser Ser Phe
                245                 250                 255

Glu Asn Arg Gly Leu Gly Ser Glu Leu Ser Lys Pro Gly Val Leu Ala
            260                 265                 270

Ser Gln Val Asp Ser Pro Phe Ser Gly Cys Phe Glu Asp Leu Ala Ile
        275                 280                 285

Ser Ala Ser Thr Ser Leu Gly Met Gly Pro Cys His Gly Pro Glu Glu
    290                 295                 300

Asn Glu Tyr Lys Ser Glu Gly Thr Phe Gly Ile His Val Ala Glu Asn
305                 310                 315                 320

Pro Ser Ile Gln Leu Leu Glu Gly Asn Pro Gly Pro Pro Ala Asp Pro
                325                 330                 335

Asp Gly Gly Pro Arg Pro Gln Ala Asp Arg Lys Phe Gln Glu Arg Glu
            340                 345                 350

Val Pro Cys His Arg Pro Ser Pro Gly Ala Leu Trp Leu Gln Val Ala
        355                 360                 365

Val Thr Gly Val Leu Val Val Thr Leu Leu Val Val Leu Tyr Arg Arg
    370                 375                 380

Arg Leu His
385

<210>  21
<211>  216
<212>  DNA
<213>  Homo sapiens

<220>
<221>  CDS
<222>  (1)..(216)
<223>  CARDIF - amino acid residues 470-540 of SEQ ID NO:01

<400>  21
gtg gct gag aac ccc agc atc cag ctc ctg gag ggc aac cct ggg cca      48
Val Ala Glu Asn Pro Ser Ile Gln Leu Leu Glu Gly Asn Pro Gly Pro
1               5                   10                  15

cct gcg gac ccg gat ggc ggc ccc agg cca caa gcc gac cgg aag ttc      96
Pro Ala Asp Pro Asp Gly Gly Pro Arg Pro Gln Ala Asp Arg Lys Phe
            20                  25                  30

cag gag agg gag gtg cca tgc cac agg ccc tca cct ggg gct ctg tgg 144
Gln Glu Arg Glu Val Pro Cys His Arg Pro Ser Pro Gly Ala Leu Trp
        35                  40                  45

ctc cag gtg gct gtg aca ggg gtg ctg gta gtc aca ctc ctg gtg gtg 192
Leu Gln Val Ala Val Thr Gly Val Leu Val Val Thr Leu Leu Val Val
    50                  55                  60

ctg tac cgg cgg cgt ctg cac tag 216
Leu Tyr Arg Arg Arg Leu His
65                  70

<210> 22
<211> 71
<212> PRT
<213> Homo sapiens

<400> 22

Val Ala Glu Asn Pro Ser Ile Gln Leu Leu Glu Gly Asn Pro Gly Pro
1               5                   10                  15

Pro Ala Asp Pro Asp Gly Gly Pro Arg Pro Gln Ala Asp Arg Lys Phe
            20                  25                  30

Gln Glu Arg Glu Val Pro Cys His Arg Pro Ser Pro Gly Ala Leu Trp
        35                  40                  45

Leu Gln Val Ala Val Thr Gly Val Leu Val Val Thr Leu Leu Val Val
    50                  55                  60

Leu Tyr Arg Arg Arg Leu His
65                  70

<210> 23
<211> 420
<212> DNA
<213> Homo sapiens

<220>
<221> CDS
<222> (1)..(420)
<223> CARDIF - amino acid residues 364-503 of SEQ ID NO:01

<400> 23
cct act agc atg gtg ctc acc aag gtg tct gcc agc aca gtc ccc act 48
Pro Thr Ser Met Val Leu Thr Lys Val Ser Ala Ser Thr Val Pro Thr
1               5                   10                  15

gac ggg agc agc aga aat gag gag acc cca gca gct cca aca ccc gcc 96
Asp Gly Ser Ser Arg Asn Glu Glu Thr Pro Ala Ala Pro Thr Pro Ala
            20                  25                  30

ggc gcc act gga ggc agc tca gcc tgg cta gac agc agc ttt gag aat 144
Gly Ala Thr Gly Gly Ser Ser Ala Trp Leu Asp Ser Ser Phe Glu Asn
        35                  40                  45

agg ggc ctt ggg tcg gag ctg agt aag cct ggc gtg ctg gca tcc cag 192
Arg Gly Leu Gly Ser Glu Leu Ser Lys Pro Gly Val Leu Ala Ser Gln
    50                  55                  60

gta gac agc ccg ttc tcg ggc tgc ttc gag gat ctt gcc atc agt gcc 240
Val Asp Ser Pro Phe Ser Gly Cys Phe Glu Asp Leu Ala Ile Ser Ala
65                  70                  75                  80

agc acc tcc ttg ggc atg ggg ccc tgc cat ggc cca gag gag aat gag 288
Ser Thr Ser Leu Gly Met Gly Pro Cys His Gly Pro Glu Glu Asn Glu
                85                  90                  95

tat aag tcc gag ggc acc ttt ggg atc cac gtg gct gag aac ccc agc 336
Tyr Lys Ser Glu Gly Thr Phe Gly Ile His Val Ala Glu Asn Pro Ser
            100                 105                 110

atc cag ctc ctg gag ggc aac cct ggg cca cct gcg gac ccg gat ggc 384
Ile Gln Leu Leu Glu Gly Asn Pro Gly Pro Pro Ala Asp Pro Asp Gly
        115                 120                 125

ggc ccc agg cca caa gcc gac cgg aag ttc cag gag 420
Gly Pro Arg Pro Gln Ala Asp Arg Lys Phe Gln Glu
    130                 135                 140

<210> 24
<211> 140
<212> PRT
<213> Homo sapiens

<400> 24

Pro Thr Ser Met Val Leu Thr Lys Val Ser Ala Ser Thr Val Pro Thr
1               5                   10                  15

Asp Gly Ser Ser Arg Asn Glu Glu Thr Pro Ala Ala Pro Thr Pro Ala
            20                  25                  30

Gly Ala Thr Gly Gly Ser Ser Ala Trp Leu Asp Ser Ser Phe Glu Asn
        35                  40                  45

Arg Gly Leu Gly Ser Glu Leu Ser Lys Pro Gly Val Leu Ala Ser Gln
    50                  55                  60

Val Asp Ser Pro Phe Ser Gly Cys Phe Glu Asp Leu Ala Ile Ser Ala
65                  70                  75                  80

Ser Thr Ser Leu Gly Met Gly Pro Cys His Gly Pro Glu Glu Asn Glu
                85                  90                  95

Tyr Lys Ser Glu Gly Thr Phe Gly Ile His Val Ala Glu Asn Pro Ser
            100                 105                 110

Ile Gln Leu Leu Glu Gly Asn Pro Gly Pro Pro Ala Asp Pro Asp Gly
        115                 120                 125

Gly Pro Arg Pro Gln Ala Asp Arg Lys Phe Gln Glu
    130                 135                 140

<210> 25
<211> 768
<212> DNA
<213> Homo sapiens

<220>
<221> CDS
<222> (1)..(768)
<223> CARDIF - amino acid residues 1-256 of SEQ ID NO:01

<400> 25
atg ccg ttt gct gaa gac aag acc tat aag tat atc tgc cgc aat ttc 48
Met Pro Phe Ala Glu Asp Lys Thr Tyr Lys Tyr Ile Cys Arg Asn Phe
1               5                   10                  15

agc aat ttt tgc aat gtg gat gtt gta gag att ctg cct tac ctg ccc 96
Ser Asn Phe Cys Asn Val Asp Val Val Glu Ile Leu Pro Tyr Leu Pro
            20                  25                  30

tgc ctc aca gca aga gac cag gat cga ctg cgg gcc acc tgc aca ctc 144
Cys Leu Thr Ala Arg Asp Gln Asp Arg Leu Arg Ala Thr Cys Thr Leu
        35                  40                  45

tca ggg aac cgg gac acc ctc tgg cat ctc ttc aat acc ctt cag cgg 192
Ser Gly Asn Arg Asp Thr Leu Trp His Leu Phe Asn Thr Leu Gln Arg
    50                  55                  60

cgg ccc ggc tgg gtg gag tac ttc att gcg gca ctg agg ggc tgt gag 240
Arg Pro Gly Trp Val Glu Tyr Phe Ile Ala Ala Leu Arg Gly Cys Glu
65                  70                  75                  80

cta gtt gat ctc gcg gac gaa gtg gcc tct gtc tac gag agc tac cag 288
Leu Val Asp Leu Ala Asp Glu Val Ala Ser Val Tyr Glu Ser Tyr Gln
                85                  90                  95

cct cgg acc tcg gac cgt ccc cca gac cca ctg gag cca ccg tca ctt 336
Pro Arg Thr Ser Asp Arg Pro Pro Asp Pro Leu Glu Pro Pro Ser Leu
            100                 105                 110

cct gct gag agg cca ggg ccc ccc aca cct gct gcg gcc cac agc atc 384
Pro Ala Glu Arg Pro Gly Pro Pro Thr Pro Ala Ala Ala His Ser Ile
        115                 120                 125

ccc tac aac agc tgc aga gag aag gag cca agt tac ccc atg cct gtc 432
Pro Tyr Asn Ser Cys Arg Glu Lys Glu Pro Ser Tyr Pro Met Pro Val
    130                 135                 140

cag gag acc cag gcg cca gag tcc cca gga gag aat tca gag caa gcc 480
Gln Glu Thr Gln Ala Pro Glu Ser Pro Gly Glu Asn Ser Glu Gln Ala
145                 150                 155                 160

ctg cag acg ctc agc ccc aga gcc atc cca agg aat cca gat ggt ggc 528
Leu Gln Thr Leu Ser Pro Arg Ala Ile Pro Arg Asn Pro Asp Gly Gly
                165                 170                 175

ccc ctg gag tcc tcc tct gac ctg gca gcc ctc agc cct ctg acc tcc 576
Pro Leu Glu Ser Ser Ser Asp Leu Ala Ala Leu Ser Pro Leu Thr Ser
            180                 185                 190

agc ggg cat cag gag aag gac aca gaa ctg ggc agt acc cac aca gca 624
Ser Gly His Gln Glu Lys Asp Thr Glu Leu Gly Ser Thr His Thr Ala
        195                 200                 205

ggt gcg acc tcc agc ctc aca cca tcc cgt ggg cct gtg tct cca tct 672
Gly Ala Thr Ser Ser Leu Thr Pro Ser Arg Gly Pro Val Ser Pro Ser
    210                 215                 220

gtc tcc ttc cag ccc ctg gcc cgt tcc acc ccc agg gca agc cgc ttg 720
Val Ser Phe Gln Pro Leu Ala Arg Ser Thr Pro Arg Ala Ser Arg Leu
225                 230                 235                 240

cct gga ccc aca ggg tca gtt gta tct act ggc acc tcc ttc tcc tcc 768
Pro Gly Pro Thr Gly Ser Val Val Ser Thr Gly Thr Ser Phe Ser Ser
                245                 250                 255

<210> 26
<211> 256
<212> PRT
<213> Homo sapiens

<400> 26

Met Pro Phe Ala Glu Asp Lys Thr Tyr Lys Tyr Ile Cys Arg Asn Phe
1               5                   10                  15

Ser Asn Phe Cys Asn Val Asp Val Val Glu Ile Leu Pro Tyr Leu Pro
            20                  25                  30

Cys Leu Thr Ala Arg Asp Gln Asp Arg Leu Arg Ala Thr Cys Thr Leu
        35                  40                  45

Ser Gly Asn Arg Asp Thr Leu Trp His Leu Phe Asn Thr Leu Gln Arg
    50                  55                  60

Arg Pro Gly Trp Val Glu Tyr Phe Ile Ala Ala Leu Arg Gly Cys Glu
65                  70                  75                  80

Leu Val Asp Leu Ala Asp Glu Val Ala Ser Val Tyr Glu Ser Tyr Gln
                85                  90                  95

Pro Arg Thr Ser Asp Arg Pro Pro Asp Pro Leu Glu Pro Pro Ser Leu
            100                 105                 110

Pro Ala Glu Arg Pro Gly Pro Pro Thr Pro Ala Ala Ala His Ser Ile
        115                 120                 125

Pro Tyr Asn Ser Cys Arg Glu Lys Glu Pro Ser Tyr Pro Met Pro Val
    130                 135                 140

Gln Glu Thr Gln Ala Pro Glu Ser Pro Gly Glu Asn Ser Glu Gln Ala
145                 150                 155                 160

Leu Gln Thr Leu Ser Pro Arg Ala Ile Pro Arg Asn Pro Asp Gly Gly
                165                 170                 175

Pro Leu Glu Ser Ser Ser Asp Leu Ala Ala Leu Ser Pro Leu Thr Ser
            180                 185                 190

Ser Gly His Gln Glu Lys Asp Thr Glu Leu Gly Ser Thr His Thr Ala
        195                 200                 205

Gly Ala Thr Ser Ser Leu Thr Pro Ser Arg Gly Pro Val Ser Pro Ser
    210                 215                 220

Val Ser Phe Gln Pro Leu Ala Arg Ser Thr Pro Arg Ala Ser Arg Leu
225                 230                 235                 240

Pro Gly Pro Thr Gly Ser Val Val Ser Thr Gly Thr Ser Phe Ser Ser
                245                 250                 255

<210>  27
<211>  1080
<212>  DNA
<213>  Homo sapiens

<220>
<221>  CDS
<222>  (1)..(1080)
<223>  CARDIF - amino acid residues 1-360 of SEQ ID NO:01

<400>  27
atg ccg ttt gct gaa gac aag acc tat aag tat atc tgc cgc aat ttc      48
Met Pro Phe Ala Glu Asp Lys Thr Tyr Lys Tyr Ile Cys Arg Asn Phe
1               5                   10                  15

agc aat ttt tgc aat gtg gat gtt gta gag att ctg cct tac ctg ccc      96
Ser Asn Phe Cys Asn Val Asp Val Val Glu Ile Leu Pro Tyr Leu Pro
            20                  25                  30

tgc ctc aca gca aga gac cag gat cga ctg cgg gcc acc tgc aca ctc      144
Cys Leu Thr Ala Arg Asp Gln Asp Arg Leu Arg Ala Thr Cys Thr Leu
        35                  40                  45

tca ggg aac cgg gac acc ctc tgg cat ctc ttc aat acc ctt cag cgg      192
Ser Gly Asn Arg Asp Thr Leu Trp His Leu Phe Asn Thr Leu Gln Arg
    50                  55                  60

cgg ccc ggc tgg gtg gag tac ttc att gcg gca ctg agg ggc tgt gag      240
Arg Pro Gly Trp Val Glu Tyr Phe Ile Ala Ala Leu Arg Gly Cys Glu
65                  70                  75                  80

cta gtt gat ctc gcg gac gaa gtg gcc tct gtc tac gag agc tac cag      288
Leu Val Asp Leu Ala Asp Glu Val Ala Ser Val Tyr Glu Ser Tyr Gln
                85                  90                  95

cct cgg acc tcg gac cgt ccc cca gac cca ctg gag cca ccg tca ctt      336
Pro Arg Thr Ser Asp Arg Pro Pro Asp Pro Leu Glu Pro Pro Ser Leu
            100                 105                 110

cct gct gag agg cca ggg ccc ccc aca cct gct gcg gcc cac agc atc      384
Pro Ala Glu Arg Pro Gly Pro Pro Thr Pro Ala Ala Ala His Ser Ile
        115                 120                 125

ccc tac aac agc tgc aga gag aag gag cca agt tac ccc atg cct gtc      432
Pro Tyr Asn Ser Cys Arg Glu Lys Glu Pro Ser Tyr Pro Met Pro Val
    130                 135                 140

cag gag acc cag gcg cca gag tcc cca gga gag aat tca gag caa gcc      480
Gln Glu Thr Gln Ala Pro Glu Ser Pro Gly Glu Asn Ser Glu Gln Ala
145                 150                 155                 160

ctg cag acg ctc agc ccc aga gcc atc cca agg aat cca gat ggt ggc      528
Leu Gln Thr Leu Ser Pro Arg Ala Ile Pro Arg Asn Pro Asp Gly Gly
                165                 170                 175

ccc ctg gag tcc tcc tct gac ctg gca gcc ctc agc cct ctg acc tcc      576
Pro Leu Glu Ser Ser Ser Asp Leu Ala Ala Leu Ser Pro Leu Thr Ser
            180                 185                 190

agc ggg cat cag gag aag gac aca gaa ctg ggc agt acc cac aca gca     624
Ser Gly His Gln Glu Lys Asp Thr Glu Leu Gly Ser Thr His Thr Ala
        195                 200                 205

ggt gcg acc tcc agc ctc aca cca tcc cgt ggg cct gtg tct cca tct     672
Gly Ala Thr Ser Ser Leu Thr Pro Ser Arg Gly Pro Val Ser Pro Ser
    210                 215                 220

gtc tcc ttc cag ccc ctg gcc cgt tcc acc ccc agg gca agc cgc ttg     720
Val Ser Phe Gln Pro Leu Ala Arg Ser Thr Pro Arg Ala Ser Arg Leu
225                 230                 235                 240

cct gga ccc aca ggg tca gtt gta tct act ggc acc tcc ttc tcc tcc     768
Pro Gly Pro Thr Gly Ser Val Val Ser Thr Gly Thr Ser Phe Ser Ser
                245                 250                 255

tca tcc cct ggc ttg gcc tct gca ggg gct gca gag ggt aaa cag ggt     816
Ser Ser Pro Gly Leu Ala Ser Ala Gly Ala Ala Glu Gly Lys Gln Gly
            260                 265                 270

gca gag agt gac cag gcc gag cct atc atc tgc tcc agt ggg gca gag     864
Ala Glu Ser Asp Gln Ala Glu Pro Ile Ile Cys Ser Ser Gly Ala Glu
        275                 280                 285

gca cct gcc aac tct ctg ccc tcc aaa gtg cct acc acc ttg atg cct     912
Ala Pro Ala Asn Ser Leu Pro Ser Lys Val Pro Thr Thr Leu Met Pro
    290                 295                 300

gtg aac aca gtg gcc ctg aaa gtg cct gcc aac cca gca tct gtc agc     960
Val Asn Thr Val Ala Leu Lys Val Pro Ala Asn Pro Ala Ser Val Ser
305                 310                 315                 320

aca gtg ccc tcc aag ttg cca act agc tca aag ccc cct ggt gca gtg     1008
Thr Val Pro Ser Lys Leu Pro Thr Ser Ser Lys Pro Pro Gly Ala Val
                325                 330                 335

cct tct aat gcg ctc acc aat cca gca cca tcc aaa ttg ccc atc aac     1056
Pro Ser Asn Ala Leu Thr Asn Pro Ala Pro Ser Lys Leu Pro Ile Asn
            340                 345                 350

tca acc cgt gct ggc atg gtg cca                                     1080
Ser Thr Arg Ala Gly Met Val Pro
        355                 360

<210>  28
<211>  360
<212>  PRT
<213>  Homo sapiens

<400>  28

Met Pro Phe Ala Glu Asp Lys Thr Tyr Lys Tyr Ile Cys Arg Asn Phe
1               5                   10                  15

Ser Asn Phe Cys Asn Val Asp Val Val Glu Ile Leu Pro Tyr Leu Pro
            20                  25                  30

Cys Leu Thr Ala Arg Asp Gln Asp Arg Leu Arg Ala Thr Cys Thr Leu
        35                  40                  45

Ser Gly Asn Arg Asp Thr Leu Trp His Leu Phe Asn Thr Leu Gln Arg
    50                  55                  60

Arg Pro Gly Trp Val Glu Tyr Phe Ile Ala Ala Leu Arg Gly Cys Glu
65                  70                  75                  80

Leu Val Asp Leu Ala Asp Glu Val Ala Ser Val Tyr Glu Ser Tyr Gln
                85                  90                  95

Pro Arg Thr Ser Asp Arg Pro Pro Asp Pro Leu Glu Pro Pro Ser Leu
            100                 105                 110

Pro Ala Glu Arg Pro Gly Pro Pro Thr Pro Ala Ala Ala His Ser Ile
        115                 120                 125

Pro Tyr Asn Ser Cys Arg Glu Lys Glu Pro Ser Tyr Pro Met Pro Val
    130                 135                 140

Gln Glu Thr Gln Ala Pro Glu Ser Pro Gly Glu Asn Ser Glu Gln Ala
145                 150                 155                 160

Leu Gln Thr Leu Ser Pro Arg Ala Ile Pro Arg Asn Pro Asp Gly Gly
                165                 170                 175

Pro Leu Glu Ser Ser Ser Asp Leu Ala Ala Leu Ser Pro Leu Thr Ser
            180                 185                 190

Ser Gly His Gln Glu Lys Asp Thr Glu Leu Gly Ser Thr His Thr Ala
        195                 200                 205

Gly Ala Thr Ser Ser Leu Thr Pro Ser Arg Gly Pro Val Ser Pro Ser
    210                 215                 220

Val Ser Phe Gln Pro Leu Ala Arg Ser Thr Pro Arg Ala Ser Arg Leu
225                 230                 235                 240

Pro Gly Pro Thr Gly Ser Val Val Ser Thr Gly Thr Ser Phe Ser Ser
                245                 250                 255

Ser Ser Pro Gly Leu Ala Ser Ala Gly Ala Ala Glu Gly Lys Gln Gly
            260                 265                 270

Ala Glu Ser Asp Gln Ala Glu Pro Ile Ile Cys Ser Ser Gly Ala Glu
        275                 280                 285

Ala Pro Ala Asn Ser Leu Pro Ser Lys Val Pro Thr Thr Leu Met Pro
    290                 295                 300

Val Asn Thr Val Ala Leu Lys Val Pro Ala Asn Pro Ala Ser Val Ser
305                 310                 315                 320

Thr Val Pro Ser Lys Leu Pro Thr Ser Ser Lys Pro Pro Gly Ala Val
                325                 330                 335

Pro Ser Asn Ala Leu Thr Asn Pro Ala Pro Ser Lys Leu Pro Ile Asn
            340                 345                 350

Ser Thr Arg Ala Gly Met Val Pro
        355                 360

<210>  29
<211>  1398
<212>  DNA
<213>  Homo sapiens

<220>
<221>  CDS
<222>  (1)..(1398)
<223>  CARDIF - amino acid residues 1-466 of SEQ ID NO:01

<400>  29
atg ccg ttt gct gaa gac aag acc tat aag tat atc tgc cgc aat ttc      48
Met Pro Phe Ala Glu Asp Lys Thr Tyr Lys Tyr Ile Cys Arg Asn Phe
1               5                   10                  15

agc aat ttt tgc aat gtg gat gtt gta gag att ctg cct tac ctg ccc      96
Ser Asn Phe Cys Asn Val Asp Val Val Glu Ile Leu Pro Tyr Leu Pro
            20                  25                  30

tgc ctc aca gca aga gac cag gat cga ctg cgg gcc acc tgc aca ctc      144
Cys Leu Thr Ala Arg Asp Gln Asp Arg Leu Arg Ala Thr Cys Thr Leu
        35                  40                  45

tca ggg aac cgg gac acc ctc tgg cat ctc ttc aat acc ctt cag cgg      192
Ser Gly Asn Arg Asp Thr Leu Trp His Leu Phe Asn Thr Leu Gln Arg
    50                  55                  60

cgg ccc ggc tgg gtg gag tac ttc att gcg gca ctg agg ggc tgt gag      240
Arg Pro Gly Trp Val Glu Tyr Phe Ile Ala Ala Leu Arg Gly Cys Glu
65                  70                  75                  80

cta gtt gat ctc gcg gac gaa gtg gcc tct gtc tac gag agc tac cag      288
Leu Val Asp Leu Ala Asp Glu Val Ala Ser Val Tyr Glu Ser Tyr Gln
                85                  90                  95

cct cgg acc tcg gac cgt ccc cca gac cca ctg gag cca ccg tca ctt      336
Pro Arg Thr Ser Asp Arg Pro Pro Asp Pro Leu Glu Pro Pro Ser Leu
            100                 105                 110

cct gct gag agg cca ggg ccc ccc aca cct gct gcg gcc cac agc atc      384
Pro Ala Glu Arg Pro Gly Pro Pro Thr Pro Ala Ala Ala His Ser Ile
        115                 120                 125

ccc tac aac agc tgc aga gag aag gag cca agt tac ccc atg cct gtc      432
Pro Tyr Asn Ser Cys Arg Glu Lys Glu Pro Ser Tyr Pro Met Pro Val
    130                 135                 140

cag gag acc cag gcg cca gag tcc cca gga gag aat tca gag caa gcc      480
Gln Glu Thr Gln Ala Pro Glu Ser Pro Gly Glu Asn Ser Glu Gln Ala
145                 150                 155                 160

ctg cag acg ctc agc ccc aga gcc atc cca agg aat cca gat ggt ggc      528
Leu Gln Thr Leu Ser Pro Arg Ala Ile Pro Arg Asn Pro Asp Gly Gly
                165                 170                 175

ccc ctg gag tcc tcc tct gac ctg gca gcc ctc agc cct ctg acc tcc      576
Pro Leu Glu Ser Ser Ser Asp Leu Ala Ala Leu Ser Pro Leu Thr Ser
            180                 185                 190

agc ggg cat cag gag aag gac aca gaa ctg ggc agt acc cac aca gca 624
Ser Gly His Gln Glu Lys Asp Thr Glu Leu Gly Ser Thr His Thr Ala
        195                 200                 205

ggt gcg acc tcc agc ctc aca cca tcc cgt ggg cct gtg tct cca tct 672
Gly Ala Thr Ser Ser Leu Thr Pro Ser Arg Gly Pro Val Ser Pro Ser
    210                 215                 220

gtc tcc ttc cag ccc ctg gcc cgt tcc acc ccc agg gca agc cgc ttg 720
Val Ser Phe Gln Pro Leu Ala Arg Ser Thr Pro Arg Ala Ser Arg Leu
225                 230                 235                 240

cct gga ccc aca ggg tca gtt gta tct act ggc acc tcc ttc tcc tcc 768
Pro Gly Pro Thr Gly Ser Val Val Ser Thr Gly Thr Ser Phe Ser Ser
                245                 250                 255

tca tcc cct ggc ttg gcc tct gca ggg gct gca gag ggt aaa cag ggt 816
Ser Ser Pro Gly Leu Ala Ser Ala Gly Ala Ala Glu Gly Lys Gln Gly
            260                 265                 270

gca gag agt gac cag gcc gag cct atc atc tgc tcc agt ggg gca gag 864
Ala Glu Ser Asp Gln Ala Glu Pro Ile Ile Cys Ser Ser Gly Ala Glu
        275                 280                 285

gca cct gcc aac tct ctg ccc tcc aaa gtg cct acc acc ttg atg cct 912
Ala Pro Ala Asn Ser Leu Pro Ser Lys Val Pro Thr Thr Leu Met Pro
    290                 295                 300

gtg aac aca gtg gcc ctg aaa gtg cct gcc aac cca gca tct gtc agc 960
Val Asn Thr Val Ala Leu Lys Val Pro Ala Asn Pro Ala Ser Val Ser
305                 310                 315                 320

aca gtg ccc tcc aag ttg cca act agc tca aag ccc cct ggt gca gtg 1008
Thr Val Pro Ser Lys Leu Pro Thr Ser Ser Lys Pro Pro Gly Ala Val
                325                 330                 335

cct tct aat gcg ctc acc aat cca gca cca tcc aaa ttg ccc atc aac 1056
Pro Ser Asn Ala Leu Thr Asn Pro Ala Pro Ser Lys Leu Pro Ile Asn
            340                 345                 350

tca acc cgt gct ggc atg gtg cca tcc aaa gtg cct act agc atg gtg 1104
Ser Thr Arg Ala Gly Met Val Pro Ser Lys Val Pro Thr Ser Met Val
        355                 360                 365

ctc acc aag gtg tct gcc agc aca gtc ccc act gac ggg agc agc aga 1152
Leu Thr Lys Val Ser Ala Ser Thr Val Pro Thr Asp Gly Ser Ser Arg
    370                 375                 380

aat gag gag acc cca gca gct cca aca ccc gcc ggc gcc act gga ggc 1200
Asn Glu Glu Thr Pro Ala Ala Pro Thr Pro Ala Gly Ala Thr Gly Gly
385                 390                 395                 400

agc tca gcc tgg cta gac agc agc ttt gag aat agg ggc ctt ggg tcg 1248
Ser Ser Ala Trp Leu Asp Ser Ser Phe Glu Asn Arg Gly Leu Gly Ser
                405                 410                 415

gag ctg agt aag cct ggc gtg ctg gca tcc cag gta gac agc ccg ttc 1296
Glu Leu Ser Lys Pro Gly Val Leu Ala Ser Gln Val Asp Ser Pro Phe
            420                 425                 430

tcg ggc tgc ttc gag gat ctt gcc atc agt gcc agc acc tcc ttg ggc 1344
Ser Gly Cys Phe Glu Asp Leu Ala Ile Ser Ala Ser Thr Ser Leu Gly
        435                 440                 445

atg ggg ccc tgc cat ggc cca gag gag aat gag tat aag tcc gag ggc 1392
Met Gly Pro Cys His Gly Pro Glu Glu Asn Glu Tyr Lys Ser Glu Gly
    450                 455                 460

acc ttt                                                                1398
Thr Phe
465

<210>  30
<211>  466
<212>  PRT
<213>  Homo sapiens

<400>  30

Met Pro Phe Ala Glu Asp Lys Thr Tyr Lys Tyr Ile Cys Arg Asn Phe
1               5                   10                  15

Ser Asn Phe Cys Asn Val Asp Val Val Glu Ile Leu Pro Tyr Leu Pro
            20                  25                  30

Cys Leu Thr Ala Arg Asp Gln Asp Arg Leu Arg Ala Thr Cys Thr Leu
        35                  40                  45

Ser Gly Asn Arg Asp Thr Leu Trp His Leu Phe Asn Thr Leu Gln Arg
    50                  55                  60

Arg Pro Gly Trp Val Glu Tyr Phe Ile Ala Ala Leu Arg Gly Cys Glu
65                  70                  75                  80

Leu Val Asp Leu Ala Asp Glu Val Ala Ser Val Tyr Glu Ser Tyr Gln
                85                  90                  95

Pro Arg Thr Ser Asp Arg Pro Pro Asp Pro Leu Glu Pro Pro Ser Leu
            100                 105                 110

Pro Ala Glu Arg Pro Gly Pro Pro Thr Pro Ala Ala Ala His Ser Ile
        115                 120                 125

Pro Tyr Asn Ser Cys Arg Glu Lys Glu Pro Ser Tyr Pro Met Pro Val
    130                 135                 140

Gln Glu Thr Gln Ala Pro Glu Ser Pro Gly Glu Asn Ser Glu Gln Ala
145                 150                 155                 160

Leu Gln Thr Leu Ser Pro Arg Ala Ile Pro Arg Asn Pro Asp Gly Gly
                165                 170                 175

Pro Leu Glu Ser Ser Ser Asp Leu Ala Ala Leu Ser Pro Leu Thr Ser
            180                 185                 190

Ser Gly His Gln Glu Lys Asp Thr Glu Leu Gly Ser Thr His Thr Ala
        195                 200                 205

Gly Ala Thr Ser Ser Leu Thr Pro Ser Arg Gly Pro Val Ser Pro Ser
    210                 215                 220

Val Ser Phe Gln Pro Leu Ala Arg Ser Thr Pro Arg Ala Ser Arg Leu
225                 230                 235                 240

Pro Gly Pro Thr Gly Ser Val Val Ser Thr Gly Thr Ser Phe Ser Ser
                245                 250                 255

Ser Ser Pro Gly Leu Ala Ser Ala Gly Ala Ala Glu Gly Lys Gln Gly
            260                 265                 270

Ala Glu Ser Asp Gln Ala Glu Pro Ile Ile Cys Ser Ser Gly Ala Glu
        275                 280                 285

Ala Pro Ala Asn Ser Leu Pro Ser Lys Val Pro Thr Thr Leu Met Pro
    290                 295                 300

Val Asn Thr Val Ala Leu Lys Val Pro Ala Asn Pro Ala Ser Val Ser
305                 310                 315                 320

Thr Val Pro Ser Lys Leu Pro Thr Ser Ser Lys Pro Pro Gly Ala Val
                325                 330                 335

Pro Ser Asn Ala Leu Thr Asn Pro Ala Pro Ser Lys Leu Pro Ile Asn
            340                 345                 350

Ser Thr Arg Ala Gly Met Val Pro Ser Lys Val Pro Thr Ser Met Val
        355                 360                 365

Leu Thr Lys Val Ser Ala Ser Thr Val Pro Thr Asp Gly Ser Ser Arg
    370                 375                 380

Asn Glu Glu Thr Pro Ala Ala Pro Thr Pro Ala Gly Ala Thr Gly Gly
385                 390                 395                 400

Ser Ser Ala Trp Leu Asp Ser Ser Phe Glu Asn Arg Gly Leu Gly Ser
                405                 410                 415

Glu Leu Ser Lys Pro Gly Val Leu Ala Ser Gln Val Asp Ser Pro Phe
            420                 425                 430

Ser Gly Cys Phe Glu Asp Leu Ala Ile Ser Ala Ser Thr Ser Leu Gly
        435                 440                 445

Met Gly Pro Cys His Gly Pro Glu Glu Asn Glu Tyr Lys Ser Glu Gly
    450                 455                 460

Thr Phe
465

<210>  31
<211>  2204
<212>  DNA
<213>  Homo sapiens

<220>
<221> CDS
<222> (54)..(1865)
<223> PLK1 - full length sequence

<400> 31
gagcggtgcg gaggctctgc tcggatcgag gtctgcagcg cagcttcggg agc atg 56
Met
1

agt gct gca gtg act gca ggg aag ctg gca cgg gca ccg gcc gac cct 104
Ser Ala Ala Val Thr Ala Gly Lys Leu Ala Arg Ala Pro Ala Asp Pro
            5                   10                  15

ggg aaa gcc ggg gtc ccc gga gtt gca gct ccc gga gct ccg gcg gcg 152
Gly Lys Ala Gly Val Pro Gly Val Ala Ala Pro Gly Ala Pro Ala Ala
        20                  25                  30

gct cca ccg gcg aaa gag atc ccg gag gtc cta gtg gac cca cgc agc 200
Ala Pro Pro Ala Lys Glu Ile Pro Glu Val Leu Val Asp Pro Arg Ser
    35                  40                  45

cgg cgg cgc tat gtg cgg ggc cgc ttt ttg ggc aag ggc ggc ttt gcc 248
Arg Arg Arg Tyr Val Arg Gly Arg Phe Leu Gly Lys Gly Gly Phe Ala
50                  55                  60                  65

aag tgc ttc gag atc tcg gac gcg gac acc aag gag gtg ttc gcg ggc 296
Lys Cys Phe Glu Ile Ser Asp Ala Asp Thr Lys Glu Val Phe Ala Gly
                70                  75                  80

aag att gtg cct aag tct ctg ctg ctc aag ccg cac cag agg gag aag 344
Lys Ile Val Pro Lys Ser Leu Leu Leu Lys Pro His Gln Arg Glu Lys
            85                  90                  95

atg tcc atg gaa ata tcc att cac cgc agc ctc gcc cac cag cac gtc 392
Met Ser Met Glu Ile Ser Ile His Arg Ser Leu Ala His Gln His Val
        100                 105                 110

gta gga ttc cac ggc ttt ttc gag gac aac gac ttc gtg ttc gtg gtg 440
Val Gly Phe His Gly Phe Phe Glu Asp Asn Asp Phe Val Phe Val Val
    115                 120                 125

ttg gag ctc tgc cgc cgg agg tct ctc ctg gag ctg cac aag agg agg 488
Leu Glu Leu Cys Arg Arg Arg Ser Leu Leu Glu Leu His Lys Arg Arg
130                 135                 140                 145

aaa gcc ctg act gag cct gag gcc cga tac tac cta cgg caa att gtg 536
Lys Ala Leu Thr Glu Pro Glu Ala Arg Tyr Tyr Leu Arg Gln Ile Val
                150                 155                 160

ctt ggc tgc cag tac ctg cac cga aac cga gtt att cat cga gac ctc 584
Leu Gly Cys Gln Tyr Leu His Arg Asn Arg Val Ile His Arg Asp Leu
            165                 170                 175

aag ctg ggc aac ctt ttc ctg aat gaa gat ctg gag gtg aaa ata ggg 632
Lys Leu Gly Asn Leu Phe Leu Asn Glu Asp Leu Glu Val Lys Ile Gly
        180                 185                 190

gat ttt gga ctg gca acc aaa gtc gaa tat gac ggg gag agg aag aag 680
Asp Phe Gly Leu Ala Thr Lys Val Glu Tyr Asp Gly Glu Arg Lys Lys
    195                 200                 205

acc ctg tgt ggg act cct aat tac ata gct ccc gag gtg ctg agc aag 728
Thr Leu Cys Gly Thr Pro Asn Tyr Ile Ala Pro Glu Val Leu Ser Lys
210                 215                 220                 225

aaa ggg cac agt ttc gag gtg gat gtg tgg tcc att ggg tgt atc atg 776
Lys Gly His Ser Phe Glu Val Asp Val Trp Ser Ile Gly Cys Ile Met
                230                 235                 240

tat acc ttg tta gtg ggc aaa cca cct ttt gag act tct tgc cta aaa 824
Tyr Thr Leu Leu Val Gly Lys Pro Pro Phe Glu Thr Ser Cys Leu Lys
            245                 250                 255

gag acc tac ctc cgg atc aag aag aat gaa tac agt att ccc aag cac 872
Glu Thr Tyr Leu Arg Ile Lys Lys Asn Glu Tyr Ser Ile Pro Lys His
        260                 265                 270

atc aac ccc gtg gcc gcc tcc ctc atc cag aag atg ctt cag aca gat 920
Ile Asn Pro Val Ala Ala Ser Leu Ile Gln Lys Met Leu Gln Thr Asp
    275                 280                 285

ccc act gcc cgc cca acc att aac gag ctg ctt aat gac gag ttc ttt 968
Pro Thr Ala Arg Pro Thr Ile Asn Glu Leu Leu Asn Asp Glu Phe Phe
290                 295                 300                 305

act tct ggc tat atc cct gcc cgt ctc ccc atc acc tgc ctg acc att 1016
Thr Ser Gly Tyr Ile Pro Ala Arg Leu Pro Ile Thr Cys Leu Thr Ile
                310                 315                 320

cca cca agg ttt tcg att gct ccc agc agc ctg gac ccc agc aac cgg 1064
Pro Pro Arg Phe Ser Ile Ala Pro Ser Ser Leu Asp Pro Ser Asn Arg
            325                 330                 335

aag ccc ctc aca gtc ctc aat aaa ggc ttg gag aac ccc ctg cct gag 1112
Lys Pro Leu Thr Val Leu Asn Lys Gly Leu Glu Asn Pro Leu Pro Glu
        340                 345                 350

cgt ccc cgg gaa aaa gaa gaa cca gtg gtt cga gag aca ggt gag gtg 1160
Arg Pro Arg Glu Lys Glu Glu Pro Val Val Arg Glu Thr Gly Glu Val
    355                 360                 365

gtc gac tgc cac ctc agt gac atg ctg cag cag ctg cac agt gtc aat 1208
Val Asp Cys His Leu Ser Asp Met Leu Gln Gln Leu His Ser Val Asn
370                 375                 380                 385

gcc tcc aag ccc tcg gag cgt ggg ctg gtc agg caa gag gag gct gag 1256
Ala Ser Lys Pro Ser Glu Arg Gly Leu Val Arg Gln Glu Glu Ala Glu
                390                 395                 400

gat cct gcc tgc atc ccc atc ttc tgg gtc agc aag tgg gtg gac tat 1304
Asp Pro Ala Cys Ile Pro Ile Phe Trp Val Ser Lys Trp Val Asp Tyr
            405                 410                 415

tcg gac aag tac ggc ctt ggg tat cag ctc tgt gat aac agc gtg ggg 1352
Ser Asp Lys Tyr Gly Leu Gly Tyr Gln Leu Cys Asp Asn Ser Val Gly
        420                 425                 430

gtg ctc ttc aat gac tca aca cgc ctc atc ctc tac aat gat ggt gac 1400
Val Leu Phe Asn Asp Ser Thr Arg Leu Ile Leu Tyr Asn Asp Gly Asp
    435                 440                 445

agc ctg cag tac ata gag cgt gac ggc act gag tcc tac ctc acc gtg 1448
Ser Leu Gln Tyr Ile Glu Arg Asp Gly Thr Glu Ser Tyr Leu Thr Val
450                 455                 460                 465

agt tcc cat ccc aac tcc ttg atg aag aag atc acc ctc ctt aaa tat 1496
Ser Ser His Pro Asn Ser Leu Met Lys Lys Ile Thr Leu Leu Lys Tyr
                470                 475                 480

ttc cgc aat tac atg agc gag cac ttg ctg aag gca ggt gcc aac atc 1544
Phe Arg Asn Tyr Met Ser Glu His Leu Leu Lys Ala Gly Ala Asn Ile
            485                 490                 495

acg ccg cgc gaa ggt gat gag ctc gcc cgg ctg ccc tac cta cgg acc     1592
Thr Pro Arg Glu Gly Asp Glu Leu Ala Arg Leu Pro Tyr Leu Arg Thr
        500                 505                 510

tgg ttc cgc acc cgc agc gcc atc atc ctg cac ctc agc aac ggc agc     1640
Trp Phe Arg Thr Arg Ser Ala Ile Ile Leu His Leu Ser Asn Gly Ser
    515                 520                 525

gtg cag atc aac ttc ttc cag gat cac acc aag ctc atc ttg tgc cca     1688
Val Gln Ile Asn Phe Phe Gln Asp His Thr Lys Leu Ile Leu Cys Pro
530                 535                 540                 545

ctg atg gca gcc gtg acc tac atc gac gag aag cgg gac ttc cgc aca     1736
Leu Met Ala Ala Val Thr Tyr Ile Asp Glu Lys Arg Asp Phe Arg Thr
                550                 555                 560

tac cgc ctg agt ctc ctg gag gag tac ggc tgc tgc aag gag ctg gcc     1784
Tyr Arg Leu Ser Leu Leu Glu Glu Tyr Gly Cys Cys Lys Glu Leu Ala
            565                 570                 575

agc cgg ctc cgc tac gcc cgc act atg gtg gac aag ctg ctg agc tca     1832
Ser Arg Leu Arg Tyr Ala Arg Thr Met Val Asp Lys Leu Leu Ser Ser
        580                 585                 590

cgc tcg gcc agc aac cgt ctc aag gcc tcc taa tagctgccct cccctccgga     1885
Arg Ser Ala Ser Asn Arg Leu Lys Ala Ser
    595                 600

ctggtgccct cctcactccc acctgcatct ggggcccata ctggttggct cccgcggtgc     1945

catgtctgca gtgtgccccc cagccccggt ggctgggcag agctgcatca tccttgcagg     2005

tgggggttgc tgtgtaagtt atttttgtac atgttcgggt gtgggttcta cagccttgtc     2065

cccctccccc tcaaccccac catatgaatt gtacagaata tttctattga attcggaact     2125

gtcctttcct tggctttatg cacattaaac agatgtgaat attcaaaaaa aaaaaaaaaa     2185

aaaaaaaaaa aaaaaaaaa                                                  2204

<210>  32
<211>  603
<212>  PRT
<213>  Homo sapiens

<400>  32

Met Ser Ala Ala Val Thr Ala Gly Lys Leu Ala Arg Ala Pro Ala Asp
1               5                   10                  15

Pro Gly Lys Ala Gly Val Pro Gly Val Ala Ala Pro Gly Ala Pro Ala
            20                  25                  30

Ala Ala Pro Pro Ala Lys Glu Ile Pro Glu Val Leu Val Asp Pro Arg
        35                  40                  45

Ser Arg Arg Arg Tyr Val Arg Gly Arg Phe Leu Gly Lys Gly Gly Phe
    50                  55                  60

Ala Lys Cys Phe Glu Ile Ser Asp Ala Asp Thr Lys Glu Val Phe Ala
65                  70                  75                  80

Gly Lys Ile Val Pro Lys Ser Leu Leu Leu Lys Pro His Gln Arg Glu
                85                  90                  95

Lys Met Ser Met Glu Ile Ser Ile His Arg Ser Leu Ala His Gln His
            100                 105                 110

Val Val Gly Phe His Gly Phe Phe Glu Asp Asn Asp Phe Val Phe Val
        115                 120                 125

Val Leu Glu Leu Cys Arg Arg Arg Ser Leu Leu Glu Leu His Lys Arg
    130                 135                 140

Arg Lys Ala Leu Thr Glu Pro Glu Ala Arg Tyr Tyr Leu Arg Gln Ile
145                 150                 155                 160

Val Leu Gly Cys Gln Tyr Leu His Arg Asn Arg Val Ile His Arg Asp
                165                 170                 175

Leu Lys Leu Gly Asn Leu Phe Leu Asn Glu Asp Leu Glu Val Lys Ile
            180                 185                 190

Gly Asp Phe Gly Leu Ala Thr Lys Val Glu Tyr Asp Gly Glu Arg Lys
        195                 200                 205

Lys Thr Leu Cys Gly Thr Pro Asn Tyr Ile Ala Pro Glu Val Leu Ser
    210                 215                 220

Lys Lys Gly His Ser Phe Glu Val Asp Val Trp Ser Ile Gly Cys Ile
225                 230                 235                 240

Met Tyr Thr Leu Leu Val Gly Lys Pro Pro Phe Glu Thr Ser Cys Leu
                245                 250                 255

Lys Glu Thr Tyr Leu Arg Ile Lys Lys Asn Glu Tyr Ser Ile Pro Lys
            260                 265                 270

His Ile Asn Pro Val Ala Ala Ser Leu Ile Gln Lys Met Leu Gln Thr
        275                 280                 285

Asp Pro Thr Ala Arg Pro Thr Ile Asn Glu Leu Leu Asn Asp Glu Phe
    290                 295                 300

Phe Thr Ser Gly Tyr Ile Pro Ala Arg Leu Pro Ile Thr Cys Leu Thr
305                 310                 315                 320

Ile Pro Pro Arg Phe Ser Ile Ala Pro Ser Ser Leu Asp Pro Ser Asn
                325                 330                 335

Arg Lys Pro Leu Thr Val Leu Asn Lys Gly Leu Glu Asn Pro Leu Pro
            340                 345                 350

Glu Arg Pro Arg Glu Lys Glu Glu Pro Val Val Arg Glu Thr Gly Glu
        355                 360                 365

Val Val Asp Cys His Leu Ser Asp Met Leu Gln Gln Leu His Ser Val
    370                 375                 380

Asn Ala Ser Lys Pro Ser Glu Arg Gly Leu Val Arg Gln Glu Glu Ala
385                 390                 395                 400

Glu Asp Pro Ala Cys Ile Pro Ile Phe Trp Val Ser Lys Trp Val Asp
                405                 410                 415

Tyr Ser Asp Lys Tyr Gly Leu Gly Tyr Gln Leu Cys Asp Asn Ser Val
            420                 425                 430

Gly Val Leu Phe Asn Asp Ser Thr Arg Leu Ile Leu Tyr Asn Asp Gly
        435                 440                 445

Asp Ser Leu Gln Tyr Ile Glu Arg Asp Gly Thr Glu Ser Tyr Leu Thr
    450                 455                 460

Val Ser Ser His Pro Asn Ser Leu Met Lys Lys Ile Thr Leu Leu Lys
465                 470                 475                 480

Tyr Phe Arg Asn Tyr Met Ser Glu His Leu Leu Lys Ala Gly Ala Asn
                485                 490                 495

Ile Thr Pro Arg Glu Gly Asp Glu Leu Ala Arg Leu Pro Tyr Leu Arg
            500                 505                 510

Thr Trp Phe Arg Thr Arg Ser Ala Ile Ile Leu His Leu Ser Asn Gly
        515                 520                 525

Ser Val Gln Ile Asn Phe Phe Gln Asp His Thr Lys Leu Ile Leu Cys
    530                 535                 540

Pro Leu Met Ala Ala Val Thr Tyr Ile Asp Glu Lys Arg Asp Phe Arg
545                 550                 555                 560

Thr Tyr Arg Leu Ser Leu Leu Glu Glu Tyr Gly Cys Cys Lys Glu Leu
                565                 570                 575

Ala Ser Arg Leu Arg Tyr Ala Arg Thr Met Val Asp Lys Leu Leu Ser
            580                 585                 590

Ser Arg Ser Ala Ser Asn Arg Leu Lys Ala Ser
        595                 600

<210>  33
<211>  990
<212>  DNA
<213>  Homo sapiens

<220>
<221>  CDS
<222>  (1)..(990)
<223>  PLK1- amino acid residues 275-603 of SEQ ID NO:31

<400>  33
aac ccc gtg gcc gcc tcc ctc atc cag aag atg ctt cag aca gat ccc      48
Asn Pro Val Ala Ala Ser Leu Ile Gln Lys Met Leu Gln Thr Asp Pro
1               5                   10                  15

act gcc cgc cca acc att aac gag ctg ctt aat gac gag ttc ttt act      96
Thr Ala Arg Pro Thr Ile Asn Glu Leu Leu Asn Asp Glu Phe Phe Thr
            20                  25                  30

tct ggc tat atc cct gcc cgt ctc ccc atc acc tgc ctg acc att cca      144
Ser Gly Tyr Ile Pro Ala Arg Leu Pro Ile Thr Cys Leu Thr Ile Pro
        35                  40                  45

cca agg ttt tcg att gct ccc agc agc ctg gac ccc agc aac cgg aag      192
Pro Arg Phe Ser Ile Ala Pro Ser Ser Leu Asp Pro Ser Asn Arg Lys
    50                  55                  60

ccc ctc aca gtc ctc aat aaa ggc ttg gag aac ccc ctg cct gag cgt      240
Pro Leu Thr Val Leu Asn Lys Gly Leu Glu Asn Pro Leu Pro Glu Arg
65                  70                  75                  80

ccc cgg gaa aaa gaa gaa cca gtg gtt cga gag aca ggt gag gtg gtc      288
Pro Arg Glu Lys Glu Glu Pro Val Val Arg Glu Thr Gly Glu Val Val
                85                  90                  95

gac tgc cac ctc agt gac atg ctg cag cag ctg cac agt gtc aat gcc      336
Asp Cys His Leu Ser Asp Met Leu Gln Gln Leu His Ser Val Asn Ala
            100                 105                 110

tcc aag ccc tcg gag cgt ggg ctg gtc agg caa gag gag gct gag gat      384
Ser Lys Pro Ser Glu Arg Gly Leu Val Arg Gln Glu Glu Ala Glu Asp
        115                 120                 125

cct gcc tgc atc ccc atc ttc tgg gtc agc aag tgg gtg gac tat tcg      432
Pro Ala Cys Ile Pro Ile Phe Trp Val Ser Lys Trp Val Asp Tyr Ser
    130                 135                 140

gac aag tac ggc ctt ggg tat cag ctc tgt gat aac agc gtg ggg gtg      480
Asp Lys Tyr Gly Leu Gly Tyr Gln Leu Cys Asp Asn Ser Val Gly Val
145                 150                 155                 160

ctc ttc aat gac tca aca cgc ctc atc ctc tac aat gat ggt gac agc      528
Leu Phe Asn Asp Ser Thr Arg Leu Ile Leu Tyr Asn Asp Gly Asp Ser
                165                 170                 175

ctg cag tac ata gag cgt gac ggc act gag tcc tac ctc acc gtg agt      576
Leu Gln Tyr Ile Glu Arg Asp Gly Thr Glu Ser Tyr Leu Thr Val Ser
            180                 185                 190

tcc cat ccc aac tcc ttg atg aag aag atc acc ctc ctt aaa tat ttc      624
Ser His Pro Asn Ser Leu Met Lys Lys Ile Thr Leu Leu Lys Tyr Phe
        195                 200                 205

cgc aat tac atg agc gag cac ttg ctg aag gca ggt gcc aac atc acg      672
Arg Asn Tyr Met Ser Glu His Leu Leu Lys Ala Gly Ala Asn Ile Thr
    210                 215                 220

ccg cgc gaa ggt gat gag ctc gcc cgg ctg ccc tac cta cgg acc tgg      720
Pro Arg Glu Gly Asp Glu Leu Ala Arg Leu Pro Tyr Leu Arg Thr Trp
225                 230                 235                 240

ttc cgc acc cgc agc gcc atc atc ctg cac ctc agc aac ggc agc gtg 768
Phe Arg Thr Arg Ser Ala Ile Ile Leu His Leu Ser Asn Gly Ser Val
                245                 250                 255

cag atc aac ttc ttc cag gat cac acc aag ctc atc ttg tgc cca ctg 816
Gln Ile Asn Phe Phe Gln Asp His Thr Lys Leu Ile Leu Cys Pro Leu
            260                 265                 270

atg gca gcc gtg acc tac atc gac gag aag cgg gac ttc cgc aca tac 864
Met Ala Ala Val Thr Tyr Ile Asp Glu Lys Arg Asp Phe Arg Thr Tyr
        275                 280                 285

cgc ctg agt ctc ctg gag gag tac ggc tgc tgc aag gag ctg gcc agc 912
Arg Leu Ser Leu Leu Glu Glu Tyr Gly Cys Cys Lys Glu Leu Ala Ser
    290                 295                 300

cgg ctc cgc tac gcc cgc act atg gtg gac aag ctg ctg agc tca cgc 960
Arg Leu Arg Tyr Ala Arg Thr Met Val Asp Lys Leu Leu Ser Ser Arg
305                 310                 315                 320

tcg gcc agc aac cgt ctc aag gcc tcc taa 990
Ser Ala Ser Asn Arg Leu Lys Ala Ser
                325

<210> 34
<211> 329
<212> PRT
<213> Homo sapiens

<400> 34

Asn Pro Val Ala Ala Ser Leu Ile Gln Lys Met Leu Gln Thr Asp Pro
1               5                   10                  15

Thr Ala Arg Pro Thr Ile Asn Glu Leu Leu Asn Asp Glu Phe Phe Thr
            20                  25                  30

Ser Gly Tyr Ile Pro Ala Arg Leu Pro Ile Thr Cys Leu Thr Ile Pro
        35                  40                  45

Pro Arg Phe Ser Ile Ala Pro Ser Ser Leu Asp Pro Ser Asn Arg Lys
    50                  55                  60

Pro Leu Thr Val Leu Asn Lys Gly Leu Glu Asn Pro Leu Pro Glu Arg
65                  70                  75                  80

Pro Arg Glu Lys Glu Glu Pro Val Val Arg Glu Thr Gly Glu Val Val
                85                  90                  95

Asp Cys His Leu Ser Asp Met Leu Gln Gln Leu His Ser Val Asn Ala
            100                 105                 110

Ser Lys Pro Ser Glu Arg Gly Leu Val Arg Gln Glu Glu Ala Glu Asp
        115                 120                 125

Pro Ala Cys Ile Pro Ile Phe Trp Val Ser Lys Trp Val Asp Tyr Ser
    130                 135                 140

Asp Lys Tyr Gly Leu Gly Tyr Gln Leu Cys Asp Asn Ser Val Gly Val
145                 150                 155                 160

Leu Phe Asn Asp Ser Thr Arg Leu Ile Leu Tyr Asn Asp Gly Asp Ser
                165                 170                 175

Leu Gln Tyr Ile Glu Arg Asp Gly Thr Glu Ser Tyr Leu Thr Val Ser
            180                 185                 190

Ser His Pro Asn Ser Leu Met Lys Lys Ile Thr Leu Leu Lys Tyr Phe
        195                 200                 205

Arg Asn Tyr Met Ser Glu His Leu Leu Lys Ala Gly Ala Asn Ile Thr
    210                 215                 220

Pro Arg Glu Gly Asp Glu Leu Ala Arg Leu Pro Tyr Leu Arg Thr Trp
225                 230                 235                 240

Phe Arg Thr Arg Ser Ala Ile Ile Leu His Leu Ser Asn Gly Ser Val
                245                 250                 255

Gln Ile Asn Phe Phe Gln Asp His Thr Lys Leu Ile Leu Cys Pro Leu
            260                 265                 270

Met Ala Ala Val Thr Tyr Ile Asp Glu Lys Arg Asp Phe Arg Thr Tyr
        275                 280                 285

Arg Leu Ser Leu Leu Glu Glu Tyr Gly Cys Cys Lys Glu Leu Ala Ser
    290                 295                 300

Arg Leu Arg Tyr Ala Arg Thr Met Val Asp Lys Leu Leu Ser Ser Arg
305                 310                 315                 320

Ser Ala Ser Asn Arg Leu Lys Ala Ser
                325

<210>  35
<211>  846
<212>  DNA
<213>  Homo sapiens

<220>
<221>  CDS
<222>  (1)..(846)
<223>  PLK1- amino acid residues 323-603 of SEQ ID NO:31

<400>  35
cca agg ttt tcg att gct ccc agc agc ctg gac ccc agc aac cgg aag      48
Pro Arg Phe Ser Ile Ala Pro Ser Ser Leu Asp Pro Ser Asn Arg Lys
1               5                   10                  15

ccc ctc aca gtc ctc aat aaa ggc ttg gag aac ccc ctg cct gag cgt      96
Pro Leu Thr Val Leu Asn Lys Gly Leu Glu Asn Pro Leu Pro Glu Arg
            20                  25                  30

ccc cgg gaa aaa gaa gaa cca gtg gtt cga gag aca ggt gag gtg gtc 144
Pro Arg Glu Lys Glu Glu Pro Val Val Arg Glu Thr Gly Glu Val Val
        35                  40                  45

gac tgc cac ctc agt gac atg ctg cag cag ctg cac agt gtc aat gcc 192
Asp Cys His Leu Ser Asp Met Leu Gln Gln Leu His Ser Val Asn Ala
    50                  55                  60

tcc aag ccc tcg gag cgt ggg ctg gtc agg caa gag gag gct gag gat 240
Ser Lys Pro Ser Glu Arg Gly Leu Val Arg Gln Glu Glu Ala Glu Asp
65                  70                  75                  80

cct gcc tgc atc ccc atc ttc tgg gtc agc aag tgg gtg gac tat tcg 288
Pro Ala Cys Ile Pro Ile Phe Trp Val Ser Lys Trp Val Asp Tyr Ser
                85                  90                  95

gac aag tac ggc ctt ggg tat cag ctc tgt gat aac agc gtg ggg gtg 336
Asp Lys Tyr Gly Leu Gly Tyr Gln Leu Cys Asp Asn Ser Val Gly Val
            100                 105                 110

ctc ttc aat gac tca aca cgc ctc atc ctc tac aat gat ggt gac agc 384
Leu Phe Asn Asp Ser Thr Arg Leu Ile Leu Tyr Asn Asp Gly Asp Ser
        115                 120                 125

ctg cag tac ata gag cgt gac ggc act gag tcc tac ctc acc gtg agt 432
Leu Gln Tyr Ile Glu Arg Asp Gly Thr Glu Ser Tyr Leu Thr Val Ser
    130                 135                 140

tcc cat ccc aac tcc ttg atg aag aag atc acc ctc ctt aaa tat ttc 480
Ser His Pro Asn Ser Leu Met Lys Lys Ile Thr Leu Leu Lys Tyr Phe
145                 150                 155                 160

cgc aat tac atg agc gag cac ttg ctg aag gca ggt gcc aac atc acg 528
Arg Asn Tyr Met Ser Glu His Leu Leu Lys Ala Gly Ala Asn Ile Thr
                165                 170                 175

ccg cgc gaa ggt gat gag ctc gcc cgg ctg ccc tac cta cgg acc tgg 576
Pro Arg Glu Gly Asp Glu Leu Ala Arg Leu Pro Tyr Leu Arg Thr Trp
            180                 185                 190

ttc cgc acc cgc agc gcc atc atc ctg cac ctc agc aac ggc agc gtg 624
Phe Arg Thr Arg Ser Ala Ile Ile Leu His Leu Ser Asn Gly Ser Val
        195                 200                 205

cag atc aac ttc ttc cag gat cac acc aag ctc atc ttg tgc cca ctg 672
Gln Ile Asn Phe Phe Gln Asp His Thr Lys Leu Ile Leu Cys Pro Leu
    210                 215                 220

atg gca gcc gtg acc tac atc gac gag aag cgg gac ttc cgc aca tac 720
Met Ala Ala Val Thr Tyr Ile Asp Glu Lys Arg Asp Phe Arg Thr Tyr
225                 230                 235                 240

cgc ctg agt ctc ctg gag gag tac ggc tgc tgc aag gag ctg gcc agc 768
Arg Leu Ser Leu Leu Glu Glu Tyr Gly Cys Cys Lys Glu Leu Ala Ser
                245                 250                 255

cgg ctc cgc tac gcc cgc act atg gtg gac aag ctg ctg agc tca cgc 816
Arg Leu Arg Tyr Ala Arg Thr Met Val Asp Lys Leu Leu Ser Ser Arg
            260                 265                 270

tcg gcc agc aac cgt ctc aag gcc tcc taa 846
Ser Ala Ser Asn Arg Leu Lys Ala Ser
        275                 280

<210> 36
<211> 281
<212> PRT

<213>  Homo sapiens

<400>  36

Pro Arg Phe Ser Ile Ala Pro Ser Ser Leu Asp Pro Ser Asn Arg Lys
1               5                   10                  15

Pro Leu Thr Val Leu Asn Lys Gly Leu Glu Asn Pro Leu Pro Glu Arg
            20                  25                  30

Pro Arg Glu Lys Glu Glu Pro Val Val Arg Glu Thr Gly Glu Val Val
        35                  40                  45

Asp Cys His Leu Ser Asp Met Leu Gln Gln Leu His Ser Val Asn Ala
    50                  55                  60

Ser Lys Pro Ser Glu Arg Gly Leu Val Arg Gln Glu Glu Ala Glu Asp
65                  70                  75                  80

Pro Ala Cys Ile Pro Ile Phe Trp Val Ser Lys Trp Val Asp Tyr Ser
                85                  90                  95

Asp Lys Tyr Gly Leu Gly Tyr Gln Leu Cys Asp Asn Ser Val Gly Val
            100                 105                 110

Leu Phe Asn Asp Ser Thr Arg Leu Ile Leu Tyr Asn Asp Gly Asp Ser
        115                 120                 125

Leu Gln Tyr Ile Glu Arg Asp Gly Thr Glu Ser Tyr Leu Thr Val Ser
    130                 135                 140

Ser His Pro Asn Ser Leu Met Lys Lys Ile Thr Leu Leu Lys Tyr Phe
145                 150                 155                 160

Arg Asn Tyr Met Ser Glu His Leu Leu Lys Ala Gly Ala Asn Ile Thr
                165                 170                 175

Pro Arg Glu Gly Asp Glu Leu Ala Arg Leu Pro Tyr Leu Arg Thr Trp
            180                 185                 190

Phe Arg Thr Arg Ser Ala Ile Ile Leu His Leu Ser Asn Gly Ser Val
        195                 200                 205

Gln Ile Asn Phe Phe Gln Asp His Thr Lys Leu Ile Leu Cys Pro Leu
    210                 215                 220

Met Ala Ala Val Thr Tyr Ile Asp Glu Lys Arg Asp Phe Arg Thr Tyr
225                 230                 235                 240

Arg Leu Ser Leu Leu Glu Glu Tyr Gly Cys Cys Lys Glu Leu Ala Ser
                245                 250                 255

Arg Leu Arg Tyr Ala Arg Thr Met Val Asp Lys Leu Leu Ser Ser Arg
            260                 265                 270

Ser Ala Ser Asn Arg Leu Lys Ala Ser
        275                 280

<210>  37
<211>  594
<212>  DNA
<213>  Homo sapiens

<220>
<221>  CDS
<222>  (1)..(594)
<223>  PLK1- amino acid residues 407-603 of SEQ ID NO:31

<400>  37
ccc atc ttc tgg gtc agc aag tgg gtg gac tat tcg gac aag tac ggc      48
Pro Ile Phe Trp Val Ser Lys Trp Val Asp Tyr Ser Asp Lys Tyr Gly
1               5                   10                  15

ctt ggg tat cag ctc tgt gat aac agc gtg ggg gtg ctc ttc aat gac      96
Leu Gly Tyr Gln Leu Cys Asp Asn Ser Val Gly Val Leu Phe Asn Asp
            20                  25                  30

tca aca cgc ctc atc ctc tac aat gat ggt gac agc ctg cag tac ata      144
Ser Thr Arg Leu Ile Leu Tyr Asn Asp Gly Asp Ser Leu Gln Tyr Ile
        35                  40                  45

gag cgt gac ggc act gag tcc tac ctc acc gtg agt tcc cat ccc aac      192
Glu Arg Asp Gly Thr Glu Ser Tyr Leu Thr Val Ser Ser His Pro Asn
    50                  55                  60

tcc ttg atg aag aag atc acc ctc ctt aaa tat ttc cgc aat tac atg      240
Ser Leu Met Lys Lys Ile Thr Leu Leu Lys Tyr Phe Arg Asn Tyr Met
65                  70                  75                  80

agc gag cac ttg ctg aag gca ggt gcc aac atc acg ccg cgc gaa ggt      288
Ser Glu His Leu Leu Lys Ala Gly Ala Asn Ile Thr Pro Arg Glu Gly
                85                  90                  95

gat gag ctc gcc cgg ctg ccc tac cta cgg acc tgg ttc cgc acc cgc      336
Asp Glu Leu Ala Arg Leu Pro Tyr Leu Arg Thr Trp Phe Arg Thr Arg
            100                 105                 110

agc gcc atc atc ctg cac ctc agc aac ggc agc gtg cag atc aac ttc      384
Ser Ala Ile Ile Leu His Leu Ser Asn Gly Ser Val Gln Ile Asn Phe
        115                 120                 125

ttc cag gat cac acc aag ctc atc ttg tgc cca ctg atg gca gcc gtg      432
Phe Gln Asp His Thr Lys Leu Ile Leu Cys Pro Leu Met Ala Ala Val
    130                 135                 140

acc tac atc gac gag aag cgg gac ttc cgc aca tac cgc ctg agt ctc      480
Thr Tyr Ile Asp Glu Lys Arg Asp Phe Arg Thr Tyr Arg Leu Ser Leu
145                 150                 155                 160

ctg gag gag tac ggc tgc tgc aag gag ctg gcc agc cgg ctc cgc tac      528
Leu Glu Glu Tyr Gly Cys Cys Lys Glu Leu Ala Ser Arg Leu Arg Tyr
                165                 170                 175

gcc cgc act atg gtg gac aag ctg ctg agc tca cgc tcg gcc agc aac      576
Ala Arg Thr Met Val Asp Lys Leu Leu Ser Ser Arg Ser Ala Ser Asn
            180                 185                 190

cgt ctc aag gcc tcc taa                                                    594
Arg Leu Lys Ala Ser
        195

<210>  38
<211>  197
<212>  PRT
<213>  Homo sapiens

<400>  38

Pro Ile Phe Trp Val Ser Lys Trp Val Asp Tyr Ser Asp Lys Tyr Gly
1               5                   10                  15

Leu Gly Tyr Gln Leu Cys Asp Asn Ser Val Gly Val Leu Phe Asn Asp
            20                  25                  30

Ser Thr Arg Leu Ile Leu Tyr Asn Asp Gly Asp Ser Leu Gln Tyr Ile
        35                  40                  45

Glu Arg Asp Gly Thr Glu Ser Tyr Leu Thr Val Ser Ser His Pro Asn
    50                  55                  60

Ser Leu Met Lys Lys Ile Thr Leu Leu Lys Tyr Phe Arg Asn Tyr Met
65                  70                  75                  80

Ser Glu His Leu Leu Lys Ala Gly Ala Asn Ile Thr Pro Arg Glu Gly
                85                  90                  95

Asp Glu Leu Ala Arg Leu Pro Tyr Leu Arg Thr Trp Phe Arg Thr Arg
            100                 105                 110

Ser Ala Ile Ile Leu His Leu Ser Asn Gly Ser Val Gln Ile Asn Phe
        115                 120                 125

Phe Gln Asp His Thr Lys Leu Ile Leu Cys Pro Leu Met Ala Ala Val
    130                 135                 140

Thr Tyr Ile Asp Glu Lys Arg Asp Phe Arg Thr Tyr Arg Leu Ser Leu
145                 150                 155                 160

Leu Glu Glu Tyr Gly Cys Cys Lys Glu Leu Ala Ser Arg Leu Arg Tyr
                165                 170                 175

Ala Arg Thr Met Val Asp Lys Leu Leu Ser Ser Arg Ser Ala Ser Asn
            180                 185                 190

Arg Leu Lys Ala Ser
        195

<210>  39
<211>  603
<212>  PRT
<213>  Artificial

<220>
<223>  protein (mutated human PLK1)

<400>  39

Met Ser Ala Ala Val Thr Ala Gly Lys Leu Ala Arg Ala Pro Ala Asp
1               5                   10                  15

Pro Gly Lys Ala Gly Val Pro Gly Val Ala Ala Pro Gly Ala Pro Ala
            20                  25                  30

Ala Ala Pro Pro Ala Lys Glu Ile Pro Glu Val Leu Val Asp Pro Arg
        35                  40                  45

Ser Arg Arg Arg Tyr Val Arg Gly Arg Phe Leu Gly Lys Gly Gly Phe
    50                  55                  60

Ala Lys Cys Phe Glu Ile Ser Asp Ala Asp Thr Lys Glu Val Phe Ala
65                  70                  75                  80

Gly Lys Ile Val Pro Lys Ser Leu Leu Leu Lys Pro His Gln Arg Glu
                85                  90                  95

Lys Met Ser Met Glu Ile Ser Ile His Arg Ser Leu Ala His Gln His
            100                 105                 110

Val Val Gly Phe His Gly Phe Phe Glu Asp Asn Asp Phe Val Phe Val
        115                 120                 125

Val Leu Glu Leu Cys Arg Arg Arg Ser Leu Leu Glu Leu His Lys Arg
    130                 135                 140

Arg Lys Ala Leu Thr Glu Pro Glu Ala Arg Tyr Tyr Leu Arg Gln Ile
145                 150                 155                 160

Val Leu Gly Cys Gln Tyr Leu His Arg Asn Arg Val Ile His Arg Asp
                165                 170                 175

Leu Lys Leu Gly Asn Leu Phe Leu Asn Glu Asp Leu Glu Val Lys Ile
            180                 185                 190

Gly Asp Phe Gly Leu Ala Thr Lys Val Glu Tyr Asp Gly Glu Arg Lys
        195                 200                 205

Lys Thr Leu Cys Gly Thr Pro Asn Tyr Ile Ala Pro Glu Val Leu Ser
    210                 215                 220

Lys Lys Gly His Ser Phe Glu Val Asp Val Trp Ser Ile Gly Cys Ile
225                 230                 235                 240

Met Tyr Thr Leu Leu Val Gly Lys Pro Pro Phe Glu Thr Ser Cys Leu
                245                 250                 255

Lys Glu Thr Tyr Leu Arg Ile Lys Lys Asn Glu Tyr Ser Ile Pro Lys
            260                 265                 270

His Ile Asn Pro Val Ala Ala Ser Leu Ile Gln Lys Met Leu Gln Thr
        275                 280                 285

Asp Pro Thr Ala Arg Pro Thr Ile Asn Glu Leu Leu Asn Asp Glu Phe
    290                 295                 300

Phe Thr Ser Gly Tyr Ile Pro Ala Arg Leu Pro Ile Thr Cys Leu Thr
305                 310                 315                 320

Ile Pro Pro Arg Phe Ser Ile Ala Pro Ser Ser Leu Asp Pro Ser Asn
                325                 330                 335

Arg Lys Pro Leu Thr Val Leu Asn Lys Gly Leu Glu Asn Pro Leu Pro
            340                 345                 350

Glu Arg Pro Arg Glu Lys Glu Glu Pro Val Val Arg Glu Thr Gly Glu
        355                 360                 365

Val Val Asp Cys His Leu Ser Asp Met Leu Gln Gln Leu His Ser Val
    370                 375                 380

Asn Ala Ser Lys Pro Ser Glu Arg Gly Leu Val Arg Gln Glu Glu Ala
385                 390                 395                 400

Glu Asp Pro Ala Cys Ile Pro Ile Phe Trp Val Ser Lys Trp Val Asp
                405                 410                 415

Tyr Ser Asp Lys Tyr Gly Leu Gly Tyr Gln Leu Cys Asp Asn Ser Val
            420                 425                 430

Gly Val Leu Phe Asn Asp Ser Thr Arg Leu Ile Leu Tyr Asn Asp Gly
        435                 440                 445

Asp Ser Leu Gln Tyr Ile Glu Arg Asp Gly Thr Glu Ser Tyr Leu Thr
    450                 455                 460

Val Ser Ser His Pro Asn Ser Leu Met Lys Lys Ile Thr Leu Leu Lys
465                 470                 475                 480

Tyr Phe Arg Asn Tyr Met Ser Glu His Leu Leu Lys Ala Gly Ala Asn
                485                 490                 495

Ile Thr Pro Arg Glu Gly Asp Glu Leu Ala Arg Leu Pro Tyr Leu Arg
            500                 505                 510

Thr Trp Phe Arg Thr Arg Ser Ala Ile Ile Leu His Leu Ser Asn Gly
        515                 520                 525

Ser Val Gln Ile Asn Phe Phe Gln Asp Ala Thr Met Leu Ile Leu Cys
    530                 535                 540

Pro Leu Met Ala Ala Val Thr Tyr Ile Asp Glu Lys Arg Asp Phe Arg
545                 550                 555                 560

Thr Tyr Arg Leu Ser Leu Leu Glu Glu Tyr Gly Cys Cys Lys Glu Leu
                565                 570                 575

Ala Ser Arg Leu Arg Tyr Ala Arg Thr Met Val Asp Lys Leu Leu Ser
            580                 585                 590

Ser Arg Ser Ala Ser Asn Arg Leu Lys Ala Ser
        595                 600

<210>  40
<211>  197
<212>  PRT
<213>  Artificial

<220>
<223>  fragment of protein (mutated PBD of the human PLK1)

<400>  40

Pro Ile Phe Trp Val Ser Lys Trp Val Asp Tyr Ser Asp Lys Tyr Gly
1               5                   10                  15

Leu Gly Tyr Gln Leu Cys Asp Asn Ser Val Gly Val Leu Phe Asn Asp
            20                  25                  30

Ser Thr Arg Leu Ile Leu Tyr Asn Asp Gly Asp Ser Leu Gln Tyr Ile
        35                  40                  45

Glu Arg Asp Gly Thr Glu Ser Tyr Leu Thr Val Ser Ser His Pro Asn
    50                  55                  60

Ser Leu Met Lys Lys Ile Thr Leu Leu Lys Tyr Phe Arg Asn Tyr Met
65                  70                  75                  80

Ser Glu His Leu Leu Lys Ala Gly Ala Asn Ile Thr Pro Arg Glu Gly
                85                  90                  95

Asp Glu Leu Ala Arg Leu Pro Tyr Leu Arg Thr Trp Phe Arg Thr Arg
            100                 105                 110

Ser Ala Ile Ile Leu His Leu Ser Asn Gly Ser Val Gln Ile Asn Phe
        115                 120                 125

Phe Gln Asp Ala Thr Met Leu Ile Leu Cys Pro Leu Met Ala Ala Val
    130                 135                 140

Thr Tyr Ile Asp Glu Lys Arg Asp Phe Arg Thr Tyr Arg Leu Ser Leu
145                 150                 155                 160

Leu Glu Glu Tyr Gly Cys Cys Lys Glu Leu Ala Ser Arg Leu Arg Tyr
                165                 170                 175

Ala Arg Thr Met Val Asp Lys Leu Leu Ser Ser Arg Ser Ala Ser Asn
            180                 185                 190

Arg Leu Lys Ala Ser
        195

<210>  41
<211>  2795
<212>  DNA
<213>  Homo sapiens

<220>
<221>  CDS
<222>  (128)..(2185)
<223>  PLK2

<400>  41
gcacaagtgg accggggtgt tgggtgctag tcggcaccag aggcaagggt gcgaggacca      60

cggccggctc ggacgtgtga ccgcgcctag ggggtggcag cgggcagtgc ggggcggcaa     120

ggcgacc atg gag ctt ttg cgg act atc acc tac cag cca gcc gcc agc       169
        Met Glu Leu Leu Arg Thr Ile Thr Tyr Gln Pro Ala Ala Ser
        1               5                   10

acc aaa atg tgc gag cag gcg ctg ggc aag ggt tgc gga gcg gac tcg       217
Thr Lys Met Cys Glu Gln Ala Leu Gly Lys Gly Cys Gly Ala Asp Ser
15                  20                  25                  30

aag aag aag cgg ccg ccg cag ccc ccc gag gaa tcg cag cca cct cag       265
Lys Lys Lys Arg Pro Pro Gln Pro Pro Glu Glu Ser Gln Pro Pro Gln
                35                  40                  45

tcc cag gcg caa gtg ccc ccg gcg gcc cct cac cac cat cac cac cat       313
Ser Gln Ala Gln Val Pro Pro Ala Ala Pro His His His His His His
            50                  55                  60

tcg cac tcg ggg ccg gag atc tcg cgg att atc gtc gac ccc acg act       361
Ser His Ser Gly Pro Glu Ile Ser Arg Ile Ile Val Asp Pro Thr Thr
        65                  70                  75

ggg aag cgc tac tgc cgg ggc aaa gtg ctg gga aag ggt ggc ttt gca       409
Gly Lys Arg Tyr Cys Arg Gly Lys Val Leu Gly Lys Gly Gly Phe Ala
    80                  85                  90

aaa tgt tac gag atg aca gat ttg aca aat aac aaa gtc tac gcc gca       457
Lys Cys Tyr Glu Met Thr Asp Leu Thr Asn Asn Lys Val Tyr Ala Ala
95                  100                 105                 110

aaa att att cct cac agc aga gta gct aaa cct cat caa agg gaa aag       505
Lys Ile Ile Pro His Ser Arg Val Ala Lys Pro His Gln Arg Glu Lys
                115                 120                 125

att gac aaa gaa ata gag ctt cac aga att ctt cat cat aag cat gta       553
Ile Asp Lys Glu Ile Glu Leu His Arg Ile Leu His His Lys His Val
            130                 135                 140

gtg cag ttt tac cac tac ttc gag gac aaa gaa aac att tac att ctc 601
Val Gln Phe Tyr His Tyr Phe Glu Asp Lys Glu Asn Ile Tyr Ile Leu
        145                 150                 155

ttg gaa tac tgc agt aga agg tca atg gct cat att ttg aaa gca aga 649
Leu Glu Tyr Cys Ser Arg Arg Ser Met Ala His Ile Leu Lys Ala Arg
    160                 165                 170

aag gtg ttg aca gag cca gaa gtt cga tac tac ctc agg cag att gtg 697
Lys Val Leu Thr Glu Pro Glu Val Arg Tyr Tyr Leu Arg Gln Ile Val
175                 180                 185                 190

tct gga ctg aaa tac ctt cat gaa caa gaa atc ttg cac aga gat ctc 745
Ser Gly Leu Lys Tyr Leu His Glu Gln Glu Ile Leu His Arg Asp Leu
                195                 200                 205

aaa cta ggg aac ttt ttt att aat gaa gcc atg gaa cta aaa gtt ggg 793
Lys Leu Gly Asn Phe Phe Ile Asn Glu Ala Met Glu Leu Lys Val Gly
            210                 215                 220

gac ttc ggt ctg gca gcc agg cta gaa ccc ttg gaa cac aga agg aga 841
Asp Phe Gly Leu Ala Ala Arg Leu Glu Pro Leu Glu His Arg Arg Arg
        225                 230                 235

acg ata tgt ggt acc cca aat tat ctc tct cct gaa gtc ctc aac aaa 889
Thr Ile Cys Gly Thr Pro Asn Tyr Leu Ser Pro Glu Val Leu Asn Lys
    240                 245                 250

caa gga cat ggc tgt gaa tca gac att tgg gcc ctg ggc tgt gta atg 937
Gln Gly His Gly Cys Glu Ser Asp Ile Trp Ala Leu Gly Cys Val Met
255                 260                 265                 270

tat aca atg tta cta ggg agg ccc cca ttt gaa act aca aat ctc aaa 985
Tyr Thr Met Leu Leu Gly Arg Pro Pro Phe Glu Thr Thr Asn Leu Lys
                275                 280                 285

gaa act tat agg tgc ata agg gaa gca agg tat aca atg ccg tcc tca 1033
Glu Thr Tyr Arg Cys Ile Arg Glu Ala Arg Tyr Thr Met Pro Ser Ser
            290                 295                 300

ttg ctg gct cct gcc aag cac tta att gct agt atg ttg tcc aaa aac 1081
Leu Leu Ala Pro Ala Lys His Leu Ile Ala Ser Met Leu Ser Lys Asn
        305                 310                 315

cca gag gat cgt ccc agt ttg gat gac atc att cga cat gac ttt ttt 1129
Pro Glu Asp Arg Pro Ser Leu Asp Asp Ile Ile Arg His Asp Phe Phe
    320                 325                 330

ttg cag ggc ttc act ccg gac aga ctg tct tct agc tgt tgt cat aca 1177
Leu Gln Gly Phe Thr Pro Asp Arg Leu Ser Ser Ser Cys Cys His Thr
335                 340                 345                 350

gtt cca gat ttc cac tta tca agc cca gct aag aat ttc ttt aag aaa 1225
Val Pro Asp Phe His Leu Ser Ser Pro Ala Lys Asn Phe Phe Lys Lys
                355                 360                 365

gca gct gct gct ctt ttt ggt ggc aaa aaa gac aaa gca aga tat att 1273
Ala Ala Ala Ala Leu Phe Gly Gly Lys Lys Asp Lys Ala Arg Tyr Ile
            370                 375                 380

gac aca cat aat aga gtg tct aaa gaa gat gaa gac atc tac aag ctt 1321
Asp Thr His Asn Arg Val Ser Lys Glu Asp Glu Asp Ile Tyr Lys Leu
        385                 390                 395

agg cat gat ttg aaa aag act tca ata act cag caa ccc agc aaa cac 1369
Arg His Asp Leu Lys Lys Thr Ser Ile Thr Gln Gln Pro Ser Lys His
    400                 405                 410

agg aca gat gag gag ctc cag cca cct acc acc aca gtt gcc agg tct 1417
Arg Thr Asp Glu Glu Leu Gln Pro Pro Thr Thr Thr Val Ala Arg Ser
415                 420                 425                 430

gga aca ccc gca gta gaa aac aag cag cag att ggg gat gct att cgg 1465
Gly Thr Pro Ala Val Glu Asn Lys Gln Gln Ile Gly Asp Ala Ile Arg
                435                 440                 445

atg ata gtc aga ggg act ctt ggc agc tgt agc agc agc agt gaa tgc 1513
Met Ile Val Arg Gly Thr Leu Gly Ser Cys Ser Ser Ser Ser Glu Cys
            450                 455                 460

ctt gaa gac agt acc atg gga agt gtt gca gac aca gtg gca agg gtt 1561
Leu Glu Asp Ser Thr Met Gly Ser Val Ala Asp Thr Val Ala Arg Val
        465                 470                 475

ctt cgg gga tgt ctg gaa aac atg ccg gaa gct gat tgc att ccc aaa 1609
Leu Arg Gly Cys Leu Glu Asn Met Pro Glu Ala Asp Cys Ile Pro Lys
    480                 485                 490

gag cag ctg agc aca tca ttt cag tgg gtc acc aaa tgg gtt gat tac 1657
Glu Gln Leu Ser Thr Ser Phe Gln Trp Val Thr Lys Trp Val Asp Tyr
495                 500                 505                 510

tct aac aaa tat ggc ttt ggg tac cag ctc tca gac cac acc gtc ggt 1705
Ser Asn Lys Tyr Gly Phe Gly Tyr Gln Leu Ser Asp His Thr Val Gly
                515                 520                 525

gtc ctt ttc aac aat ggt gct cac atg agc ctc ctt cca gac aaa aaa 1753
Val Leu Phe Asn Asn Gly Ala His Met Ser Leu Leu Pro Asp Lys Lys
            530                 535                 540

aca gtt cac tat tac gca gag ctt ggc caa tgc tca gtt ttc cca gca 1801
Thr Val His Tyr Tyr Ala Glu Leu Gly Gln Cys Ser Val Phe Pro Ala
        545                 550                 555

aca gat gct cct gag caa ttt att agt caa gtg acg gtg ctg aaa tac 1849
Thr Asp Ala Pro Glu Gln Phe Ile Ser Gln Val Thr Val Leu Lys Tyr
    560                 565                 570

ttt tct cat tac atg gag gag aac ctc atg gat ggt gga gat ctg cct 1897
Phe Ser His Tyr Met Glu Glu Asn Leu Met Asp Gly Gly Asp Leu Pro
575                 580                 585                 590

agt gtt act gat att cga aga cct cgg ctc tac ctc ctt cag tgg cta 1945
Ser Val Thr Asp Ile Arg Arg Pro Arg Leu Tyr Leu Leu Gln Trp Leu
                595                 600                 605

aaa tct gat aag gcc cta atg atg ctc ttt aat gat ggc acc ttt cag 1993
Lys Ser Asp Lys Ala Leu Met Met Leu Phe Asn Asp Gly Thr Phe Gln
            610                 615                 620

gtg aat ttc tac cat gat cat aca aaa atc atc atc tgt agc caa aat 2041
Val Asn Phe Tyr His Asp His Thr Lys Ile Ile Ile Cys Ser Gln Asn
        625                 630                 635

gaa gaa tac ctt ctc acc tac atc aat gag gat agg ata tct aca act 2089
Glu Glu Tyr Leu Leu Thr Tyr Ile Asn Glu Asp Arg Ile Ser Thr Thr
    640                 645                 650

ttc agg ctg aca act ctg ctg atg tct ggc tgt tca tca gaa tta aaa 2137
Phe Arg Leu Thr Thr Leu Leu Met Ser Gly Cys Ser Ser Glu Leu Lys
655                 660                 665                 670

aat cga atg gaa tat gcc ctg aac atg ctc tta caa aga tgt aac tga 2185
Asn Arg Met Glu Tyr Ala Leu Asn Met Leu Leu Gln Arg Cys Asn
                675                 680                 685

aagacttttc gaatggaccc tatgggactc ctcttttcca ctgtgagatc tacagggaag 2245

ccaaaagaat gatctagagt atgttgaaga agatggacat gtggtggtac gaaaacaatt 2305

cccctgtggc ctgctggact ggttggaacc agaacaggct aaggcataca gttcttgact 2365

ttggacaatc caagagtgaa ccagaatgca gttttccttg agatacctgt tttaaaaggt 2425

ttttcagaca attttgcaga aaggtgcatt gattcttaaa ttctctctgt tgagagcatt 2485

tcagccagag gactttggaa ctgtgaatat acttcctgaa ggggagggag aagggaggaa 2545

gctcccatgt tgtttaaagg ctgtaattgg agcagctttt ggctgcgtaa ctgtgaacta 2605

tggccatata taattttttt tcattaattt ttgaagatac ttgtggctgg aaaagtgcat 2665

tccttgttaa taaacttttt atttattaca gcccaaagag cagtatttat tatcaaaatg 2725

tctttttttt tatgttgacc attttaaacc gttggcaata aagagtatga aaacgcagaa 2785

aaaaaaaaaa 2795

<210> 42
<211> 685
<212> PRT
<213> Homo sapiens

<400> 42

Met Glu Leu Leu Arg Thr Ile Thr Tyr Gln Pro Ala Ala Ser Thr Lys
1               5                   10                  15

Met Cys Glu Gln Ala Leu Gly Lys Gly Cys Gly Ala Asp Ser Lys Lys
            20                  25                  30

Lys Arg Pro Pro Gln Pro Pro Glu Glu Ser Gln Pro Pro Gln Ser Gln
        35                  40                  45

Ala Gln Val Pro Pro Ala Ala Pro His His His His His His Ser His
    50                  55                  60

Ser Gly Pro Glu Ile Ser Arg Ile Ile Val Asp Pro Thr Thr Gly Lys
65                  70                  75                  80

Arg Tyr Cys Arg Gly Lys Val Leu Gly Lys Gly Gly Phe Ala Lys Cys
                85                  90                  95

Tyr Glu Met Thr Asp Leu Thr Asn Asn Lys Val Tyr Ala Ala Lys Ile
            100                 105                 110

Ile Pro His Ser Arg Val Ala Lys Pro His Gln Arg Glu Lys Ile Asp
        115                 120                 125

Lys Glu Ile Glu Leu His Arg Ile Leu His His Lys His Val Val Gln
    130                 135                 140

Phe Tyr His Tyr Phe Glu Asp Lys Glu Asn Ile Tyr Ile Leu Leu Glu
145                 150                 155                 160

Tyr Cys Ser Arg Arg Ser Met Ala His Ile Leu Lys Ala Arg Lys Val
                165                 170                 175

Leu Thr Glu Pro Glu Val Arg Tyr Tyr Leu Arg Gln Ile Val Ser Gly
            180                 185                 190

Leu Lys Tyr Leu His Glu Gln Glu Ile Leu His Arg Asp Leu Lys Leu
        195                 200                 205

Gly Asn Phe Phe Ile Asn Glu Ala Met Glu Leu Lys Val Gly Asp Phe
    210                 215                 220

Gly Leu Ala Ala Arg Leu Glu Pro Leu Glu His Arg Arg Arg Thr Ile
225                 230                 235                 240

Cys Gly Thr Pro Asn Tyr Leu Ser Pro Glu Val Leu Asn Lys Gln Gly
                245                 250                 255

His Gly Cys Glu Ser Asp Ile Trp Ala Leu Gly Cys Val Met Tyr Thr
            260                 265                 270

Met Leu Leu Gly Arg Pro Pro Phe Glu Thr Thr Asn Leu Lys Glu Thr
        275                 280                 285

Tyr Arg Cys Ile Arg Glu Ala Arg Tyr Thr Met Pro Ser Ser Leu Leu
    290                 295                 300

Ala Pro Ala Lys His Leu Ile Ala Ser Met Leu Ser Lys Asn Pro Glu
305                 310                 315                 320

Asp Arg Pro Ser Leu Asp Asp Ile Ile Arg His Asp Phe Phe Leu Gln
                325                 330                 335

Gly Phe Thr Pro Asp Arg Leu Ser Ser Ser Cys Cys His Thr Val Pro
            340                 345                 350

Asp Phe His Leu Ser Ser Pro Ala Lys Asn Phe Phe Lys Lys Ala Ala
        355                 360                 365

Ala Ala Leu Phe Gly Gly Lys Lys Asp Lys Ala Arg Tyr Ile Asp Thr
    370                 375                 380

His Asn Arg Val Ser Lys Glu Asp Glu Asp Ile Tyr Lys Leu Arg His
385                 390                 395                 400

Asp Leu Lys Lys Thr Ser Ile Thr Gln Gln Pro Ser Lys His Arg Thr
                405                 410                 415

Asp Glu Glu Leu Gln Pro Pro Thr Thr Thr Val Ala Arg Ser Gly Thr
            420                 425                 430

Pro Ala Val Glu Asn Lys Gln Gln Ile Gly Asp Ala Ile Arg Met Ile
        435                 440                 445

Val Arg Gly Thr Leu Gly Ser Cys Ser Ser Ser Ser Glu Cys Leu Glu
    450                 455                 460

Asp Ser Thr Met Gly Ser Val Ala Asp Thr Val Ala Arg Val Leu Arg
465                 470                 475                 480

Gly Cys Leu Glu Asn Met Pro Glu Ala Asp Cys Ile Pro Lys Glu Gln
                485                 490                 495

Leu Ser Thr Ser Phe Gln Trp Val Thr Lys Trp Val Asp Tyr Ser Asn
            500                 505                 510

Lys Tyr Gly Phe Gly Tyr Gln Leu Ser Asp His Thr Val Gly Val Leu
        515                 520                 525

Phe Asn Asn Gly Ala His Met Ser Leu Leu Pro Asp Lys Lys Thr Val
    530                 535                 540

His Tyr Tyr Ala Glu Leu Gly Gln Cys Ser Val Phe Pro Ala Thr Asp
545                 550                 555                 560

Ala Pro Glu Gln Phe Ile Ser Gln Val Thr Val Leu Lys Tyr Phe Ser
                565                 570                 575

His Tyr Met Glu Glu Asn Leu Met Asp Gly Gly Asp Leu Pro Ser Val
            580                 585                 590

Thr Asp Ile Arg Arg Pro Arg Leu Tyr Leu Leu Gln Trp Leu Lys Ser
        595                 600                 605

Asp Lys Ala Leu Met Met Leu Phe Asn Asp Gly Thr Phe Gln Val Asn
    610                 615                 620

Phe Tyr His Asp His Thr Lys Ile Ile Ile Cys Ser Gln Asn Glu Glu
625                 630                 635                 640

Tyr Leu Leu Thr Tyr Ile Asn Glu Asp Arg Ile Ser Thr Thr Phe Arg
                645                 650                 655

Leu Thr Thr Leu Leu Met Ser Gly Cys Ser Ser Glu Leu Lys Asn Arg
            660                 665                 670

Met Glu Tyr Ala Leu Asn Met Leu Leu Gln Arg Cys Asn
        675                 680                 685

<210>  43
<211>  702

<212>  DNA
<213>  Homo sapiens

<220>
<221>  CDS
<222>  (1)..(702)
<223>  PLK2 - amino acid residues 453-685 of SEQ ID NO:41

<400>  43
ctt ggc agc tgt agc agc agc agt gaa tgc ctt gaa gac agt acc atg      48
Leu Gly Ser Cys Ser Ser Ser Ser Glu Cys Leu Glu Asp Ser Thr Met
1               5                   10                  15

gga agt gtt gca gac aca gtg gca agg gtt ctt cgg gga tgt ctg gaa      96
Gly Ser Val Ala Asp Thr Val Ala Arg Val Leu Arg Gly Cys Leu Glu
            20                  25                  30

aac atg ccg gaa gct gat tgc att ccc aaa gag cag ctg agc aca tca      144
Asn Met Pro Glu Ala Asp Cys Ile Pro Lys Glu Gln Leu Ser Thr Ser
        35                  40                  45

ttt cag tgg gtc acc aaa tgg gtt gat tac tct aac aaa tat ggc ttt      192
Phe Gln Trp Val Thr Lys Trp Val Asp Tyr Ser Asn Lys Tyr Gly Phe
    50                  55                  60

ggg tac cag ctc tca gac cac acc gtc ggt gtc ctt ttc aac aat ggt      240
Gly Tyr Gln Leu Ser Asp His Thr Val Gly Val Leu Phe Asn Asn Gly
65                  70                  75                  80

gct cac atg agc ctc ctt cca gac aaa aaa aca gtt cac tat tac gca      288
Ala His Met Ser Leu Leu Pro Asp Lys Lys Thr Val His Tyr Tyr Ala
                85                  90                  95

gag ctt ggc caa tgc tca gtt ttc cca gca aca gat gct cct gag caa      336
Glu Leu Gly Gln Cys Ser Val Phe Pro Ala Thr Asp Ala Pro Glu Gln
            100                 105                 110

ttt att agt caa gtg acg gtg ctg aaa tac ttt tct cat tac atg gag      384
Phe Ile Ser Gln Val Thr Val Leu Lys Tyr Phe Ser His Tyr Met Glu
        115                 120                 125

gag aac ctc atg gat ggt gga gat ctg cct agt gtt act gat att cga      432
Glu Asn Leu Met Asp Gly Gly Asp Leu Pro Ser Val Thr Asp Ile Arg
    130                 135                 140

aga cct cgg ctc tac ctc ctt cag tgg cta aaa tct gat aag gcc cta      480
Arg Pro Arg Leu Tyr Leu Leu Gln Trp Leu Lys Ser Asp Lys Ala Leu
145                 150                 155                 160

atg atg ctc ttt aat gat ggc acc ttt cag gtg aat ttc tac cat gat      528
Met Met Leu Phe Asn Asp Gly Thr Phe Gln Val Asn Phe Tyr His Asp
                165                 170                 175

cat aca aaa atc atc atc tgt agc caa aat gaa gaa tac ctt ctc acc      576
His Thr Lys Ile Ile Ile Cys Ser Gln Asn Glu Glu Tyr Leu Leu Thr
            180                 185                 190

tac atc aat gag gat agg ata tct aca act ttc agg ctg aca act ctg      624
Tyr Ile Asn Glu Asp Arg Ile Ser Thr Thr Phe Arg Leu Thr Thr Leu
        195                 200                 205

ctg atg tct ggc tgt tca tca gaa tta aaa aat cga atg gaa tat gcc      672
Leu Met Ser Gly Cys Ser Ser Glu Leu Lys Asn Arg Met Glu Tyr Ala
    210                 215                 220

ctg aac atg ctc tta caa aga tgt aac tga                              702
Leu Asn Met Leu Leu Gln Arg Cys Asn

225                 230

<210>  44
<211>  233
<212>  PRT
<213>  Homo sapiens

<400>  44

Leu Gly Ser Cys Ser Ser Ser Ser Glu Cys Leu Glu Asp Ser Thr Met
1               5                   10                  15

Gly Ser Val Ala Asp Thr Val Ala Arg Val Leu Arg Gly Cys Leu Glu
            20                  25                  30

Asn Met Pro Glu Ala Asp Cys Ile Pro Lys Glu Gln Leu Ser Thr Ser
        35                  40                  45

Phe Gln Trp Val Thr Lys Trp Val Asp Tyr Ser Asn Lys Tyr Gly Phe
    50                  55                  60

Gly Tyr Gln Leu Ser Asp His Thr Val Gly Val Leu Phe Asn Asn Gly
65                  70                  75                  80

Ala His Met Ser Leu Leu Pro Asp Lys Lys Thr Val His Tyr Tyr Ala
                85                  90                  95

Glu Leu Gly Gln Cys Ser Val Phe Pro Ala Thr Asp Ala Pro Glu Gln
            100                 105                 110

Phe Ile Ser Gln Val Thr Val Leu Lys Tyr Phe Ser His Tyr Met Glu
        115                 120                 125

Glu Asn Leu Met Asp Gly Gly Asp Leu Pro Ser Val Thr Asp Ile Arg
    130                 135                 140

Arg Pro Arg Leu Tyr Leu Leu Gln Trp Leu Lys Ser Asp Lys Ala Leu
145                 150                 155                 160

Met Met Leu Phe Asn Asp Gly Thr Phe Gln Val Asn Phe Tyr His Asp
                165                 170                 175

His Thr Lys Ile Ile Ile Cys Ser Gln Asn Glu Glu Tyr Leu Leu Thr
            180                 185                 190

Tyr Ile Asn Glu Asp Arg Ile Ser Thr Thr Phe Arg Leu Thr Thr Leu
        195                 200                 205

Leu Met Ser Gly Cys Ser Ser Glu Leu Lys Asn Arg Met Glu Tyr Ala
    210                 215                 220

Leu Asn Met Leu Leu Gln Arg Cys Asn
225                 230